# EUROPEAN PATENT APPLICATION

(11) **EP 1 849 863 A1**
(43) Date of publication of application: **31.10.2007**
(21) Application number: 06701439.9
(22) Date of filing: 23.01.2006
(51) Int. Cl.: C12N 5/10, A01K 67/027, C12P 21/02, C12N 15/13, C12N 15/16, C12N 15/62, C12N 15/67

(54) **NON-HUMAN CHIMERIC ANIMAL AND USE THEREOF**

(30) Priority: 21.01.2005 JP 2005014826
(71) Applicant: Kirin Pharma Kabushiki Kaisha, Shibuya-ku Tokyo (JP)
(72) Inventor: KAKITANI, Makoto, c/o Kirin Pharma Kabushiki Kaisha, Takasaki-shi, Gunma, 370-1295 (JP); TOMIZUKA, Kazuma, c/o Kirin Pharma Kabushiki Kaisha, Takasaki-shi, Gunma, 3701295 (JP)
(74) Representative: HOFFMANN EITLE
(86) International application number: PCT/JP2006/301379
(87) International publication number: WO 2006/078072

(57) **Abstract**

This invention provides: a pluripotent cell derived from a non-human animal comprising foreign DNA that encodes a desired protein in such a manner that the expression of the desired protein is regulated by the control region of a gene expressed in certain cells and/or tissue, wherein the foreign DNA is bound to a nucleic acid fragment comprising a promoter/the whole or part of 5' non-translational region/a leader sequence coding region derived from a gene expressed in certain cells and/or tissue, and wherein in said cell one or more drug resistant marker genes used for introducing the foreign DNA into the genome have been removed; a chimeric non-human animal that is prepared from the pluripotent cell and highly expresses the desired protein, or a progeny thereof; a method for producing a desired protein using the chimeric animal; and a method for analyzing *in vivo* function of a desired gene using the chimeric animal.

SEQUENCE LISTING

<110> Kirin Beer Kabushiki Kaisha

<120> Chimeric non-human animals and uses thereof

<130> PH-2709-PCT

<150> JP 2005-014826
<151> 2005-01-21

<160> 65

<170> PatentIn Ver. 2.1

<210> 1
<211> 31
<212> DNA
<213> Artificial Sequence

<220>
<223> Description of Artificial Sequence:primer

<400> 1
tcgacttaat taaggccggc cctagctagc a                                31

<210> 2

<211> 31

<212> DNA

<213> Artificial Sequence

<220>

<223> Description of Artificial Sequence:primer

<400> 2

agcttgctag ctagggccgg ccttaattaa g                                    31

<210> 3

<211> 45

<212> DNA

<213> Artificial Sequence

<220>

<223> Description of Artificial Sequence:primer

<400> 3

ccttaattaa agttatgtgt cctagagggc tgcaaactca agatc                     45

<210> 4

<211> 53

<212> DNA

<213> Artificial Sequence

<220>

<223> Description of Artificial Sequence:primer

<400> 4
ttggccggcc ttggcgccag tggaacctgg aatgataaac acaaagatta ttg          53

<210> 5
<211> 33
<212> DNA
<213> Artificial Sequence

<220>
<223> Description of Artificial Sequence:primer

<400> 5
ggggtaccag cttttgtgtt tgacccttcc cta                                33

<210> 6
<211> 34
<212> DNA
<213> Artificial Sequence

<220>
<223> Description of Artificial Sequence:primer

<400> 6
ccgctcgaga gctaaaccta ctgtatggac aggg                               34

<210> 7
<211> 34
<212> DNA
<213> Artificial Sequence

<220>
<223> Description of Artificial Sequence:primer

<400> 7
cccaagctta gctcaaacca gcttaggcta caca                                34

<210> 8
<211> 33
<212> DNA
<213> Artificial Sequence

<220>
<223> Description of Artificial Sequence:primer

<400> 8
cgggatccct agaacgtgtc tgggccccat gaa                                 33

<210> 9
<211> 25
<212> DNA
<213> Artificial Sequence

<220>

<223> Description of Artificial Sequence:primer

<400> 9

cagtcctggg cgccccacca cgcct 25

<210> 10

<211> 35

<212> DNA

<213> Artificial Sequence

<220>

<223> Description of Artificial Sequence:primer

<400> 10

ttggccggcc tcatctgtcc cctgtcctgc aggcc 35

<210> 11

<211> 45

<212> DNA

<213> Artificial Sequence

<220>

<223> Description of Artificial Sequence:primer

<400> 11

ccttaattaa atattttcct ccttctccta ccagtaccca ctctt 45

<210> 12
<211> 53
<212> DNA
<213> Artificial Sequence

<220>
<223> Description of Artificial Sequence:primer

<400> 12
ttggccggcc ttggcgcctc tggacagtat gactagaaaa aagcaaaata gag 53

<210> 13
<211> 41
<212> DNA
<213> Artificial Sequence

<220>
<223> Description of Artificial Sequence:primer

<400> 13
cgggatcccg gccaccatgg gggtgcacga atgtcctgcc t 41

<210> 14
<211> 36

<212> DNA
<213> Artificial Sequence

<220>
<223> Description of Artificial Sequence:primer

<400> 14
ccgctcgagc gctatctgtc ccctgtcctg caggcc 36

<210> 15
<211> 26
<212> DNA
<213> Artificial Sequence

<220>
<223> Description of Artificial Sequence:primer

<400> 15
ggccaggccc tgttggtcaa ctcttc 26

<210> 16
<211> 25
<212> DNA
<213> Artificial Sequence

<220>
<223> Description of Artificial Sequence:primer

<400> 16
cgcttgtggg gaagcctcca agacc 25

<210> 17
<211> 28
<212> DNA
<213> Artificial Sequence

<220>
<223> Description of Artificial Sequence:primer

<400> 17
tcgagtcgcg acaccggcgg gcgcgccc 28

<210> 18
<211> 28
<212> DNA
<213> Artificial Sequence

<220>
<223> Description of Artificial Sequence:primer

<400> 18
tcgagggcgc gcccgccggt gtcgcgac 28

<210> 19

<211> 29

<212> DNA

<213> Artificial Sequence

<220>

<223> Description of Artificial Sequence:primer

<400> 19

ggccgcttaa ttaaggccgg ccgtcgacg 29

<210> 20

<211> 29

<212> DNA

<213> Artificial Sequence

<220>

<223> Description of Artificial Sequence:primer

<400> 20

aattcgtcga cggccggcct taattaagc 29

<210> 21

<211> 49

<212> DNA

<213> Artificial Sequence

<220>

<223> Description of Artificial Sequence:primer

<400> 21

ataagaatgc ggccgcaaag ctggtgggtt aagactatct cgtgaagtg 49

<210> 22

<211> 45

<212> DNA

<213> Artificial Sequence

<220>

<223> Description of Artificial Sequence:primer

<400> 22

acgcgtcgac tcacaggttg gtccctctct gtgtgtggtt gctgt 45

<210> 23

<211> 42

<212> DNA

<213> Artificial Sequence

<220>

<223> Description of Artificial Sequence:primer

<400> 23

ttggcgcgcc ctccctagga ctgcagttga gctcagattt ga 42

<210> 24

<211> 44

<212> DNA

<213> Artificial Sequence

<220>

<223> Description of Artificial Sequence:primer

<400> 24

ccgctcgagt cttactgtct cagcaacaat aatataaaca gggg 44

<210> 25

<211> 25

<212> DNA

<213> Artificial Sequence

<220>

<223> Description of Artificial Sequence:primer

<400> 25

catacaaaca gatacacaca tatac 25

<210> 26

<211> 25

<212> DNA

<213> Artificial Sequence

<220>
<223> Description of Artificial Sequence:primer

<400> 26
gtcattaatg gaaggaagct ctcta                                                25

<210> 27
<211> 25
<212> DNA
<213> Artificial Sequence

<220>
<223> Description of Artificial Sequence:primer

<400> 27
tcttactaga gttctcacta gctct                                                25

<210> 28
<211> 25
<212> DNA
<213> Artificial Sequence

<220>
<223> Description of Artificial Sequence:primer

<400> 28

ggaaccaaag aatgaggaag ctgtt 25

<210> 29

<211> 38

<212> DNA

<213> Artificial Sequence

<220>

<223> Description of Artificial Sequence:primer

<400> 29

atctcgagga accactttcc tgaggacaca gtgatagg 38

<210> 30

<211> 38

<212> DNA

<213> Artificial Sequence

<220>

<223> Description of Artificial Sequence:primer

<400> 30

atgaattcct aacactcatt cctgttgaag ctcttgac 38

<210> 31

<211> 32

<212> DNA

<213> Artificial Sequence

<220>

<223> Description of Artificial Sequence:primer

<400> 31

atgaattcag acaaaggtcc tgagacgcca cc 32

<210> 32

<211> 42

<212> DNA

<213> Artificial Sequence

<220>

<223> Description of Artificial Sequence:primer

<400> 32

atggatcctc gagtcgactg gatttcaggg caactaaaca tt 42

<210> 33

<211> 32

<212> DNA

<213> Artificial Sequence

<220>

<223> Description of Artificial Sequence:primer

<400> 33
atgaattcgc ccctctccct cccccccccc ta                                32

<210> 34
<211> 38
<212> DNA
<213> Artificial Sequence

<220>
<223> Description of Artificial Sequence:primer

<400> 34
atgaattcgt cgacttgtgg caagcttatc atcgtgtt                          38

<210> 35
<211> 8
<212> DNA
<213> Artificial Sequence

<220>
<223> Description of Artificial Sequence:primer

<400> 35
agtcgaca                                                            8

<210> 36
<211> 14
<212> DNA
<213> Artificial Sequence

<220>
<223> Description of Artificial Sequence:primer

<400> 36
aatttgtcga ctgc 14

<210> 37
<211> 44
<212> DNA
<213> Artificial Sequence

<220>
<223> Description of Artificial Sequence:primer

<400> 37
cccaagcttt ggtgattatt cagagtagtt ttagatgagt gcat 44

<210> 38
<211> 45
<212> DNA
<213> Artificial Sequence

<220>
<223> Description of Artificial Sequence:primer

<400> 38
acgcgtcgac tttgtctttg aactttggtc cctagctaat tacta 45

<210> 39
<211> 63
<212> DNA
<213> Artificial Sequence

<220>
<223> Description of Artificial Sequence:primer

<400> 39
acgcgtcgac gcggccggcc gcgctagcag acaaaggtcc tgagacgcca ccaccagctc 60
ccc 63

<210> 40
<211> 43
<212> DNA
<213> Artificial Sequence

<220>
<223> Description of Artificial Sequence:primer

<400> 40

gaagatctca agtgcaaaga ctcactttat tgaatatttt ctg 43

<210> 41

<211> 42

<212> DNA

<213> Artificial Sequence

<220>

<223> Description of Artificial Sequence:primer

<400> 41

ggaattcaga caaaggtcct gagacgccac caccagctcc cc 42

<210> 42

<211> 44

<212> DNA

<213> Artificial Sequence

<220>

<223> Description of Artificial Sequence:primer

<400> 42

cccaagcttg cctcctcaaa cctaccatgg cccagagaaa taag 44

<210> 43

<211> 51

<212> DNA

<213> Artificial Sequence

<220>

<223> Description of Artificial Sequence:primer

<400> 43

ataagaatgc ggccgcctca gagcaaatgg gttctacagg cctaacaacc t 51

<210> 44

<211> 44

<212> DNA

<213> Artificial Sequence

<220>

<223> Description of Artificial Sequence:primer

<400> 44

ccggaattcc taacactcat tcctgttgaa gctcttgaca atgg 44

<210> 45

<211> 39

<212> DNA

<213> Artificial Sequence

<220>

<223> Description of Artificial Sequence:primer

<400> 45
ccgctcgagc ggccaccatg ggggtgcacg aatgtcctg 39

<210> 46
<211> 33
<212> DNA
<213> Artificial Sequence

<220>
<223> Description of Artificial Sequence:primer

<400> 46
ccgctcgagc ggtcatctgt cccctgtcct gca 33

<210> 47
<211> 17
<212> DNA
<213> Artificial Sequence

<220>
<223> Description of Artificial Sequence:linker

<400> 47
ggccaggcgc gccttgc 17

<210> 48
<211> 17
<212> DNA
<213> Artificial Sequence

<220>
<223> Description of Artificial Sequence:linker

<400> 48
ggccgcaagg cgcgcct                                                    17

<210> 49
<211> 20
<212> DNA
<213> Artificial Sequence

<220>
<223> Description of Artificial Sequence:linker

<400> 49
taagggctag ctagggccgg                                                 20

<210> 50
<211> 18
<212> DNA
<213> Artificial Sequence

<220>

<223> Description of Artificial Sequence:linker

<400> 50

ccctagctag cccttaat 18

<210> 51

<211> 10

<212> DNA

<213> Artificial Sequence

<220>

<223> Description of Artificial Sequence:linker

<400> 51

tcgagttaac 10

<210> 52

<211> 10

<212> DNA

<213> Artificial Sequence

<220>

<223> Description of Artificial Sequence:linker

<400> 52

agctgttaac 10

<210> 53
<211> 27
<212> DNA
<213> Artificial Sequence

<220>
<223> Description of Artificial Sequence:linkerr

<400> 53
agctgtcgac ttaattaagg ccggccg 27

<210> 54
<211> 27
<212> DNA
<213> Artificial Sequence

<220>
<223> Description of Artificial Sequence:linker

<400> 54
ctagcggccg gccttaatta agtcgac 27

<210> 55
<211> 26

<212> DNA
<213> Artificial Sequence

<220>
<223> Description of Artificial Sequence:primer

<400> 55
ggccaggccc tgttggtcaa ctcttc 26

<210> 56
<211> 25
<212> DNA
<213> Artificial Sequence

<220>
<223> Description of Artificial Sequence:primer

<400> 56
cgcttgtggg gaagcctcca agacc 25

<210> 57
<211> 25
<212> DNA
<213> Artificial Sequence

<220>
<223> Description of Artificial Sequence:primer

<400> 57

caccatggag aaggccgggg cccac                                          25

<210> 58

<211> 25

<212> DNA

<213> Artificial Sequence

<220>

<223> Description of Artificial Sequence:primer

<400> 58

atcatacttg gcaggtttct ccagg                                          25

<210> 59

<211> 92

<212> DNA

<213> Artificial Sequence

<220>

<223> Description of Artificial Sequence:linker

<400> 59

tcgataactt cgtataaagt atcctatacg aagttataga tctataactt cgtataaagt 60

atcctatacg aagttatgtt taaacgttaa cg                                  92

<210> 60

<211> 92

<212> DNA

<213> Artificial Sequence

<220>

<223> Description of Artificial Sequence:linker

<400> 60

tcgacgttaa cgtttaaaca taacttcgta taggatactt tatacgaagt tatagatcta 60

taacttcgta taggatactt tatacgaagt ta 92

<210> 61

<211> 26

<212> DNA

<213> Artificial Sequence

<220>

<223> Description of Artificial Sequence:primer

<400> 61

ggccaggccc tgttggtcaa ctcttc 26

<210> 62

<211> 25

<212> DNA

<213> Artificial Sequence

<220>
<223> Description of Artificial Sequence:primer

<400> 62
cgcttgtggg gaagcctcca agacc                                          25

<210> 63
<211> 25
<212> DNA
<213> Artificial Sequence

<220>
<223> Description of Artificial Sequence:primer

<400> 63
caccatggag aaggccgggg cccac                                          25

<210> 64
<211> 25
<212> DNA
<213> Artificial Sequence

<220>
<223> Description of Artificial Sequence:primer

<400> 64

atcatacttg gcaggtttct ccagg 25

<210> 65

<211> 39

<212> DNA

<213> Mus musculus

<400> 65

agtttctgca cgggcagtca gttagcagca ctcactgtg 39

## Description

### FIELD OF THE INVENTION

The present invention relates to a chimeric non-human animal with enhanced ability to express foreign DNA and a progeny thereof, and to use of the same. Specifically, the present invention further relates to a method for analyzing functions of a desired protein or a gene encoding the same and/or a method for producing a useful substance by using such a chimeric non-human animal or progeny thereof.

### BACKGROUND OF THE INVENTION

Historical research outcomes of sequencing the entire human genome nucleotides (International Human Genome Sequencing Consortium, Nature, 409:860-921, 2001) have brought a new research subject of elucidating functions of a great number of novel genes. For example, in human chromosome 22, which is the second smallest of the 24 human chromosomes and whose entire nucleotide sequence was first determined (Dunham et al., Nature, 402:489-495, 1999), it was predicted that 545 genes (excluding pseudogenes) are present. Of them, 247 genes are known in terms of their nucleotide and amino acid sequences, 150 genes are novel ones that are homologous to known genes, and 148 genes are novel ones that are homologous to the sequences whose functions are unknown and which have been registered in the Expressed Sequence Tag (EST) database. In addition, through the analysis using the software (GENESCAN) which enables a direct prediction of a gene from the genomic sequences, it was predicted that there might exist further 325 novel genes whose transcriptional products have not been identified (Dunham et al., supra). Clarifying the *in vivo* functions of genes and proteins (as gene products) is important not only for understanding of a program of the life activity but also for development of a novel medicament to overcome a variety of human diseases. Thus, there is a big demand for development of techniques to efficiently elucidate the function of a novel gene in the post-genomic life science and medical researches.

The embryonic stem cell (or ES cell) refers to an undifferentiated cell line, which is established from an inner cell mass of the blastocyst and has an ability to differentiate into various types of somatic tissue including germ cells. In the case of mice, for example, when ES cell is injected into an early murine embryo (i.e., host embryo), a chimeric mouse is born having somatic cells which are a mixture of cells derived from the ES cell and the host embryo. In particular, a chimeric mouse having a germ cell derived from ES cell and capable of transmitting the genetic information of the ES cell to its progeny is called a germ-line chimera. When germ-line chimeras are mutually crossed, or when a germ-line chimera is crossed with an appropriate mouse line, F1 mice having the ES cell-derived genetic information are born. If ES cells are previously engineered in such techniques by modifying a certain gene in the cell or by inserting a certain gene into the cell, a knock-out (KO) mouse, transgenic (Tg) mouse, or knock-in (KI) mouse can be produced. From the analyzed outcomes of KO mice which have been so far produced by many researchers, important information and many human-disease animal models were provided or produced in a wide variety of fields from fundamental biology to clinical medicine. The KO mouse is still the most widely used tool for clarifying an *in vivo* function of a gene. On the other hand, the KI mouse is produced by inserting certain foreign DNA into a particular murine gene in the manner of homologous recombination (Le Mouellic et al., 2002; Japanese Patent No. 3,298,842) or of random insertion (Gossler et al., Science, 244: 463-465, 1989), in general. Furthermore, mice produced by inserting an expression unit comprising a certain promoter, foreign DNA, and a polyA addition site into a particular chromosomal region have been reported as suitable for analyzing the *in vivo* functions of many genes (Tomizuka et al., 2003, WO 03/041,495).

However, for the Tg mouse, KI mouse or KO mouse, a lot of time and labor are required for manipulating only a single gene. Usually, the efficiency of homologous recombination is about one per 100-10,000 random insertion clones. To improve the ratio of homologous recombinants to randomly inserted recombinants, various attempts have been hitherto made. For example, Deng & Capecchi (Mol. Cell. Biol., 12:3365-71, 1992) reported that the longer the length of a genomic DNA of the homologous region contained in a vector, the more preferable, and that it is preferable to use the isogenic DNA which is a genomic DNA from the same murine species as that from which ES cell for use in targeting is derived. Furthermore, the method most widely used at present is to employ KO vectors comprising a negative selection marker outside the homologous genomic DNA region, in addition to the selection marker. The negative selection method utilizes a phenomenon where the cells having random inserts die because of expression of a virulent negative marker, whereas the homologous recombinants survive because such virulent expression does not occur. Examples of the negative selection marker include HSV-tk gene (in this case, culture medium must contain a thymidine analogue such as ganciclovir or FIAU) reported by Mansour et al. (Nature, 336:348-352, 1988), and DT-A (diphtheria toxin A chain) reported by Yagi et al. (Anal. Biochem., 214:77-86, 1993). When the negative selection theoretically works, all colonies presumably become homologous recombinants. However, actually, the rate of homologous recombinants greatly varies from report to report. The efficiency of obtaining homologous recombinants by the negative selection method (i.e., the concentration effect) is generally several folds higher than other methods.

Not only mice produced from mutant ES cell lines but also mammalian cell lines, which have a gene modified or destroyed by homologous recombination, are important materials in clarifying a function of the modified or destroyed gene. Furthermore, homologous recombination has been considered as an ultimate therapy for diseases (especially, hereditary diseases) caused by defect or mutation of a gene. Nevertheless, the ratio of homologous recombinants to randomly inserted recombinants in the mammalian cell lines or the primary culture cells is equal to or lower than that in murine ES cells. In this context, it has been desired to improve said ratio in applying this method to gene-function analysis and gene therapy at a cellular level (Yanez & Poter, Gene Therapy, 5:149-159, 1998).

In the present stage where the entire human genome nucleotide sequence has been determined, what is desired next is a system capable of exhaustively analyzing in *vivo* functions for multiple novel genes. For this purpose, it is necessary to reliably, easily and simultaneously produce a plurality of types of animal individuals capable of highly expressing a transfer gene. Of the novel genes brought by the human genomic information, genes encoding secretory proteins homologous to cytokines, growth factors, and hormones are interested as research subjects since they directly act as medicaments. In other words, developing new efficient methods of analyzing *in vivo* functions of genes encoding secretory proteins or gene products presumably facilitates development of medicaments for treating human diseases.

### DISCLOSURE OF THE INVENTION

An object of the present invention is to provide an embryonic stem (ES) cell having an improved efficiency of homologous recombination and having an increased expression level of a protein encoded by DNA introduced in a certain chromosome region.

Another object of the present invention is to provide a method for preparing a genetically recombinant non-human animal that expresses a desired protein with the use of a genetically modified ES cell.

Further object of the present invention is to provide a simple and highly reproducible method for analyzing functions of a target gene and/or producing a useful substance using a genetically recombinant non-human animal or a progeny thereof.

We have now discovered that use of an ES cell comprising a drug resistant marker gene expression unit inserted into a certain chromosome region, i.e. an RS element region, could remarkably improve the ratio of homologous recombinants of the gene targeting vector comprising the immunoglobulin gene C_{K} exon region located about 25 kb upstream of the RS element region to randomly inserted recombinants thereof. In the past, the fact that modification that had been previously provided in a given chromosome region would influence the homologous recombination efficiency in gene targeting that utilizes a targeting vector that does not contain the sequence of the above region was not known, and such a finding was surprising.

Furthermore, we have now succeeded in preparing a chimeric mouse by injecting a genetically modified ES cell into a B cell deficient host embryo. In the chimeric animal prepared by such a technique, the effects of overexpression of gene products derived from the structural genes that had been introduced were observed, regardless of the chimeric rate of the coat color. With the utilization of such a system, it was confirmed that a chimeric animal that expresses the transfer genes in a more efficient and reliable manner at a higher level than a conventional technique could be obtained.

Thus, we have now discovered: a method for preparing a chimeric non-human animal that expresses a desired protein; a chimeric non-human animal that expresses a desired protein or a progeny thereof; or a method that remarkably enhances the expression level of a desired protein than a conventional technique, by utilizing cells or tissue from said animal, or hybridomas derived therefrom.

When the object of the invention disclosed in PCT International Application WO 00/10383 (published March 2, 2000) filed by the applicant of the present invention, i.e., identification of *in vivo* functions of genes whose secretory functions are unknown, is taken into consideration, further improvement in the expression level (i.e., the blood serum level) of a chimeric non-human animal could lead to enhanced efficiency of identification of functions of the gene. When no change was observed in a non-human animal in which a gene whose functions are unknown had been expressed, for example, the amount of the gene of interest secreted from the B cell is not large enough. Thus, such amount may not have reached the critical concentration required in the vicinity of tissue as a target of the gene. In such a case, if expression is possible at a higher level, then the gene product may be provided at a level exceeding the critical concentration, whereby a phenotype may be observed. In order to produce a useful protein for medical and other purposes in animal body fluid, a wide variety of transgenic animals have been produced. In order to attain a sufficient expression level, however, numerous transgenic animals should be first prepared, and adequate transgenic animals should be selected therefrom. Thus, it was difficult to attain individuals that expresse the transfer genes at high levels. The system of the present invention that enables foreign DNA to express at high levels, accordingly, has a great deal of potential in industry.

To further improve the expression level of a transfer gene (i.e., the blood serum level) in the chimeric non-human animals as disclosed in WO 03/041495 (published March 2, 2000) filed by the applicant of the present invention, we conducted concentrated studies, as a result, we have now found a method that could further elevate the expression level of a transferred gene. The present invention includes the following two different methods A and B to achieve the above-mentioned objects. Method A is concerned with a gene fragment comprising a promoter/a 5' non-translational region/a leader sequence coding region, which fragment is contained in a transfer gene expression cassette. Method B involves the removal of a drug resistant gene marker that is present in the vicinity of the transfer gene. More surprisingly, simultaneous performance of method A and method B would result in a synergistic effect, i.e.the that foreign DNA can be expressed at a much higher level than the conventional methods for preparing a Tg mouse.

Hereafter, methods A and B are briefly described.

### Method A:

Regarding the gene fragment containing a promoter/a 5' non-translational region/a leader sequence coding region, which fragment is contained in the transfer gene expression cassette, modifications as shown below, which differ from the method disclosed in WO 03/041495, were applied.
(1) Concerning the Igκ promoter, in addition to a promoter derived from the Igκ variable region identical to the promoter of the above disclosure, comparison was performed using a promoter derived from a different Igκ variable region.
(2) As the Igκ promoter, a genomic fragment (about 450 bp) stretched upstream, which is longer than the above disclosure (about 300 bp), was used.
(3) A sequence stretched from the promoter of (1) and (2) above, the transcription initiation point, the intron, toward the leader sequence coding region was obtained from the murine immunoglobulin κ gene genome.
(4) In the above prior art disclosure, the native leader sequence coding region of the transfer gene was used; however, a sequence prepared by artificially binding the leader sequence coding region of the Igκ variable region included in the fragment described in (3) to the transfer gene from which the native leader sequence coding region had been removed was used in the present invention.

### Method B:

The obtained knock-in ES cells were subjected to the treatment below, in addition to the method described in WO 03/041495.
(1) The puromycin resistant gene cassette located downstream of the transfer gene was removed with the use of the Cre/loxP system.
(2) The puromycin resistant gene cassette located downstream of the transfer gene and the neomycin resistant gene cassette located in the RS region were removed with the use of the Cre/loxP system.

In addition to the method disclosed in WO 03/041495, the method described in A above was performed. As a result, the expression level (i.e., the blood serum level) was enhanced by 10 times or more compared with the method disclosed in WO 03/041495. When the same promoter region as the conventional technique was compared with the different promoter region that was newly examined, the latter was found to produce greater effects. When the method described in B above was performed in addition to the method disclosed in WO 03/041495, the expression level (i.e., the blood serum level) was enhanced by 10 times or more compared with the method disclosed in WO 03/041495. Further, surprisingly, the simultaneous performance of method A and method B enhanced the expression level (i.e., the blood serum level) by 100 times or more when compared with the method disclosed in WO 03/041495.

The illustrative knock-in chimeric mice used in method A, method B, and method AB, which is the combination of method A and method B, of the present invention exhibited the serum human erythropoietin (hEPO) levels remarkably higher than the levels observed in a conventional method for preparing a transgenic (Tg) animal or in the foreign DNA forced expression system via viral vector administration. The serum endogenous EPO level of a normal animal is considered to be 10 pg/ml or lower; however, such a level is raised to 100 pg/ml or higher upon expression of foreign DNA. This results in the expression of the phenotype that indicates the increased blood erythrocytes. In the case of the hEPO-Tg mouse prepared for the first time, for example, the serum hEPO level is found to be 100-150 pg/ml, and the phenotype that indicates the increased blood erythrocytes was observed (Semenza et al., Proc. Natl. Acad. Sci. U.S.A., 86: 2301-2305, 1989). Further, in the Tg mouse that expresses hEPO at a higher level, which has been recently prepared, the serum hEPO level was found to be about 1,250 pg/ml, a more prominent phenotype was observed, compared with the aforementioned Tg mouse, i.e., spleen hypertrophy involving extramedullary hemopoiesis or a hematocrit value of 0.9 or higher (Ruschitzka et al., Proc. Natl. Acad. Sci. U.S.A., 97: 11609-11613). In the knock-in chimeric mouse comprising the hEPO expression unit containing the Igκ promoter inserted downstream of the Cκ exon polyA addition site disclosed in WO 03/041495, however, the serum hEPO level was several ng/ml, which was higher than that of the Tg mouse according to the aforementioned conventional technique. The serum hEPO levels detected upon forced expression via administration of adeno-associated virus or retrovirus containing the hEPO gene expression cassette to the mouse were approximately several ng/ml (Villeval et al., Leukemia, 6:107-115, 1992; Kessker et al., Proc. Natl. Acad. Sci. U.S.A., 93:14082-14087, 1996).

As described in the Examples below, when method A was performed in addition to the method disclosed in WO 03/041495, the serum hEPO level was 45 ng/ml or higher. When method B was performed, the serum hEPO level was 50 ng/ml or higher. When method A and method B were simultaneously performed, the serum hEPO level was as high as 1,000 ng/ml or higher. This value was several hundred times or higher than the highest expression level attained with the use of the hEPO expressing Tg mouse prepared by the conventional technique. That is, the method of the present invention was found to be effective for the expression of foreign DNA in transgenic animals.

### SUMMARY OF THE INVENTION

The present invention based on the above findings is summarized as follows.

The first aspect of the present invention provides pluripotent cells derived from non-human animals comprising foreign DNA that encodes a desired protein in such a manner that the expression of the desired protein is regulated by the control region of a gene expressed in certain cells and/or tissue, wherein the foreign DNA is bound to a nucleic acid fragment comprising a leader sequence coding region derived from the gene expressed in certain cells and/or tissue.

Also, the present invention provides pluripotent cells derived from non-human animals comprising on the genome foreign DNA that encodes a desired protein in such a manner that the expression of the desired protein is regulated by the control region of a gene expressed in certain cells and/or tissue, wherein the foreign DNA is bound to a nucleic acid fragment comprising a leader sequence coding region derived from the gene expressed in certain cells and/or tissue.

To this end, the present invention is advantageous in that the foreign DNA that encodes a desired protein is overexpressed at a significant level in a chimeric non-human animal derived from the aforementioned cell or a progeny thereof.

According to one embodiment of the present invention, the gene expressed in certain cells and/or tissue is the immunoglobulin gene, preferably the immunoglobulin light chain gene, and more preferably the immunoglobulin κ light chain gene.

According to another embodiment of the present invention, the nucleic acid fragment can further comprise a promoter of the gene expressed in certain cells and/or tissue. In addition to the promoter, the nucleic acid fragment can further comprise the whole or part of the entire 5' non-translational region between the promoter and the leader sequence coding region of the gene expressed in certain cells and/or tissue.

According to another embodiment of the present invention, the nucleic acid fragment comprises a nucleic acid sequence comprising a promoter/the whole of part of 5' non-translational region/a leader sequence coding region of the gene expressed in certain cells and/or tissue. Such a nucleic acid fragment is particularly preferable in the present invention. A specific example is a nucleic acid fragment comprising the promoter/the whole or part of the entire 5' non-translational region/the leader sequence coding region of the immunoglobulin gene derived from a non-human animal. The whole 5' non-translational region is further preferable.

According to another embodiment of the present invention, the nucleic acid fragment is greater than 300 bp in length. The length of the nucleic acid fragment is, but is not limited to, 350 to 500 bp, and more preferably 400 to 450 bp, for example.

According to another embodiment of the present invention, a sequence encoding a poly A signal region is ligated to a site downstream of foreign DNA encoding a desired protein.

According to another embodiment of the present invention, one or more drug resistant marker genes used for introducing the foreign DNA into the genome are removed from the pluripotent cells. Preferably, all drug resistant marker genes are removed therefrom. In Examples below, for example, when foreign DNA is homologously recombined on the genome, a drug resistant marker gene is inserted into the RS element located downstream of the genomic immunoglobulin gene (particularly the immunoglobulin κ light chain gene) of a non-human animal, specifically a mouse, or a region having functions equivalent thereto, and then this drug resistant marker gene is removed after homologous integration of foreign DNA.

In the present invention, preferably, the nucleic acid fragment further comprises the whole or part of the entire 5' non-translational region between the promoter and the leader sequence coding region of the gene expressed in certain cells and/or tissue and one or more drug resistant marker genes used for introducing foreign DNA into the genome are removed from the pluripotent cells.

According to another embodiment of the present invention, the alleles of the gene expressed in certain cells and/or tissue are inactivated.

According to another embodiment of the present invention, the pluripotent cells are embryonic stem (ES) cells. ES cells are murine ES cells, for example.

The second aspect of the present invention provides a method for preparing a chimeric non-human animal that overexpresses foreign DNA encoding a desired protein, comprising the steps of: preparing a pluripotent cell derived from non-human animals and introducing the resulting cell into a host embryo to obtain a chimeric embryo; transplanting the chimeric embryo to a surrogate mother of a cognate non-human animal; and selecting a chimeric non-human animal that expresses foreign DNA encoding a desired protein from among the resulting offspring animals.

According to an embodiment, the chimeric non-human animal is a mouse.

According to the other embodiment, the pluripotent cells are ES cells.

The third aspect of the present invention provides a chimeric non-human animal, which is prepared by the above method and which overexpresses foreign DNA encoding a desired protein.

The fourth aspect of the present invention provides a progeny of a non-human animal, which is prepared by mutual crossing of the chimeric non-human animals or crossing of a chimeric non-human animal and a cognate non-human animal and which overexpresses foreign DNA encoding a desired protein.

The fifth aspect of the present invention provides a method for preparing a protein, comprising expressing desired foreign DNA using the above chimeric non-human animal or a progeny thereof, a cell or tissue obtained therefrom, or a hybridoma obtained therefrom, and recovering a protein produced that encoded by the DNA.

The sixth aspect of the present invention provides a method for analyzing *in vivo* function of a desired protein or DNA encoding the desired protein, comprising comparing a phenotype of the chimeric non-human animal or a progeny thereof with a phenotype of a corresponding wild-type non-human animal that does not contain foreign DNA encoding a desired protein and thereby determining a difference between such phenotypes.

The terms used in the present invention are defined as follows.

The term "genome" used herein refers to chromosome DNA that plays a key role in determining the genetic information contained in a nucleus of a living cell.

The term "non-human animal" as used herein refers to an animal excluding a human and is generally selected from vertebrates including fish, reptile, amphibian, bird, and mammal, preferably mammals. Since chimeric non-human animals are preferably produced by use of embryonic stem cells as pluripotent cells in the invention, any non-human animals from which embryonic stem cells can be established (for example, mouse, cow, sheep, pig, hamster, monkey, goat, rabbit, and rat), or any other non-human animals from which embryonic stem cells will be established in future, are encompassed in the non-human animal to be intended by the invention.

The term "chimeric non-human animal" as used herein refers to an animal established from differentiated cells derived from a pluripotent cell (as described below) or a host embryo (Bradley et al., Nature, 309: 255-6, 1984). Experimentally, animals whose cells are completely from a host embryo (0% chimera) or animals whose cells are completely from a pluripotent cell (100% chimera) could be born Such animals are not strictly "chimera" but are included in the "chimeric non-human animal" for the convenience sake.

The term "pluripotent cell" as used herein refers to a cell capable of differentiating into at least two types of cells or tissue of a chimeric non-human animal which is produced by injecting the cell into a host embryo or by forming an aggregated embryo. Specific examples of the pluripotent cell include embryonic stem cells (ES cells), embryonic germ cells (EG cells) and embryonic carcinoma cells (EC cells).

The term "embryonic stem cell" as used herein, also called ES (embryonic stem) cell, refers to a cultured cell derived from the early embryo and characterized in that it has a proliferative potency while maintaining an undifferentiated state (or totipotency). In other words, the embryonic stem cell means a cell line established by culturing a cell of inner cell mass, i.e. an undifferentiated stem cell present in the early embryo (blastocyst stage) of an animal, so that the cell line can continuously proliferate while maintaining an undifferentiated state. The term "embryonic germ cell," also called "EG cell," refers to a cultured cell derived from the primordial germ cell and characterized in that it has almost the same potency as that of the embryonic stem cell. The embryonic germ cell means a cell line established by culturing the primordial germ cell obtained from the embryo of several days to several weeks after fertilization (for example, about 8.5-day old embryo in mouse), so that the cell line can continuously proliferate while maintaining an undifferentiated state.

The term "a desired protein" as used herein refers to a protein that is to be intentionally expressed in at least one type of cell and/or tissue of a chimeric non-human animal produced by the method of the present invention. It is no matter whether the protein is known or unknown in function. Examples of the desired proteins may be mammalian proteins such as functional secretory proteins, functional membrane proteins, functional intracellular or intranuclear proteins, and soluble portions of functional membrane proteins with added secretory signal. The term "functional" as used herein means possession of a specific role, effect or function *in vivo.* In the case of a protein known in function, a new finding as to interrelation between functions of the protein may be provided by observing what effect is brought by the protein when it is highly expressed in at least one type of cell and/or tissue of a chimeric non-human animal. In the case of a protein unknown in function, a hint for elucidating the function of the protein may be found by observing any effect brought by the protein when it is highly expressed. In the present invention, the "desired protein" is expressed in a chimeric non-human animal into which a gene encoding the protein is introduced; however, it may be acceptable if it is not expressed or slightly expressed in certain cells and/or tissue of interest wherein the protein is intended to be expressed. Also, the "desired protein" may be derived from a xenogenic animal. As long as it is a "desired protein" of interest, any types of proteins may be used.

The term "a nucleic acid sequence encoding a desired protein" as used herein may be either endogenous DNA or exogenous DNA. Also exogenous DNA includes a DNA derived from human. In the specification, the term "a structural gene encoding a desired protein" is interchangeably used.

The term "expression" of a protein as used herein has the same meaning as expression of a gene encoding the protein.

The term "a leader sequence coding region" as used herein refers to the 5' region upstream of the translation initiation codon of the gene expressed in certain cells and/or tissue. In other words, it refers to a region comprising a nucleic acid sequence encoding the N-terminal sequence, i.e., a so-called precursor protein sequence from which a mature protein sequence has been removed. The "secretory signal sequence" and the "signal peptide coding region" are also within the scope of the leader sequence coding region.

The term "control region" as used herein refers collectively to "control sequence," "regulatory sequence" and "regulatory region" and indicates a region for controlling or regulating gene expression (i.e., transcription, translation, or protein synthesis). Examples of such a control region include, but are not limited to, a promoter, enhancer, and silencer. Also the term "control region" as used herein may contain a functional element (such as a promoter sequence) or a plurality of elements (such as a promoter sequence and an enhancer sequence). Furthermore, the "promoter sequence" is a kind of control region known by those skilled in the art and indicates a nucleotide sequence upstream of a structural gene to which RNA polymerase is bound at the initiation time of translation.

The term "internal ribosomal entry site" as used herein is simply referred to as "IRES" and is known as an element enabling polycistronic expression. The IRES forms a specific RNA secondary structure and is a site enabling initiation of ribosomal translation from an initiation codon downstream thereof In the case of a mammal, the IRES binds to a decode subunit of a ribosome thereby causing a conformational change such that a protein coding region adjacent to the decode subunit is pulled into the decoding site. In this manner, IRES is presumably involved in the event initiating the translation and protein synthesis (Spahn et al. Science 291:1959, 2001).

The term "polyA signal region" as used herein refers to a nucleotide sequence, which is positioned at the end of the transcription region and directs polyadenylation to the 3' non-translational region of pre-mRNA after transcription.

The terms "upstream" and "downstream" as used herein refer to the direction to 5' end or 3' end, respectively, in a nucleic acid sequence such as genome or polynucleotide.

The terms "bp (base pair)" and "Kb or kb (kilo base pair)" as used herein refer to the length or distance of a nucleic acid sequence. "One (1) bp" indicates a single base pair, and "1 Kb" corresponds to 1,000 bp.

The term "allele" as used herein refers to genes which are located in homologous regions of a homologous chromosome in an organism having a polyploidal genome and are functionally homologous. The allele is usually all expressed. The term "allelic exclusion" refers to the phenomenon where traits derived from both allelic genes are expressed in an organism individual, however, one of the allelic genes is only expressed at random in individual cells, whereas expression of the other gene is excluded. This phenomenon is usually seen in antibody genes and T cell receptor genes, wherein because the recombination of one allelic variable region gene is interpreted as a signal, only one complete gene is produced.

The term "a soluble portion of a membrane protein with added secretory signal" as used herein refers to an extracellular domain of membrane protein molecule to which a secretory signal (or signal sequence) is bound.

The term "immunoglobulin light chain gene" as used herein refers to a gene encoding a light chain (or L-chain) of an immunoglobulin (Ig) molecule. The light chains include κ-chain and λ-chain, each of which consists of variable (V) and constant (C) regions. The light chain gene is constituted of a single C region gene, a plurality of V region genes, and a plurality of joint (J) region genes. In the present invention, the light chain constant region gene is preferably used, and the κ light chain constant region gene is more preferable particularly when pluripotent murine cells are employed. Since genomic analyses of human and mouse among mammals have been virtually completed, genomic information is available at present. As a result of the analyses (i.e., comparison of genomic sequence homology), the human and murine genomes have % homology of about 85%. For these reasons, mouse can preferably be selected as an animal species, and murine pluripotent cells can preferably be used. In the present invention, however, animals other than mouse, for example, cow, sheep, pig, hamster, monkey, goat, rabbit, and rat may be used. The immunoglobulin gene sequence of an animal, if the sequencing has been completed, is available from documents or the databases such as the GenBank (NCBI in U.S.A.) and EMBL (EBI in Europe). In the case where the sequencing of a gene has not yet been made, it can be determined by combination of common techniques such as fragmentation of genomic DNA with restriction enzymes, mapping, construction of genomic library, cloning, and (automatic) sequencing (Genome Analysis Basic, by S.B. Primrose, translated by Asao Fujiyama, 1996, Shupringer Fairlark, Tokyo). The sequence information of an immunoglobulin gene of mouse is available under the GenBank Accession Nos. NG004051 (mouse IgGλ) and VO1569 (mouse IgGκ constant region).

The term "a host embryo of a non-human animal devoid of certain cells and/or tissue" or "deficient host embryo" as used herein refers to a host embryo of a non-human animal to which pluripotent cell is to be injected and which is devoid of the certain cells and/or tissue.

The term "progeny" of a chimeric non-human animal as used herein refers to a non-human animal, which is obtained by mutually crossing chimeric non-human animals according to the present invention or by crossing a chimeric non-human animal according to the present invention with a cognate non-human animal, and which is capable of expressing a desired protein at least in the certain cells and/or tissue.

The term "phenotype" as used herein refers to a trait inherent in an animal or a trait of an animal emerging as a result of gene introduction.

The term "proliferative tumor cell" as used herein refers to a tumorigenic cell having permanent proliferative potency, e.g., plasmacytoma (or myeloma cells) which can produce immunoglobulins.

The term "hybridoma" as used herein refers to a hybrid cell obtainable by fusing a cell derived from the tissue or cell of a chimeric non-human animal according to the present invention or its progeny, with a proliferative tumor cell.

The term "targeting vector" as used herein refers to a vector having an expression unit of a gene encoding a desired protein. When the vector is introduced into a target chromosome region by means of homologous recombination, the desired protein is expressed. The term "knock out vector" as used herein refers to a vector for use in destroying or inactivating a desired gene of a non-human animal by homologous recombination. Furthermore, the term "knock out" or "gene knock out" refers to destroying or inactivating a target gene by introducing a structure for inhibiting the expression of the gene into a target locus by homologous recombination.

The term "recombining segment (RS) element" as used herein refers to the sequence: agtttctgcacgggcagtcagttagcagcactcactgtg (SEQ ID NO: 65), which is located about 25 Kb downstream of the immunoglobulin κ light chain constant region gene on the murine chromosome 6, and has nonamer and heptamer signal sequences (Daitch et al., J. Immunol., 149: 832-840, 1992). As a result of analysis, most of the B cells expressing λ chain are deficient in Cκ exon or Jκ-Cκ region. It is reported that this deficiency is due to recombination between said exon or region and a DNA sequence (i.e., recombining sequence or RS element) located 25 kb downstream of the Cκ exon on the murine chromosome 6 (Durdik et al., Nature, 307, 749-752, 1984; Moore et al., Proc. Natl. Acad. Sci. U.S.A.). Mammalian animals other than mice also have regions having functions equivalent to those of the RS element downstream of the genomic immunoglobulin genes. When foreign DNA is incorporated into the genome in a homologous manner, a drug resistant marker gene may be incorporated in this region as in the case of the RS element to improve the efficiency for homologous recombination of foreign DNA.

The present invention can give an advantage such that a chimeric non-human animal or its progeny according to the present invention, a cell or tissue obtained therefrom, or a hybridoma obtained therefrom enables foreign DNA to express at a level significantly higher, for example several hundred times higher, than a conventional technique.

This description includes part or all of the contents as disclosed in the description and/or drawings of Japanese Patent Application No. 2005-014826, which is a priority document of the present application.

### BRIEF DESCRIPTION OF THE DRAWINGS

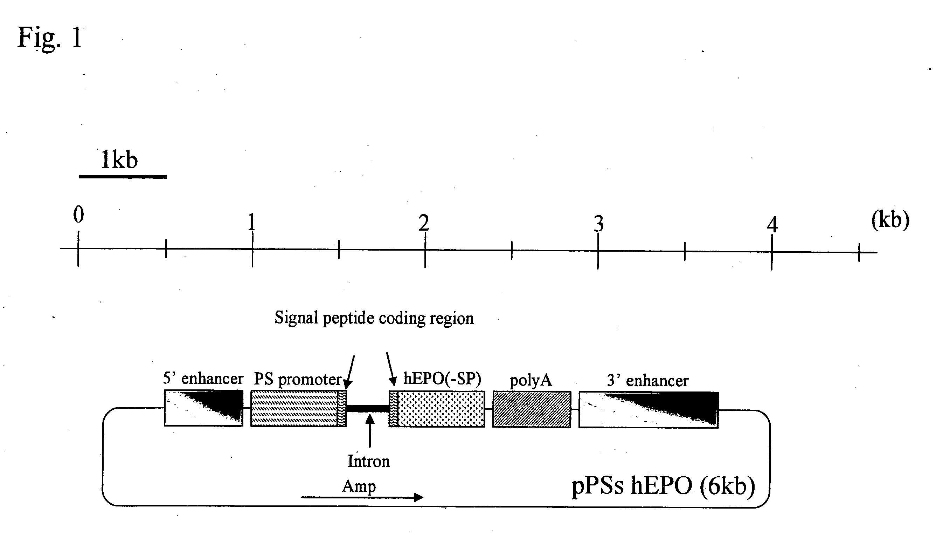

Fig. 1 shows the structure of the pPSs hEPO *in vitro* vector, wherein 5' enhancer is the 5' enhancer region of murine Igκ, PS promoter is the murine Igκ promoter region PS, signal peptide coding region is the murine Igκ signal peptide coding region downstream of the PS promoter, intron is the intron region sandwiched by the murine Igκ signal peptide coding regions, hEPO(-SP) is the human EPO gene containing no inherent signal peptide coding region, poly A is the murine Igκ polyA signal region, 3' enhancer is the 3' enhancer region of murine Igκ, and Amp is the ampicillin resistant gene.

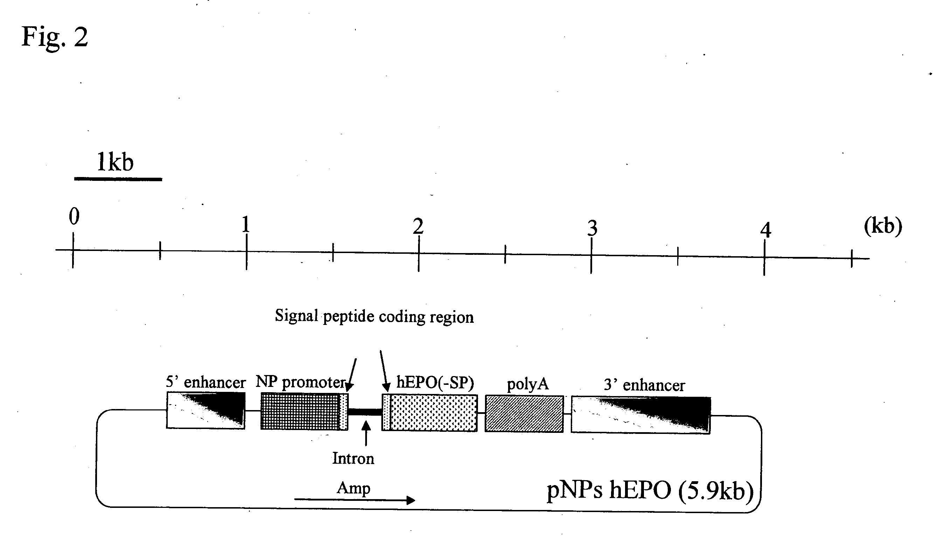

Fig. 2 shows the structure of the pNPs hEPO *in vitro* vector, wherein 5' enhancer is the 5' enhancer region of murine Igκ, NP promoter is the murine Igκ promoter region NP, signal peptide coding region is the murine Igκ signal peptide coding region downstream of NP promoter, intron is the intron region sandwiched by murine Igκ signal peptide coding regions, hEPO(-SP) is the human EPO gene containing no inherent signal peptide coding region, polyA is the murine Igκ poly A signal region, 3' enhancer is the 3' enhancer region of murine Igκ, and Amp is the ampicillin resistant gene.

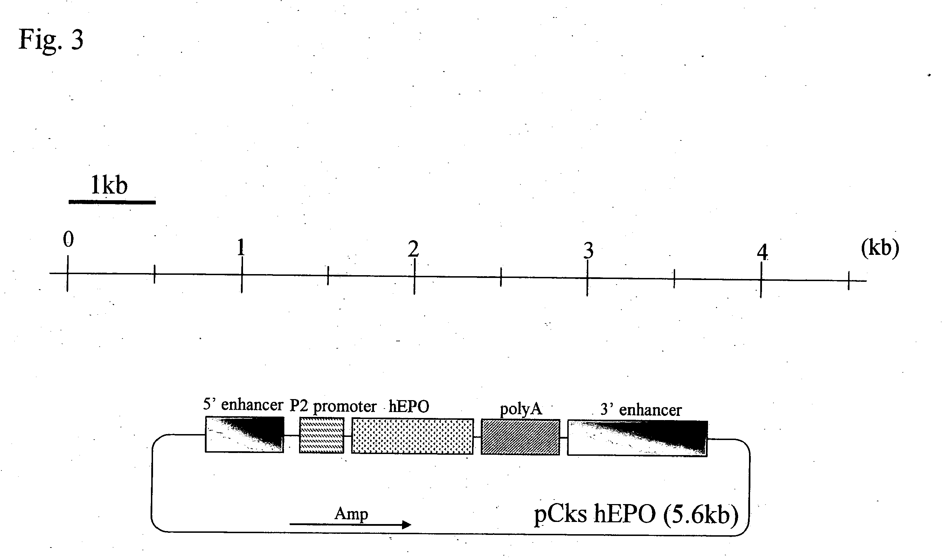

Fig. 3 shows the structure of the pCks hEPO *in vitro* vector, wherein 5' enhancer is the 5' enhancer region of murine Igκ, P2 promoter is the murine Igκ promoter region 2, hEPO is the human EPO gene, polyA is the murine Igκ polyA signal region, 3' enhancer is the 3' enhancer region of murine Igκ, and Amp is the ampicillin resistant gene.

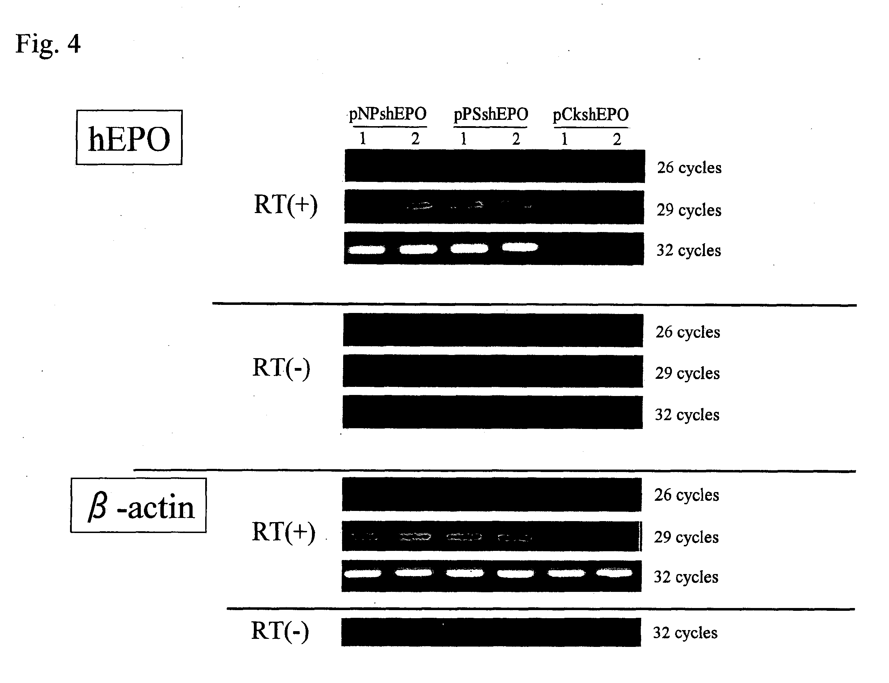

Fig. 4 shows the results of RT-PCR analysis obtained by introducing the pNPs *hEPO in vitro* vector, the pPSs hEPO *in vitro* vector, and pCks *hEPO in vitro* vector into myeloma cells, obtaining RNA therefrom, and using the obtained RNA to perform RT-PCR, wherein hEPO is the human EPO, and RT(-) is PCR without reverse transcription.

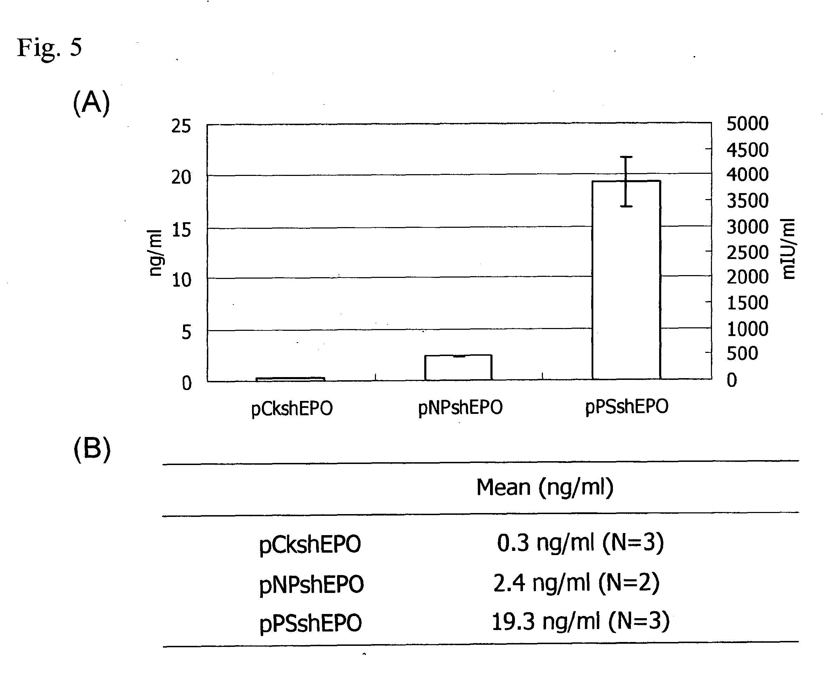

Fig. 5 shows the results of assaying the human EPO level in the culture supernatant obtained via ELISA after the pCks *hEPO in vitro* vector, the pNPs hEPO *in vitro* vector, and the pPSs hEPO *in vitro* vector had been introduced into myeloma cells. Fig. 5A is a bar chart showing the measured values, and Fig. 5B shows the average of the measured values.

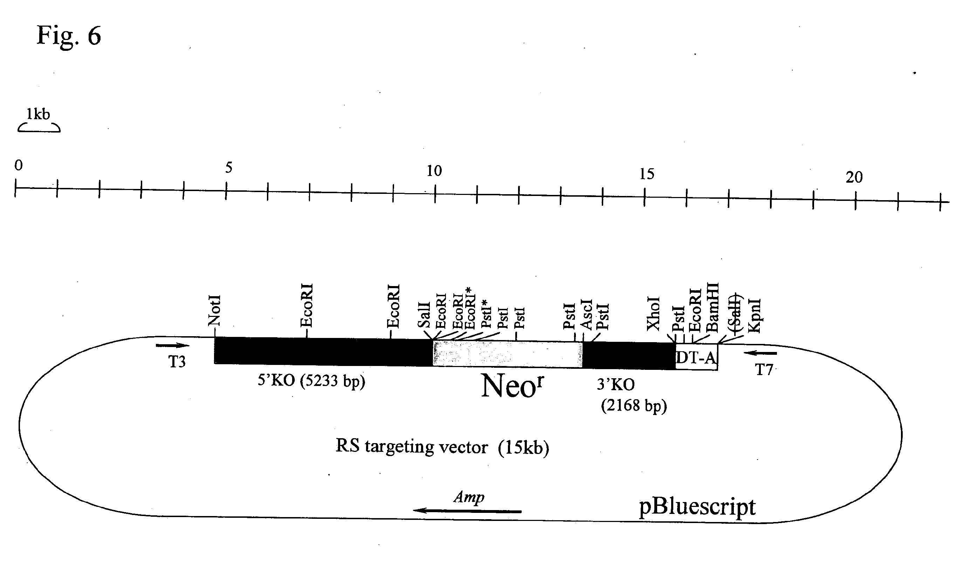

Fig. 6 shows the structure of a murine RS element targeting vector, pBlueRS-LoxP-Neo-DT-A-3' KO-5' KO, wherein 5' KO is the 5' region upstream of the murine RS element, Neo^{r} is a neomycin resistant gene comprising loxP sequences at both ends, 3' KO is the 3' region downstream of the murine RS element, DT-A is a diphtheria toxin A chain gene, and pBluescript is a cloning vector.

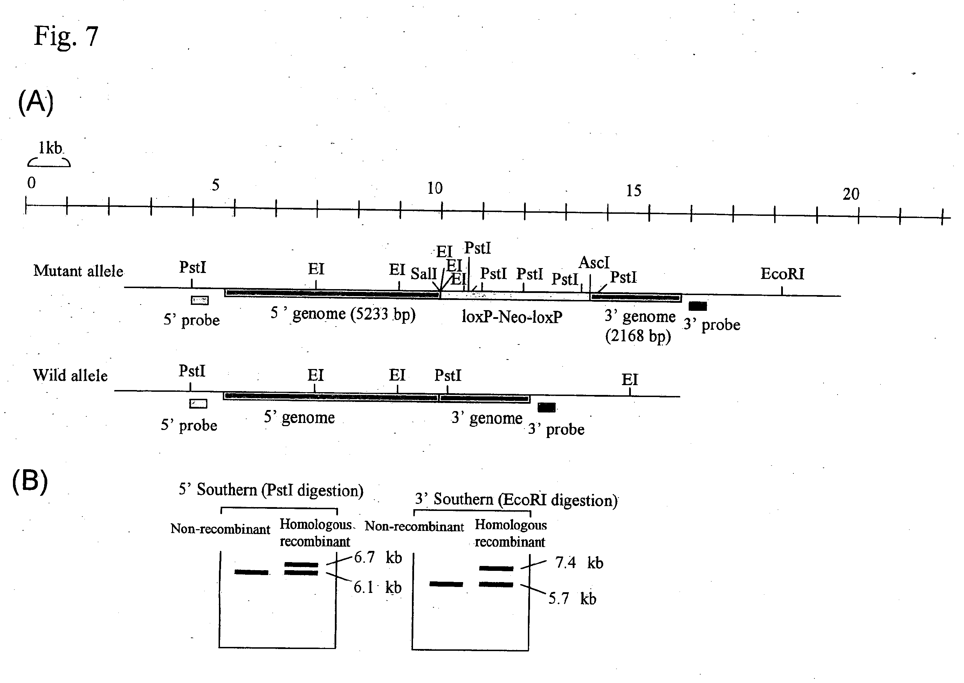

Fig. 7 shows the allelic structure in which the neomycin resistant gene has been inserted in place of the murine RS element (Fig. 7A), and the positions of probes for Southern analysis (Fig. 7B), wherein 5' genome is the 5' region upstream of the murine RS element, 3' genome is the 3' region downstream of the murine RS element, 5' probe is a probe for Southern analysis to confirm insertion of a targeting vector into the 5' side, 3' probe is a probe for Southern analysis to confirm insertion of a targeting vector into the 3' side, and loxP-neo-loxP is a neomycin resistant gene comprising loxP sequences at both ends.

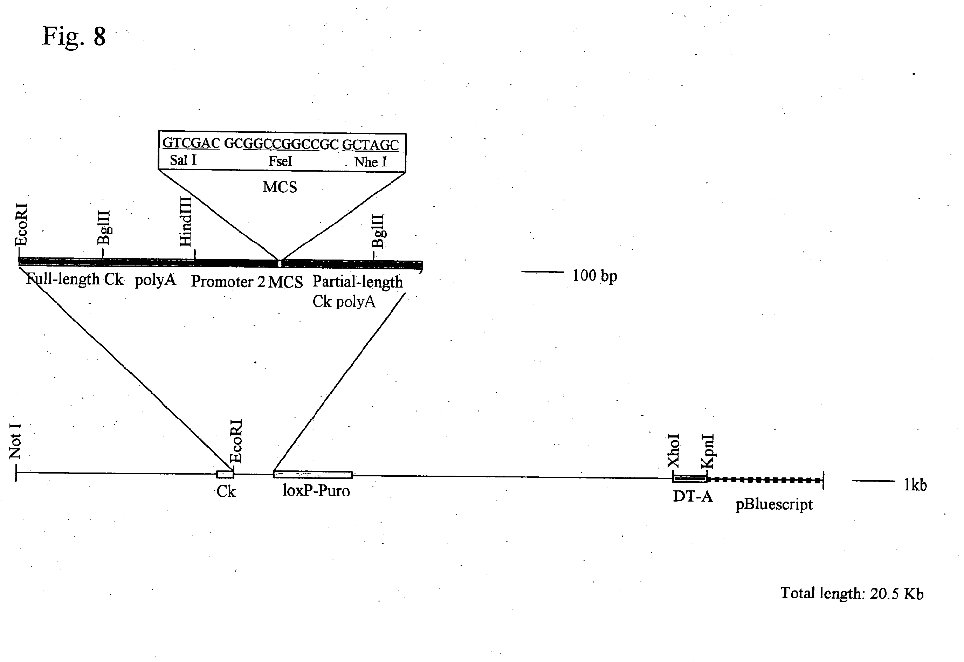

Fig. 8 shows the structure of a pCκP2 KI vector, wherein promoter 2 is the murine Igκ promoter region gene 2, MCS is a multicloning site, Cκ is the murine Igκ gene constant region, full-length Cκ polyA is a 436-bp murine Igκ polyA signal region downstream of the Cκ termination codon, partial-length Cκ polyA is a 309-bp murine Igκ polyA signal region downstream of the Cκ termination codon, loxP-Puro is a puromycin resistant gene comprising loxP sequences at both ends, DT-A is a diphtheria toxin A chain gene, and pBluescript is a cloning vector.

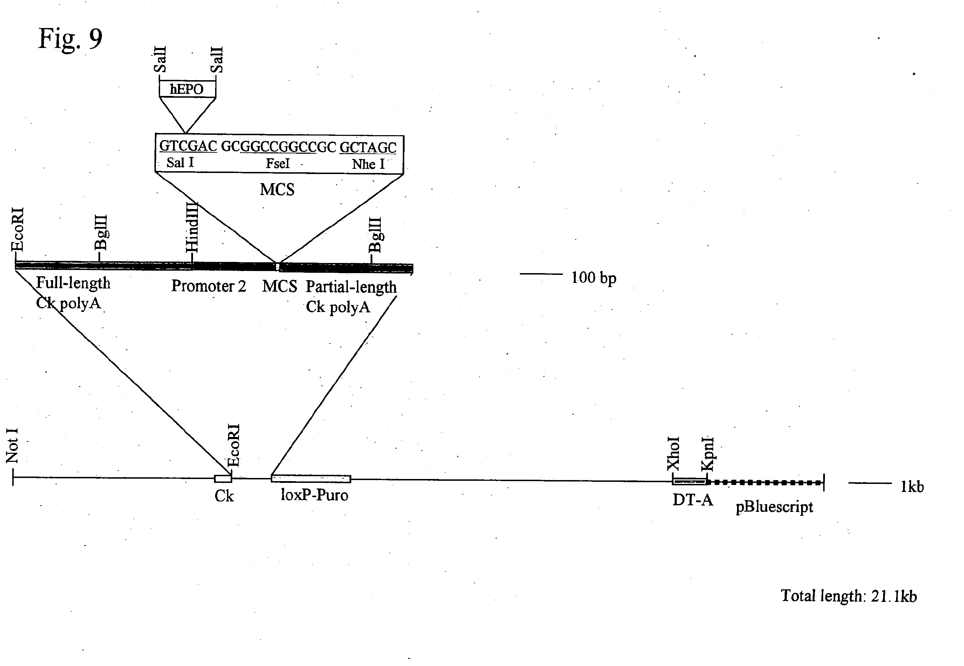

Fig. 9 shows the structure of a pCκP2 hEPO KI vector which has a human EPO gene inserted into the cloning site, wherein Promoter 2 is the murine Igκ promoter region gene 2, hEPO is a human EPO gene, Cκ is the murine Igκ gene constant region, full-length Cκ polyA is a 436-bp murine Igκ polyA signal region downstream of the Cκ termination codon, partial-length Cκ polyA is a 309-bp murine Igκ polyA signal region downstream of the Cκ termination codon, loxP-Puro is a puromycin resistant gene comprising loxP sequences at both ends, DT-A is a diphtheria toxin A chain gene, and pBluescript is a cloning vector.

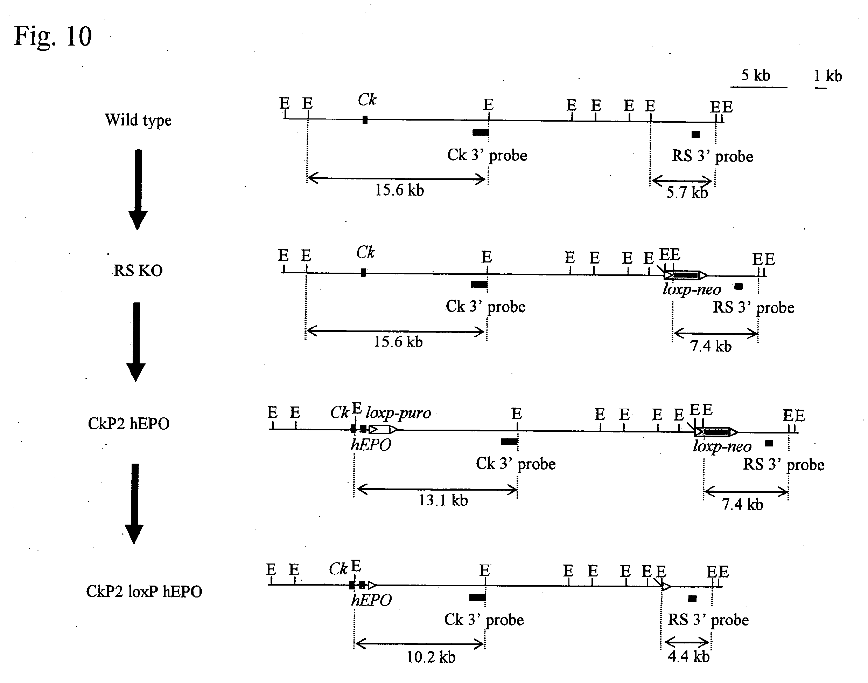

Fig. 10 shows the allelic structure targeted by the drug resistant gene (loxp-neo), the allelic structure targeted by the human EPO gene and the drug resistant gene (loxp-puro) with the use of the pCk hEPO KI vector, the allelic structure from which two types of drug resistant genes (loxp-neo and loxp-puro) have been simultaneously removed, and the position of the probe for Southern analysis, wherein hEPO is a human EPO gene, Cκ is the murine Igκ gene constant region, loxp-puro is the puromycin resistant gene comprising loxP sequences at both ends, loxp-neo is the neomycin resistant gene comprising loxP sequences at both ends, Ck3' probe is the probe for Southern analysis for selecting clones into which the hEPO+loxp-puro genes have been introduced and from which the loxp-puro genes have been removed, RS3' probe is the probe for Southern analysis for selecting clones into which the loxp-neo genes have been introduced and from which the genes have been removed, and E is an EcoRI restriction site.

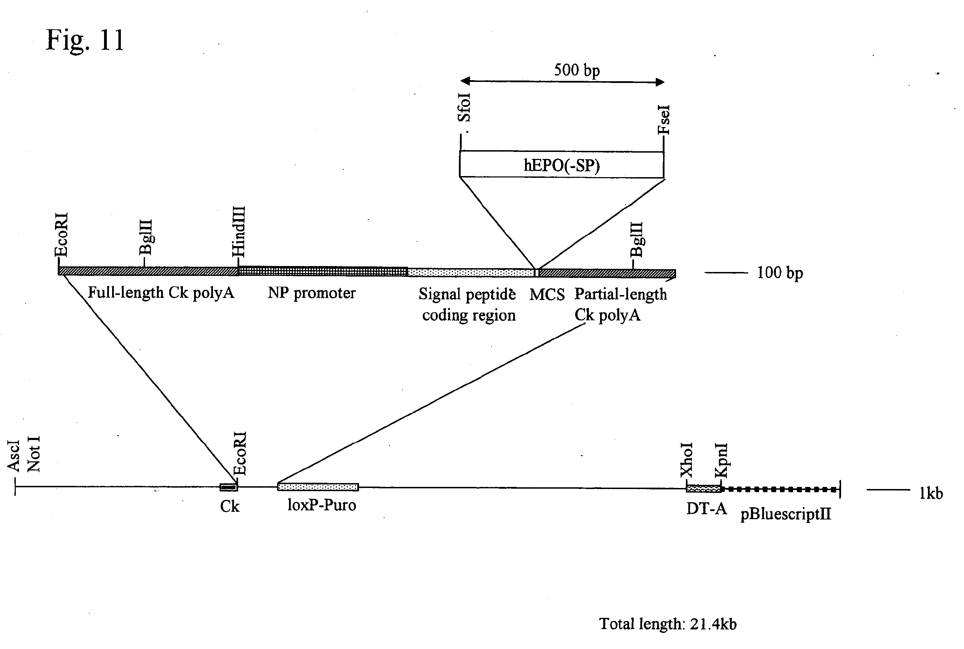

Fig 11 shows the structure of the pNP hEPO KI vector comprising the human EPO gene, having no inherent signal peptide coding region, inserted into the cloning site, wherein NP promoter is the murine Igκ promoter region NP, signal peptide coding region is the murine Igκ signal peptide coding region downstream of the NP promoter, hEPO(-SP) is the human EPO gene having no inherent signal peptide coding region, full-length Cκ polyA is the a 436-bp murine Igκ polyA signal region downstream of the Cκ termination codon, partial-length Cκ polyA is a 309-bp murine Igκ polyA signal region downstream of the Cκ termination codon, Cκ is the murine Igκ gene constant region, loxp-Puro is the puromycin resistant gene comprising loxP sequences at both ends, DT-A is a diphtheria toxin A chain gene, and pBluescript is a cloning vector.

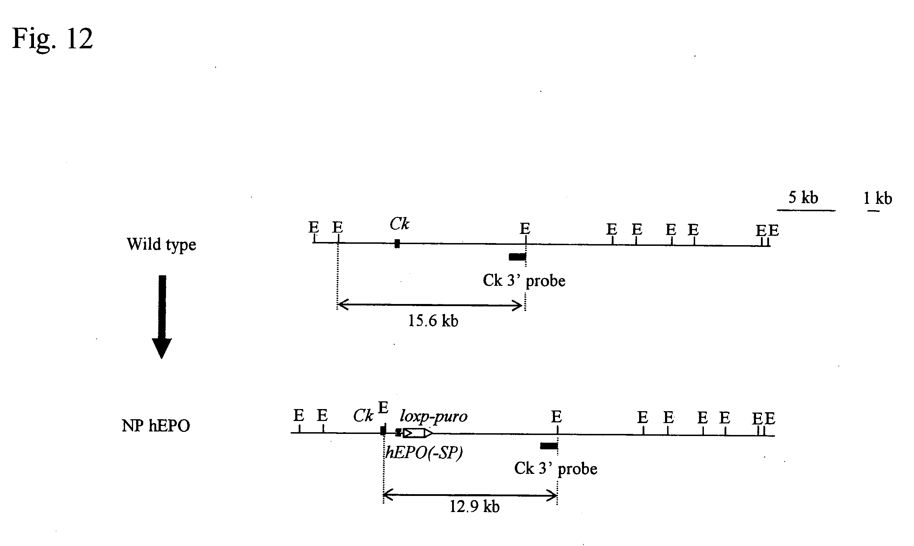

Fig. 12 shows the allelic structure targeted by the human EPO gene and the drug resistant gene (loxp-puro) using the pNP hEPO KI vector and the position of a probe for Southern analysis, wherein hEPO(-SP) is the human EPO gene having no inherent signal peptide coding region, Cκ is the murine Igκ gene constant region, loxp-puro is the puromycin resistant gene comprising loxP sequences at both ends, Ck3' probe is a probe for Southern analysis for selecting the clones into which the hEPO(-SP)+loxp-puro genes have been introduced, and E is an EcoRI restriction site.

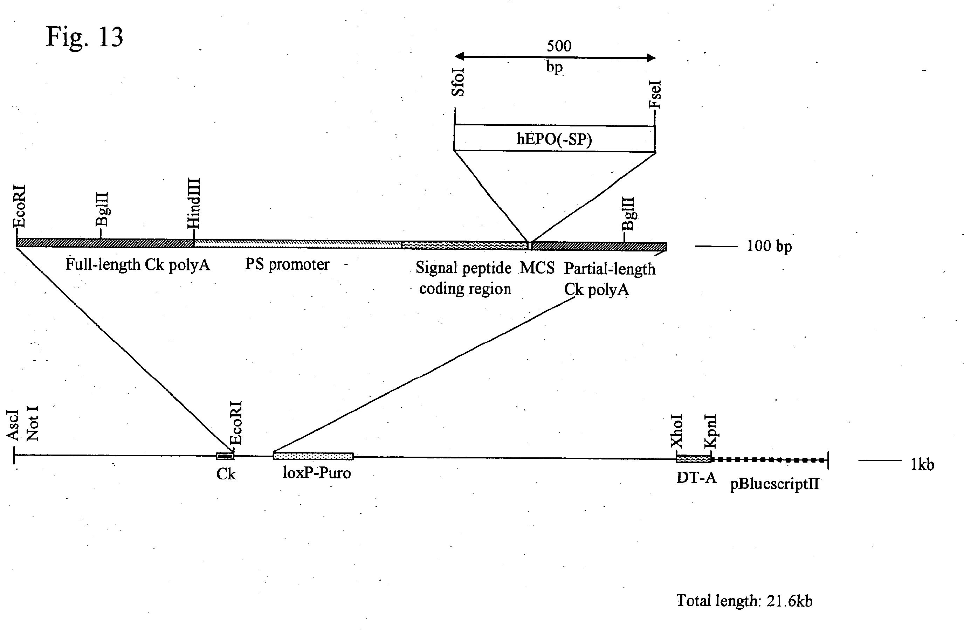

Fig. 13 shows the structure of the pPS hEPO KI vector comprising the human EPO gene, having no inherent signal peptide coding region, inserted into the cloning site, wherein PS promoter is the murine Igκ promoter region PS, signal peptide coding region is the murine Igκ signal peptide coding region downstream of the PS promoter, hEPO(-SP) is the human EPO gene having no inherent signal peptide coding region, full-length Cκ polyA is a 436-bp murine Igκ polyA signal region downstream of the Cκ termination codon, partial-length Cκ polyA is a 309-bp murine Igκ polyA signal region downstream of the Cκ termination codon, Cκ is the murine Igκ gene constant region, loxp-Puro is the puromycin resistant gene comprising loxP sequences at both ends, DT-A is a diphtheria toxin A chain gene, and pBluescript is a cloning vector.

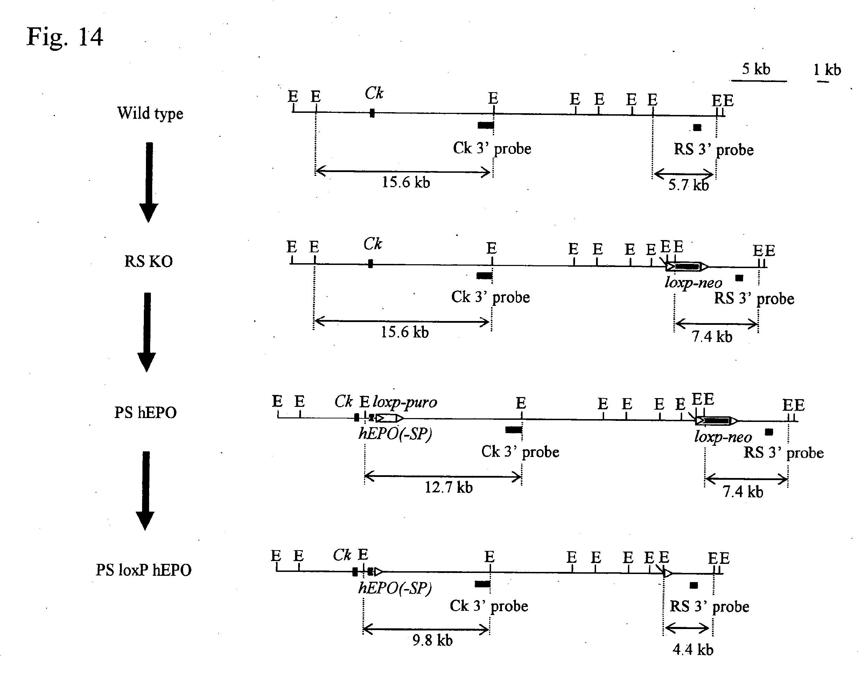

Fig. 14 shows the allelic structure targeted by the drug resistant gene (loxp-neo), the allelic structure targeted by the human EPO gene and the drug resistant gene (loxp-puro) with the use of the pPS hEPO KI vector, the allelic structure from which drug resistant genes (loxp-neo and loxp-puro) have been simultaneously removed, and the position of the probe for Southern analysis, wherein hEPO(-SP) is the human EPO gene from which the inherent leader sequence coding region has been removed, Cκ is the murine Igκ gene constant region, loxp-puro is the puromycin resistant gene comprising loxP sequences at both ends, loxp-neo is the neomycin resistant gene comprising loxP sequences at both ends, Ck3' probe is a probe for Southern analysis for selecting clones into which the hEPO(-SP)+loxp-puro genes have been introduced and from which the loxp-puro genes have been removed, RS3' probe is a probe for Southern analysis for selecting clones into which the loxp-neo gene have been introduced and from which the genes have been removed, and E is an EcoRI restriction site.

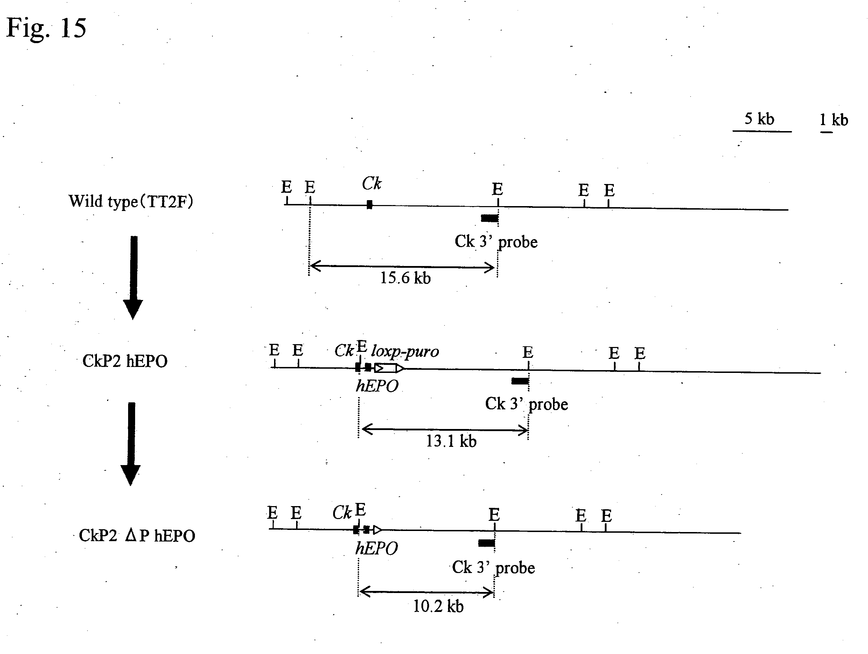

Fig. 15 shows the allelic structure targeted by the drug resistant gene (loxp-neo), the allelic structure targeted by the human EPO gene and the drug resistant gene (loxp-puro) using the pCkP2 hEPO KI vector, the allelic structure from which the drug resistant genes (loxp-neo and loxp-puro) have been removed, and the position of the probe for Southern analysis, wherein hEPO is a human EPO gene, Cκ: is the murine Igκ gene constant region, loxp-puro is the puromycin resistant gene comprising loxP sequences at both ends, Ck3' probe is a probe for Southern analysis for selecting clones into which the hEPO+loxp-puro genes have been introduced and from which the loxp-puro genes have been removed, and E is an EcoRI restriction site.

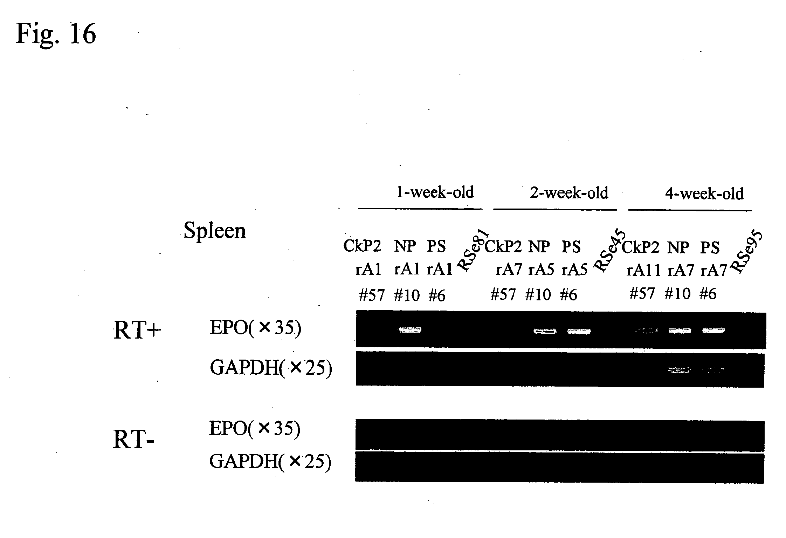

Fig. 16 shows the results of RT-PCR analysis obtained by using RNA prepared from the spleens of chimeric mice (1-week-old to 4-week-old) prepared from the CkP2 hEPO murine ES cell, the NP hEPO murine ES cell, the PS hEPO murine ES cell, and the RS element targeting murine ES cell (indicating an agarose gel electrophoretic band), wherein CkP2 is the results concerning the samples obtained from spleen of a chimeric mouse prepared from the CkP2 hEPO murine ES cell; NP is the results concerning the samples obtained from spleen of a chimeric mouse prepared from the NP hEPO murine ES cell; PS is the results concerning the samples obtained from spleen of a chimeric mouse prepared from the PS hEPO murine ES cell; RSe is the results concerning the samples obtained from spleen of a control chimeric mouse prepared from the RS element targeting murine ES cell; EPO is human EPO; GAPDH is murine glyceraldehyde-3-phosphate dehydrogenase; RT+ is reverse transcription followed by PCR; and RT- is PCR without reverse transcription.

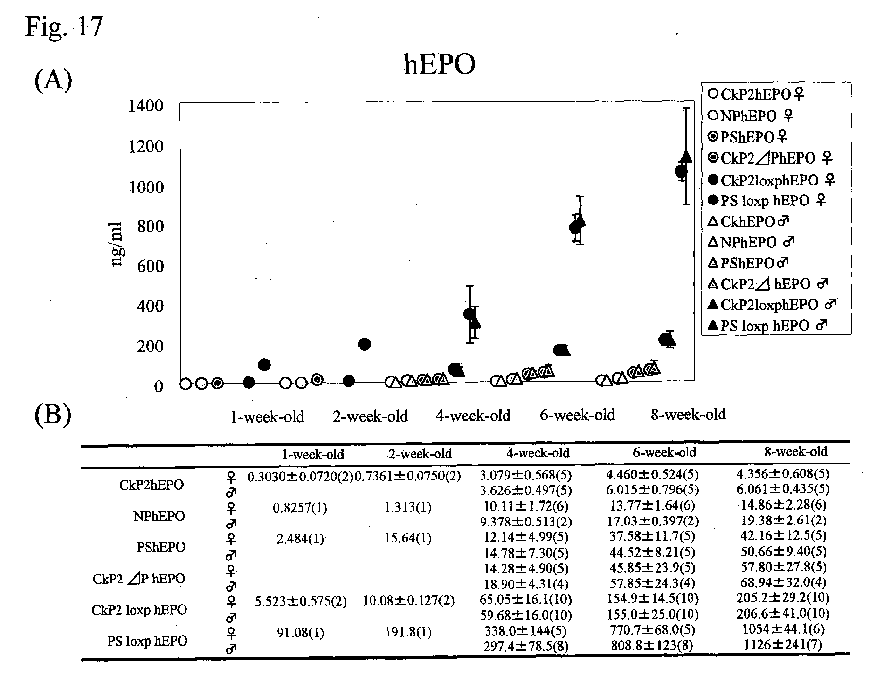

Fig. 17 shows the serum human EPO levels of the CkP2 hEPO murine ES cell-derived chimeric mouse, the NP hEPO murine ES cell-derived chimeric mouse, the PS hEPO murine ES cell-derived chimeric mouse, the CkP2 ΔP EPO murine TT2F cell-derived chimeric mouse, the CkP2 loxP hEPO murine ES cell-derived chimeric mouse, and the PS loxP hEPO murine ES cell-derived chimeric mouse from 1-week-old to 8-week-old. Fig. 17A is a chart of the measured values and Fig. 17B shows measured values, wherein a numerical value is the number of specimens.

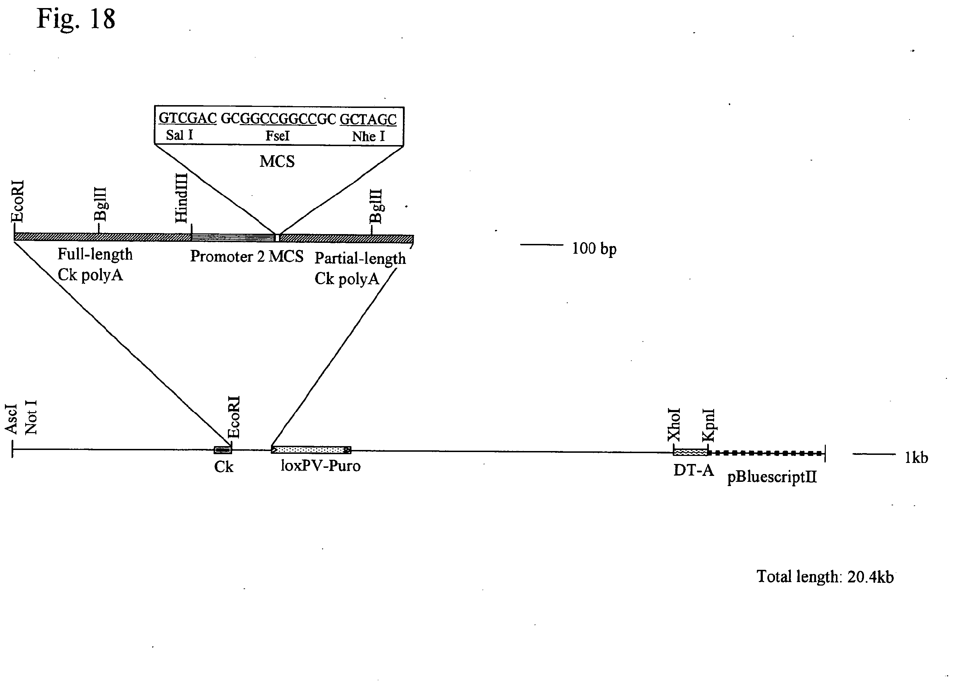

Fig. 18 shows the structure of the pCk loxPV KI vector, wherein promoter 2 is the murine Igκ promoter region 2, MCS is the multicloning site, Cκ is the murine Igκ gene constant region, full-length Cκ polyA is a 436-bp murine Igκ polyA signal region downstream of the Cκ termination codon, partial-length Cκ polyA is a 309-bp murine Igκ polyA signal region downstream of the Cκ termination codon, loxPV-Puro is the puromycin resistant gene having mutant loxP sequences at both ends, DT-A is a diphtheria toxin A chain gene, and pBluescript is a cloning vector.

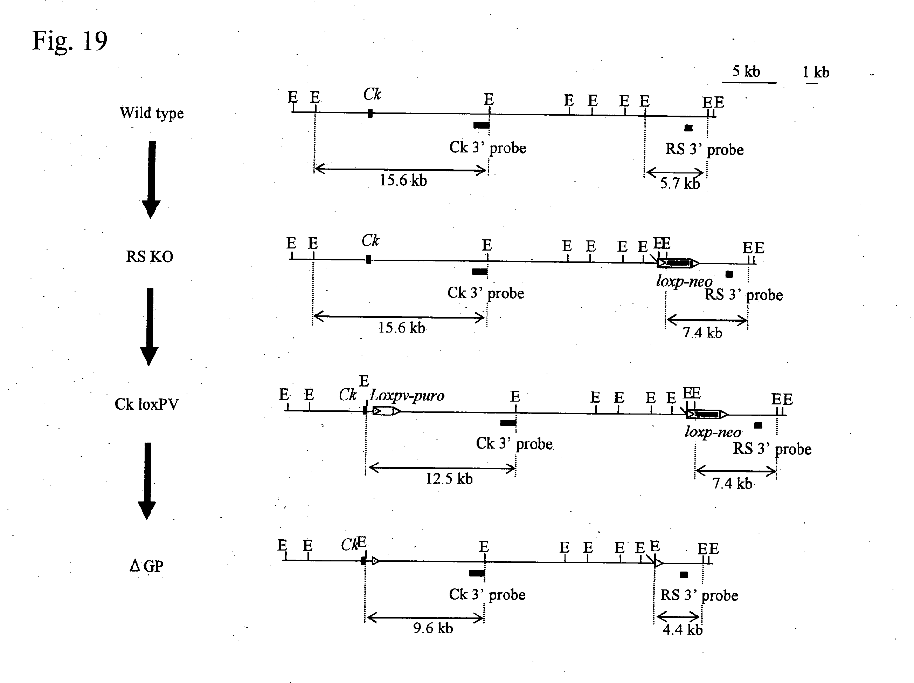

Fig. 19 shows the allelic structure targeted by the drug resistant gene (loxp-neo), the allelic structure targeted by the drug resistant gene (loxpv-puro) using the pCk loxPV KI vector, the allelic structure from which the drug resistant genes (loxp-neo and loxpv-puro) have been removed, and the position of a probe for Southern analysis, wherein Cκ is the murine Igκ gene constant region, loxpv-puro is the puromycin resistant gene comprising loxPV sequences, which are partially mutated loxP sequences, at both ends; loxp-neo is the neomycin resistant gene comprising loxP sequences at both ends, Ck3' probe is a probe for Southern analysis for selecting clones into which the loxpv-puro gene has been introduced and from which the loxpv-puro gene has been removed, RS3' probe is a probe for Southern analysis for selecting clones into which the loxp-neo gene has been introduced and from which the gene has been removed, and E is an EcoRI restriction site.

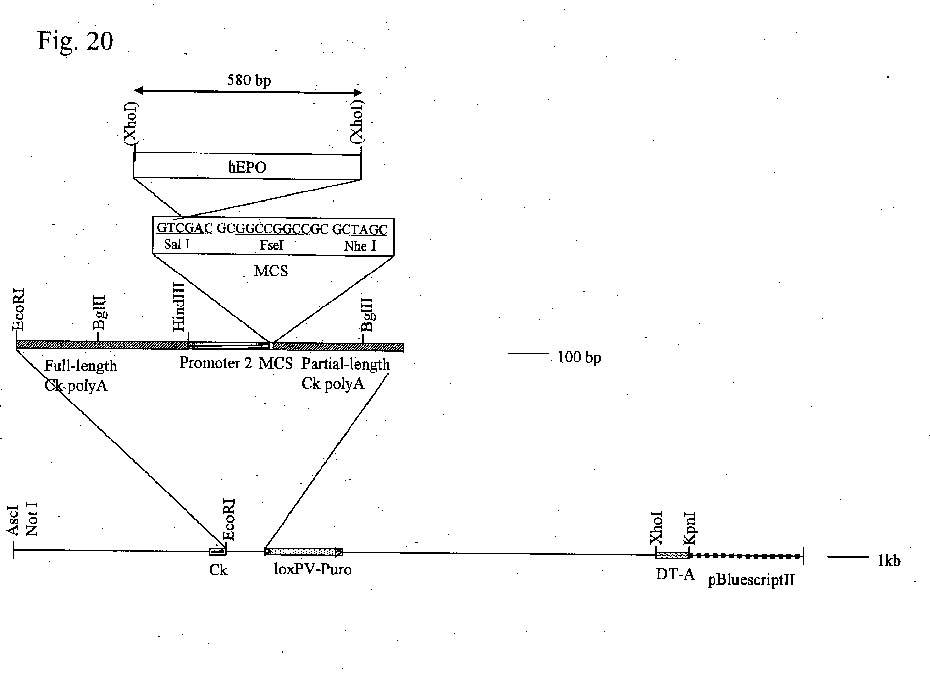

Fig. 20 shows the structure of the pCk loxPV hEPO KI vector comprising the human EPO gene inserted into the cloning site, wherein promoter 2 is the murine Igκ promoter region 2, hEPO is a human EPO gene, Cκ is the murine Igκ gene constant region, full-length Cκ polyA is a 436-bp murine Igκ polyA signal region downstream of the Cκ termination codon, partial-length Cκ polyA is a 309-bp murine Igκ polyA signal region downstream of the Cκ termination codon, loxPV-Puro is the puromycin resistant gene comprising loxPV sequences, which are partially mutated loxP sequences, at both ends, DT-A is a diphtheria toxin A chain gene, and pBluescript is a cloning vector.

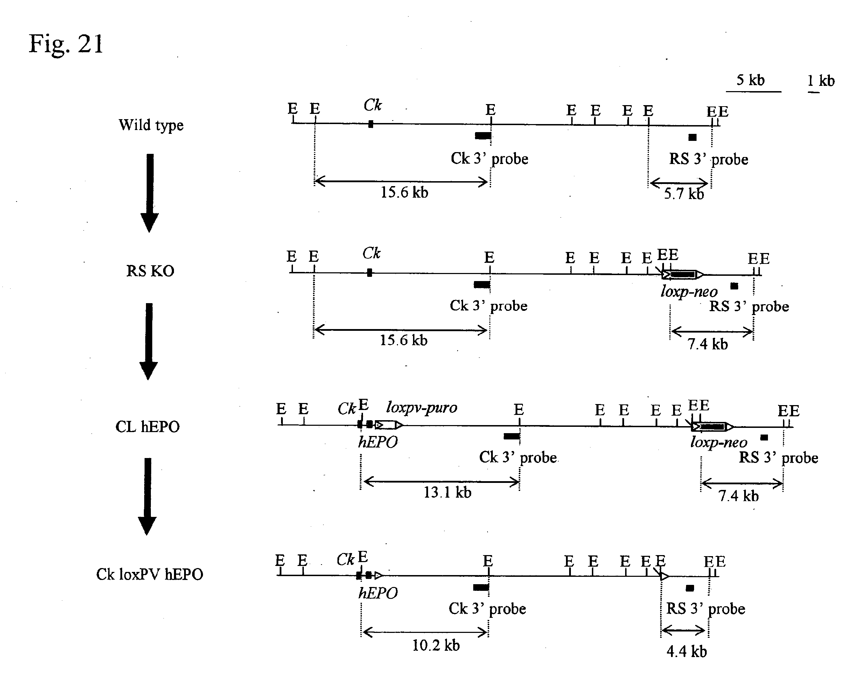

Fig. 21 shows the allelic structure targeted by the drug resistant gene (loxp-neo), the allelic structure targeted by the human EPO gene and the drug resistant gene (loxpv-puro) using the pCk loxPV hEPO KI vector, the allelic structure from which the drug resistant genes (loxp-neo and loxpv-puro) have been removed, and the position of a probe for Southern analysis, wherein hEPO is a human EPO gene, Cκ is the murine Igκ gene constant region, loxpv-puro is the puromycin resistant gene comprising loxPV sequences, which are partially mutated loxP sequences, at both ends, loxp-neo is the neomycin resistant gene comprising loxP sequences at both ends, Ck3' probe is a probe for Southern analysis for selecting clones into which the hEPO+loxpv-puro gene has been introduced and from which the loxpv-puro gene has been removed, RS3' probe is a probe for Southern analysis for selecting clones into which the loxp-neo gene has been introduced and from which the gene has been removed, and E is an EcoRI restriction site.

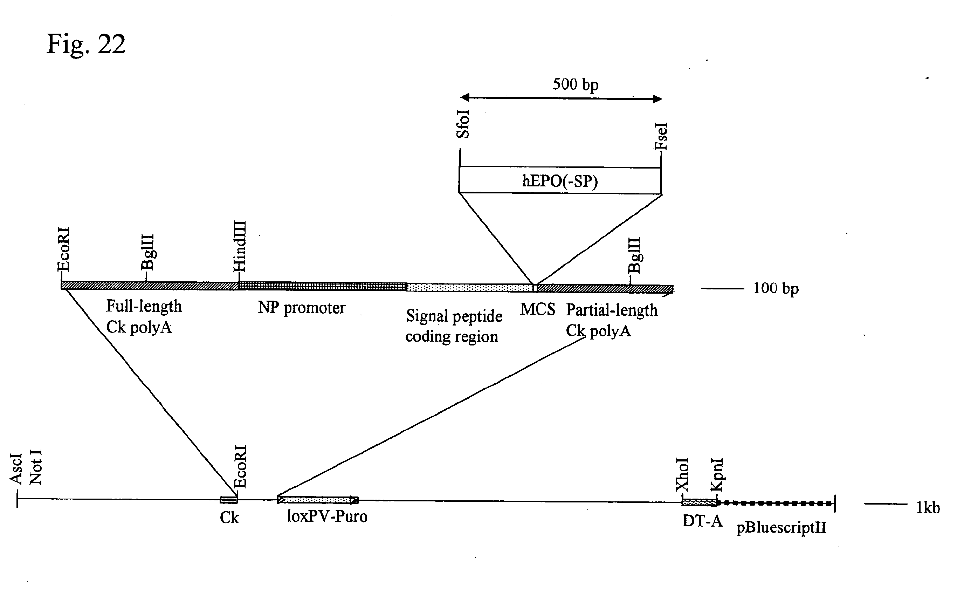

Fig. 22 shows the structure of the pNP loxPV hEPO KI vector comprising the human EPO gene inserted into the cloning site, wherein NP promoter is the murine Igκ promoter region comprising an about 300-bp longer upstream sequence than the promoter 2, signal peptide coding region is the murine Igκ signal peptide coding region downstream of the NP promoter, hEPO(-SP) is the human EPO gene having no inherent signal peptide coding region, full-length Cκ polyA is a 436-bp murine Igκ polyA signal region downstream of the Cκ termination codon, partial-length Cκ polyA is a 309-bp murine Igκ polyA signal region downstream of the Cκ termination codon, loxPV-Puro is the puromycin resistant gene comprising loxPV sequences, which are partially mutated loxP sequences, at both ends, DT-A is a diphtheria toxin A chain gene, and pBluescript is a cloning vector.

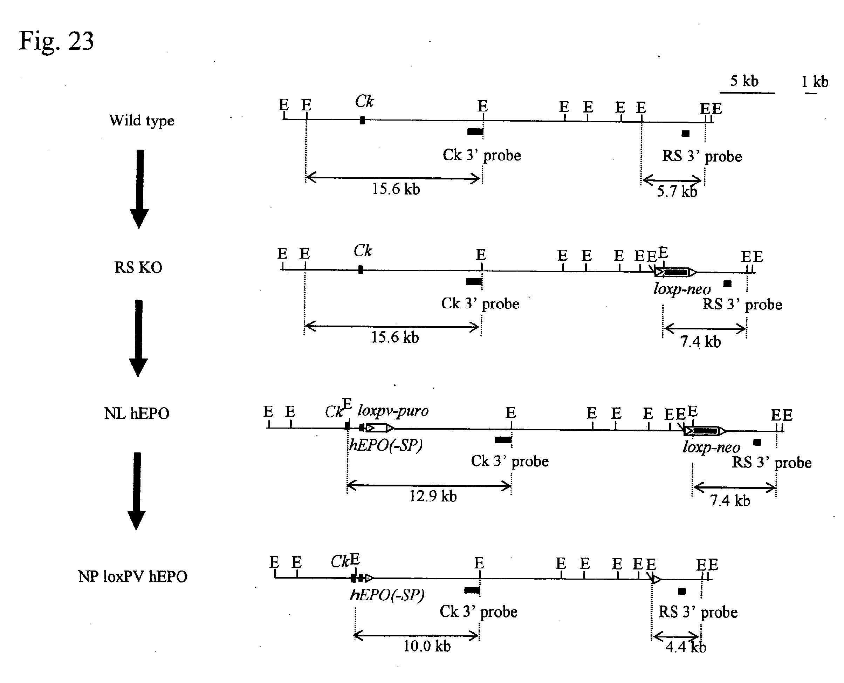

Fig. 23 shows the allelic structure targeted by the drug resistant gene (loxp-neo), the allelic structure targeted by the human EPO gene and the drug resistant gene (loxpv-puro) using the pNP loxPV hEPO KI vector, the allelic structure from which the drug resistant genes (loxp-neo and loxpv-puro) have been removed, and the position for a probe for Southern analysis, wherein hEPO(-SP) is the human EPO gene having no inherent signal peptide coding region, Cκ is the murine Igκ gene constant region, loxpv-puro is the puromycin resistant gene comprising loxPV sequences, which are partially mutated loxP sequences, at both ends, loxp-neo is the neomycin resistant gene comprising loxP sequences at both ends, Ck3' probe is a probe for Southern analysis for selecting clones into which the hEPO+loxpv-puro gene has been introduced and from which the loxpv-puro gene has been removed, RS3' probe is a probe for Southern analysis for selecting clones into which the loxp-neo gene has been introduced and from which the gene has been removed, and E is an EcoRI restriction site.

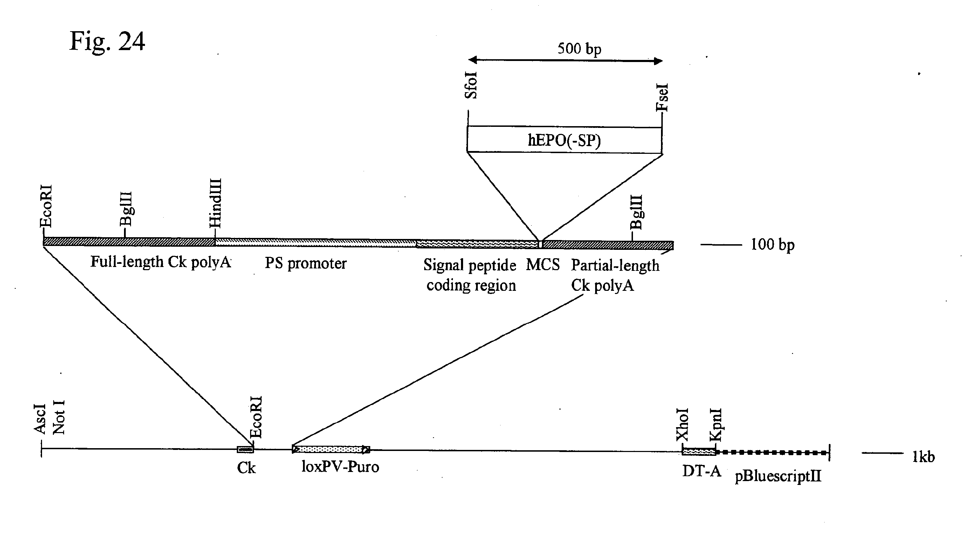

Fig. 24 shows the structure of the pUS hEPO KI vector comprising the human EPO gene inserted into the cloning site, wherein PS promoter is the murine Igκ promoter region PS, signal peptide coding region is the murine Igκ signal peptide coding region downstream of the PS promoter, hEPO(-SP) is the human EPO gene having no inherent signal peptide coding region, Cκ is the murine Igκ gene constant region, full-length Cκ polyA is a 436-bp murine Igκ polyA signal region downstream of the Cκ termination codon, partial-length Cκ polyA is a 309-bp murine Igκ polyA signal region downstream of the Cκ termination codon, loxPV-Puro is the puromycin resistant gene comprising loxPV sequences, which are partially mutated loxP sequences, at both ends, DT-A is a diphtheria toxin A chain gene, and pBluescript is a cloning vector.

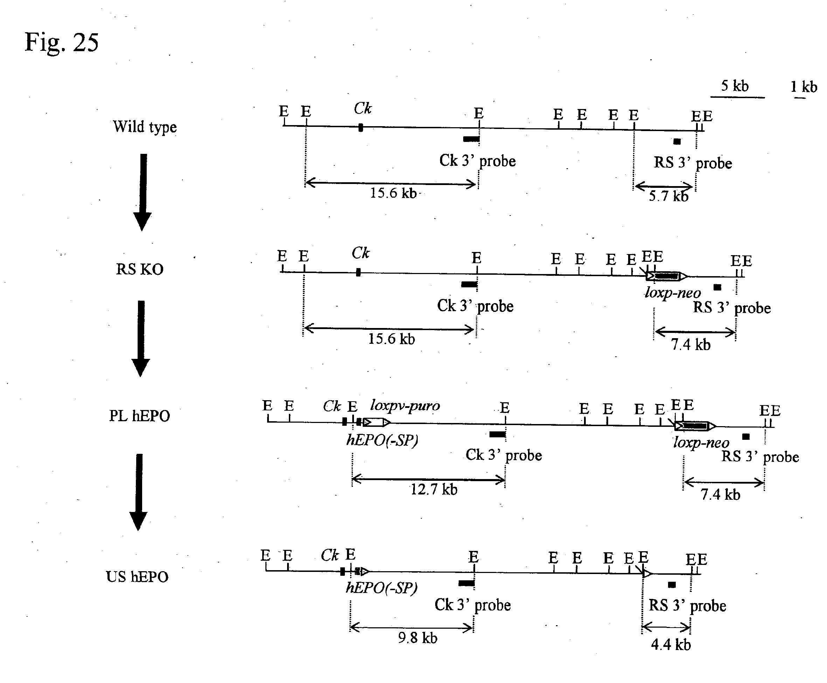

Fig. 25 shows the allelic structure targeted by the drug resistant gene (loxp-neo), the allelic structure targeted by the human EPO gene and the drug resistant gene (loxpv-puro) using the pPS loxPV hEPO KI vector, the allelic structure from which the drug resistant genes (loxp-neo and loxpv-puro) have been removed, and the position for a probe for Southern analysis, wherein hEPO(-SP) is the human EPO gene having no inherent signal peptide coding region, Cκ is the murine Igκ gene constant region, loxpv-puro is the puromycin resistant gene comprising loxPV sequences, which are partially mutated loxP sequences, at both ends, loxp-neo is the neomycin resistant gene comprising loxP sequences at both ends, Ck3' probe is a probe for Southern analysis for selecting clones into which the hEPO+loxpv-puro gene has been introduced and from which the loxpv-puro gene has been removed, RS3' probe is a probe for Southern analysis for selecting clones into which the loxp-neo gene has been introduced and from which the gene has been removed, and E is an EcoRI restriction site.

### PREFERRED EMBODIMENTS OF THE INVENTION

The present invention will be described in more detail below.

### 1. Preparation of pluripotent cells comprising a drug resistant marker gene expression unit inserted into a given chromosome region

In the method of producing a chimeric non-human animal according to the present invention, pluripotent cells are first prepared, which are derived from a non-human animal and comprise (or comprise in a genomic level) a nucleic acid sequence (e.g., a structural gene) encoding a desired protein. The nucleic acid sequence is arranged such that the expression of the desired protein can be controlled by the control region of a gene to be expressed in the certain cells and/or tissue.

In the present invention, pluripotent cells as defined above can be used. A murine embryonic stem (ES) cell is preferable. A chromosome region to be modified is preferably the RS element, and particularly preferably the RS element located about 25 kb downstream of the immunoglobulin κ light chain constant region gene of mouse chromosome 6.

The gene that is expressed in certain cells and/or tissue may be expressed in a tissue-specific manner, or such gene may be constitutively expressed.

In the present invention, the expression unit containing cDNA ligated to the promoter of the gene to be expressed in certain cells and/or tissue is inserted into a site in the vicinity of the gene to be expressed in certain cells and/or tissue. Thus, such cDNA can be expressed in certain cells and/or tissue. As the gene to be expressed in certain cells and/or tissue, a foreign genetic factor that can be maintained outside the chromosome of a non-human animal, such as a gene contained in a plasmid vector (Elbrecht et al., Mol. Cell Biol., 7: 1276-1279, 1987), a human chromosome or a fragment thereof (Tomizuka et al., Nat. Genet. 16 133-143, 1997; Tomizuka et al., Proc. Natl. Acad. Sci. U.S.A. , 97, 722-727, 2000), or an artificial human chromosome vector (Kuroiwa et al., Gene Therapy, 9, 708-712, 2002), can be used, as well as the endogenous gene on the non-human animal genome. Further, a gene on a foreign gene fragment inserted into the chromosome of a non-human animal can also be used (Mendez et al., Nat. Genet., 15, 146-156, 1997). For example, Tomizuka et al. reported a transchromosomic mouse line that has and genetically transmits the human chromosome 14 fragment (SC20) (Proc. Natl. Acad. Sci., U.S.A., 97, 722-727, 2000). The SC20 fragment comprises the entire human immunoglobulin heavy chain locus. In the aforementioned transchromosomic mouse, a variety of human immunoglobulin heavy chain genes are found to be expressed in a B lymphocyte-specific manner as with the case of humans. That is, insertion of the expression unit comprising the immunoglobulin heavy chain promoter and cDNA ligated thereto in the vicinity of the human immunoglobulin heavy chain locus on the SC20 fragment enables expression of such cDNA in a B lymphocyte-specific manner.

Examples of such a gene expressed tissue-specifically include immunoglobulin light chain or heavy chain gene, T cell receptor gene, myoglobin gene, crystalline gene, rennin gene, lipase gene, and albumin gene An example of such a gene constitutively expressed is hypoxanthine guanine phosphoribosyl transferase (HPRT) gene. When the gene is expressed in a chimeric non-human animal tissue-specifically, an embryo devoid of the cell and/or tissue expressed by the gene can be employed as the host embryo (described later). When the gene is constitutively expressed, the embryo to be employed may be devoid of any cell and/or tissue.

The arrangement (alternatively, ligation or insertion) of a nucleic acid sequence encoding a desired protein is required to be performed such that the expression of the desired protein can be controlled at least by the control region of the gene to be expressed in certain cells and/or tissue. Accordingly, the nucleic acid sequence is arranged downstream of the control region of the gene to be expressed in certain cells and/or tissue.

Alternatively, the nucleic acid sequence encoding a desired protein is arranged as follows; an internal ribosomal entry site (IRES) is interposed between the termination codon of the gene to be expressed in certain cells and/or tissue and a sequence encoding a polyA signal region, and the nucleic acid sequence encoding a desired protein is arranged downstream of the IRES. More specifically, the nucleic acid sequence is present between the termination codon of the gene to be expressed in certain cells and/or tissue and the sequence encoding the polyA signal region while being functionally ligated with the IRES in a genomic level. Examples of the polyA signal region usable in constructing a targeting vector include, but are not limited to, a polyA signal region of the gene to be expressed in certain cells and/or tissue and another polyA sequences known in the art such as polyA signal region derived from simian virus 40 (SV40).

Alternatively, a nucleic acid sequence encoding a desired protein may be arranged as follows: a sequence encoding a second polyA signal region is arranged between the termination codon of the gene to be expressed in certain cells and/or tissue and the aforementioned sequence encoding the polyA signal region; a promoter sequence is arranged downstream of the second polyA signal region; and the nucleic acid sequence encoding a desired protein is arranged downstream of the promoter sequence. More specifically, the nucleic acid sequence is present on the genome while being functionally ligated with the promoter sequence and the sequence encoding the polyA signal region; at the same time, a gene(s) inherently present on the genome and expressed in certain cells and/or tissue is also functionally ligated with the promoter sequence and the sequence encoding the polyA signal region. The promoter sequence used in constructing the targeting vector is not particularly limited as long as it controls the expression of a gene in certain cells and/or tissue. Preferably, use may be made of the promoter for the gene to be expressed in the aforementioned certain cells and/or tissue. Where two promoters are present in a targeting vector, these two promoters may be the same or different as long as they control the expression of the gene in the same cell and/or tissue. Furthermore, the sequence encoding the polyA signal region used in constructing a targeting vector is not particularly limited as long as it is a sequence encoding a polyA signal region known in the art. Examples of the polyA signal region include a polyA signal region derived from the same origin of the promoter or a polyA signal region derived from simian virus 40 (SV40). As in the promoter, when two sequences encoding polyA signal regions are present in the targeting vector, these sequences may be the same or different.

Furthermore, the nucleic acid sequence encoding a desired protein may be arranged downstream of a polyA signal region of the gene to be expressed in the certain cells and/or tissue in the order of the promoter sequence, the nucleic acid sequence, and the sequence encoding a polyA signal region. More specifically, the nucleic acid sequence may be present downstream of the polyA signal of the gene to be expressed in the certain cells and/or tissue while it is functionally ligated (in a cassette format, for example) to both the promoter and the sequence encoding the polyA signal region. The promoter sequence used in constructing a targeting vector is not particularly limited as long as it controls the expression of the gene in certain cells and/or tissue. Preferably, use may be made of the promoter of the gene to be expressed in the aforementioned certain cells and/or tissue. Furthermore, the sequence encoding the polyA signal region in constructing a targeting vector is not particularly limited as long as it is the sequence of a polyA signal region known in the art. Examples of the polyA signal region include a polyA signal region derived from the same origin as the promoter and a polyA signal region derived from simian virus 40 (SV40). When there are two sequences encoding polyA signal regions in a targeting vector, they may be the same or different. The distance between the 3' end of the polyA signal region of the gene to be expressed in the certain cells and/or tissue and the 5' end of a promoter sequence controlling the expression of a nucleic acid sequence encoding a desired protein is not particularly limited as long as the nucleic acid sequence can be expressed in the certain cells and/or tissue. However, as the distance increases, the stability of a transcript, mRNA, may be undesirably affected. In addition, the size of the structure of a targeting vector becomes larger. As a result, it is difficult to construct such a vector. For these reasons, it is preferable that the distance between the 3' end of the polyA signal region and the 5' end of the promoter sequence controlling the expression of the nucleic acid sequence encoding a desired protein falls within 1 Kb.

The nucleic acid sequence encoding a desired protein can comprise a polyA signal region of the gene to be expressed in certain cells and/or tissue and, downstream thereof, a sequence comprising the promoter/5' non-translational region/leader sequence coding region derived from the gene expressed in certain cells and/or tissue, a sequence derived from a nucleic acid sequence encoding a desired protein by removal of the leader sequence coding region, and a polyA signal region-encoding sequence, arranged in that order. Specifically, a nucleic acid sequence derived from the nucleic acid sequence encoding a desired protein by removal of the leader sequence coding region is functionally ligated to a sequence consisting of the promoter/5' non-translational region/leader sequence coding region and a polyA signal region encoding sequence (in a cassette format, for example) and such nucleic acid sequence is present downstream of a polyA signal of the gene expressed in certain cells and/or tissue. When constructing a targeting vector, the sequence comprising the promoter/5' non-translational region/leader sequence coding region used herein is not particularly limited, provided that it can control the expression of a gene in the certain cells and/or tissue. Preferably, the sequence comprising the promoter/5' non-translational region/leader sequence coding region of the gene to be expressed in certain cells and/or tissue is used. Also, the sequence consisting of the promoter/5' non-translational region/leader sequence coding region may be a continuous sequence on the genome, or such sequence may be an artificially ligated sequence of functional regions such as the promoter, the 5' non-translational region, and the leader sequence coding region. The 5' non-translational region and the leader sequence coding region used herein may or may not comprise an intron or introns. According to a preferable embodiment of the present invention, the sequence comprising the promoter/5' non-translational region/leader sequence coding region is derived from the murine immunoglobulin κ light chain gene The murine immunoglobulin κ light chain gene comprises many segments of variable (V) regions on the genome, each comprising the inherent sequence comprising the promoter/5' non-translational region/leader sequence coding region. As the sequence comprising the promoter/5' non-translational region/leader sequence coding region derived from the murine immunoglobulin κ light chain gene, any sequence comprising the promoter/5' non-translational region/leader sequence coding regions of many variable (V) regions may be used. The sequence consisting of the promoter/5' non-translational region/leader sequence coding region derived from the murine immunoglobulin κ light chain gene is preferably of the NP type described in Examples, and more preferably of the PS type described in Examples.

Furthermore, the sequence encoding the polyA signal region in constructing a targeting vector is not particularly limited as long as it is the sequence of a polyA signal region known in the art. Examples of the polyA signal region include a polyA signal region derived from the same origin as the promoter and a polyA signal region derived from simian virus 40 (SV40). When there are two sequences encoding polyA signal regions in a targeting vector, they may be the same or different. A preferable example of a polyA signal region encoding sequence is a polyA signal located downstream of the Cκ exon of the murine immunoglobulin κ chain gene. The distance between the 3' end of the polyA signal region of the gene expressed in the certain cells and/or tissue and the 5' end of a promoter sequence controlling the expression of a nucleic acid sequence encoding a desired protein is not particularly limited as long as the nucleic acid sequence can be expressed in the certain cells and or tissue. However, as the distance increases, the stability of a transcript, mRNA, may be undesirably affected. In addition, the size of the structure of a targeting vector becomes larger. As a result, it is difficult to construct such a vector. For these reasons, it is preferable that the distance between the 3' end of the polyA signal region and the 5' end of the promoter sequence controlling the expression of the nucleic acid sequence encoding a desired protein falls within 1 Kb.

When a nucleic acid sequence (e.g., a structural gene) encoding a desired protein is arranged in the vicinity of the control region, the nucleic acid sequence may be inserted in the vicinity of the control region, or may be arranged immediately downstream of the control sequence of the gene expressed in the certain cells and/or tissue in such a manner that it replaces an original structural gene, with respect to one of the alleles of an ES cell. To explain more specifically, since the original structural gene replaced by the nucleic acid sequence encoding a desired protein can be expressed by the other allele, the cells and/or tissue can remain normal.

An immunoglobulin κ chain gene is expressed by joining many V and J gene segments recombinantly, as mentioned above. As a result of the joining, the promoter sequence present in the vicinity of the upstream region of each V gene segment comes in the vicinity of an enhancer sequence present downstream of J gene segments. The enhancer sequence cannot activate the promoter until such an arrangement takes place (Picard et al., Nature, 307:80-2, 1984). More specifically, the nucleic acid sequence encoding a desired protein can be arranged in the vicinity of the enhancer sequence by artificially linking it to the promoter sequence of the immunoglobulin κ chain gene. A sequence derived from the nucleic acid sequence encoding a desired protein by removal of the leader sequence coding region can be arranged in the vicinity of the aforementioned enhancer sequence while being artificially ligated to a sequence consisting of the promoter/5' non-translational region/leader sequence coding region derived from the immunoglobulin κ chain gene. It is known that another enhancer sequence is present further downstream in the immunoglobulin κ chain gene locus (Meyer et al., EMBO J. 8:1959-64, 1989). Likewise, the gene is highly expressed in B cells under the influence of a plurality of enhancers.

The pluripotent cells derived from a non-human animal and containing a genome having a nucleic acid sequence encoding a desired protein as mentioned above are hereafter referred to as cells with transferred genes or ES cells with transferred genes. These cells can be obtained as described below, for example.

### 1-1. Obtaining ES cells with transferred gene

### (1) Construction of RS element targeting vector

An RS element targeting vector comprising genomic sequences in the upstream and downstream regions of a murine RS element sequence and a selection marker inserted instead of the RS element sequence is constructed.

Each of the genome sequences corresponding to the upstream and downstream regions of the murine RS element sequence may be constituted of a certain number of nucleotides, for example, desirably 2 kb or more and the total of the upstream and downstream genome sequences is desirably 7 kb or more.

Further, a selection marker is inserted instead of the RS element in the vector. For example, neomycin resistant gene, puromycin resistant gene, blasticidin resistant gene, or GFP gene can be used as the selection marker.

Furthermore, the structure of a targeting vector may be modified in order to improve the homologous recombination efficiency. More specifically, the homologous recombination efficiency can be increased by engineering a negative selection marker for excluding cells with targeting vectors randomly inserted into the genome so as not to be exposed at the end(s) of the vector when the targeting vector is linearized.

More specifically, in the targeting vector linearized, a gene serving as a negative selection marker is desirably engineered such that its 5'- and 3'-ends are positioned at least 1 Kb, preferably 2 Kb or more apart from the 5'- and 3'-ends of the targeting vector, respectively. Since a region utilized for homologous recombination with a genome (i.e., homologous recombination region) is usually located at one of the 5' end and the 3' end of a negative selection marker, the distance from the one of the ends of the vector comes to be 3 Kb or more, in most cases. On the other hand, the other end of the negative selection marker is often arranged close to the other end of the vector. In the present invention, the negative selection marker is engineered such that the end of the negative selection marker, which is not arranged next to the homologous recombination region, is arranged at a distance of at least 1 kb apart from the end of linearized vector. In this manner, homologous recombination efficiency is increased. As the sequence to ensure the distance from the end of the vector, the sequence of a plasmid vector such as pUC, which is employed in constructing a targeting vector, may be used as it is (without removing when the targeting vector is linearized). Alternatively, as the sequence, a new non-coding sequence not homologous to a desired targeting region may be arranged next to the negative selection marker. The vector is linearized by probing restriction enzyme recognition sites of the targeting vector in use and selecting an appropriate restriction recognition site, thereby ensuring a proper distance between the vector end and the negative selection marker end. In this manner, the effect of improving homologous recombination efficiency can be attained. Even if such an appropriate restriction site is not found, an appropriate restriction enzyme recognition sequence can be introduced into a desired position of a targeting vector by a method using PCR (Akiyama et al., Nucleic Acids Research, 2000, Vol. 28, No. 16, E77.).

It is suggested that taking the structure of a targeting vector as mentioned above allows the frequency of attacks of the negative selection marker to a nuclease to reduce in a cell, thereby elevating the efficiency of homologous recombination.

In short, the present invention provides a gene targeting vector characterized in that the 5'- and 3'-ends of a gene structure functioning as a negative selection marker are apart from at least 1 Kb, preferably 3 Kb or more from the 5' end and 3' end of the linearized targeting vector, respectively, and provides a method for targeting a gene using the targeting vector. In the targeting vector, any negative selection marker may be used as long as it is known in the art. Preferably, a diphtheria toxin A chain gene may be used as the negative selection marker.

### (2) Obtaining RS element targeting murine ES cells

Murine ES cell can be usually established by the method as described below. Male and female mice are crossed. The 2.5-day old embryo after fertilization is taken and cultured *in vitro* in culture medium for ES cells. The embryo developed till the blastocyst stage is separated from the cultured embryos, and is seeded and cultured on a medium with feeder cells. From the cultured embryos, embryos growing in an ES cell like form are selected. A cell mass is taken from the embryos thus selected, dispersed in the medium for ES cells containing trypsin, cultured in the medium with feeder cells, and then, subcultured in the medium for ES cells. The grown cells are isolated.

An RS element targeting murine ES cell can be obtained by use of a targeting vector in accordance with any method known in the art as described in, for example, Bio-Manual Series 8, Gene Targeting (by Shinichi Aizawa, 1995, Yodosha, Japan). More specifically, the targeting vector as constructed above is introduced into murine ES cells by electroporation or lipofection to obtain murine ES cells devoid of the RS element and having a resistant gene inserted into the deleted region. Through the procedures as mentioned above, it is possible to obtain murine ES cells enhanced in homologous recombination efficiency in a chromosomal region downstream of the immunoglobulin light chain constant region gene.

### (3) Construction of targeting vector

First, a targeting vector is constructed in such a manner that it comprises a gene to be expressed in certain cells/or tissue and, downstream thereof, a sequence derived from the nucleic acid sequence encoding a desired protein by removal of the leader sequence coding region functionally ligated to a sequence consisting of the promoter/5' non-translational region/leader sequence coding region and a sequence encoding a polyA signal region inserted therein (in a cassette format, for example).

As the nucleic acid sequence to be introduced, cDNA or intron-containing genomic DNA may be used as long as it comprises a sequence spanning from a boundary point between the leader amino acid sequence and the mature protein product to the termination codon, which is deduced with the use of, for example, signal P, as a computer program for detecting a leader sequence of a secretory protein. The type of the protein encoded by the nucleic acid sequence may not be limited. The nucleic acid sequence to be used in the present invention may be used for highly expressing/secreting the protein encoded by the nucleic acid sequence or for elucidating the function of the protein. Accordingly, as long as the nucleotide sequence can be specified, any type of the nucleic acid sequence may be used. Examples of such a nucleic acid sequence (or structural gene) include nucleotide sequences of genes encoding functional proteins derived from a mammal, preferably a human, such as genes encoding secretory proteins, genes encoding membrane proteins, and genes encoding intracellular or intranuclear proteins.

In order to modify the animal genome so as to insert a sequence derived from a nucleic acid sequence encoding a desired protein by removal of the leader sequence coding region functionally ligated to a sequence consisting of the promoter/5' non-translational region/leader sequence coding region and the polyA signal region encoding sequence into a site downstream of the gene to be expressed in certain cells and/or tissue (in a cassette format, for example), a targeting vector is prepared. To DNA of the resulting vector is inserted a nucleic acid sequence comprising a sequence derived from a nucleic acid sequence encoding a desired protein by removal of the leader sequence coding region functionally ligated to the sequence consisting of the promoter/5' non-translational region/leader sequence coding region and the polyA signal region encoding sequence (in a cassette format, for example). Examples of vectors that can be used for this purpose include plasmid and virus vectors. A person skilled in the art can easily select and obtain a vector that can be used as a targeting vector. A specific example of a vector is, but is not limited to, the pCk loxPV KI vector (see the Examples below). The targeting vector comprises an adequate restriction enzyme cleavage site as the site into which the cassette is to be inserted at a site downstream of the polyA signal region of the gene to be expressed in certain cells and/or tissue. Further, a vector can comprise a selection marker, such as the puromycin resistant gene, the neomycin resistant gene, the blasticidin resistant gene, and the GFP gene, according to need

### (4) Introduction of a targeting vector into pluripotent cell derived from a non-human animal and selection of homologous recombinant

Pluripotent cells derived from a non-human animal each can be transformed by a targeting vector in accordance with a method known in the art, for example, described in Bio-Manual Series 8, Gene Targeting (by Shinichi Aizawa, 1995, Yodosha, Japan) More specifically, the targeting vector as constructed above may be introduced into each of the pluripotent cells by electroporation, lipofection, or other means.

Moreover, the targeting vector may be modified to increase the efficiency of homologous recombination. More specifically, the homologous recombination efficiency can be increased by engineering a negative selection marker, which is for excluding cells with targeting vectors randomly inserted into the genome, so as not to be exposed to the ends of the target vector when the vector is linearized.

More specifically, in the linearized targeting vector, the 5'- and 3'-ends of a gene serving as a negative selection marker are desirably engineered such that they are positioned at least 1 Kb, preferably 2 Kb or more apart from the 5'- and 3'-ends of the targeting vector. Since a region utilized for homologous recombination with a genome (i.e., homologous recombination region) is usually located at either one of the 5' end and the 3' end of the negative selection marker, the distance from the end of the vector comes to be 3 Kb or more, in most cases. On the other hand, the other end of the negative selection marker is often arranged close to the other end of the vector. In the present invention, the negative selection marker is engineered such that the end of the negative selection marker, which is not arranged next to the homologous recombination region, is located at a distance of at least 1 kb, apart from the end of linearized vector. In this manner, homologous recombination efficiency is elevated. As the sequence used for ensuring the distance from the end of the vector, the sequence of a plasmid vector such as pUC, which is employed in constructing the targeting vector, may be used as it is (without removing when the target vector is linearized). Alternatively, as the sequence, a new non-coding sequence not homologous to a desired targeting region may be arranged next to the negative selection marker. The vector is linearized by probing restriction enzyme recognition sites of the targeting vector in use and selecting an appropriate restriction site, thereby ensuring a proper distance between the vector end and the negative selection marker end. In this manner, the effect of improving homologous recombination efficiency can be attained. Even if such an appropriate restriction site is not found, an appropriate restriction enzyme recognition sequence can be introduced into a desired position of a targeting vector by a method using PCR (Akiyama et al., Nucleic Acids Research, 2000, Vol. 28, No. 16, E77.).

It is suggested that taking the structure of a targeting vector as mentioned above allows the frequency of attacks of the negative selection marker to a nuclease to reduce in a cell, thereby elevating the efficiency of homologous recombination.

In short, the present invention provides a gene targeting vector characterized in that the 5' end and 3' end of a gene functioning as a negative selection marker are apart from at least 1 Kb, preferably 3 Kb or more from the 5' end and 3' end of the linearized targeting vector, respectively, and provides a method for targeting a gene using the targeting vector. In the targeting vector, any negative selection marker may be used as long as it is known in the art. Preferably, a diphtheria toxin A chain gene may be used.

As a positive selection marker, the pLoxP-Puro-derived puromycin resistant cassette disclosed in PCT International Application WO 00/10383 (published March 2, 2000) filed by the applicant of the present invention can be used. This drug resistant cassette comprises at both ends Lox-P sequences in forward direction. By the method disclosed in WO 00/10383, the drug resistant gene can be removed from the pluripotent cells comprising the targeting vector inserted therein.

As the positive selection marker used for removing the RS sequence, the pLoxP-Stneo-derived Neo resistant cassette disclosed in PCT International Application WO 00/10383 (published March 2, 2000) filed by the applicant of the present invention can be used. This drug resistant cassette comprises at both ends LoxP sequences in forward direction. By the method disclosed in WO 00/10383, the drug resistant gene can be removed from the pluripotent cells comprising the targeting vector inserted therein.

As LoxP sequences located in the forward direction at both ends of the drug resistant cassette, mutant LoxP sequences (Lee et al., Gene, 216:55-65, 1998) can be used for the drug resistant marker for positive selection contained in the targeting vector (e.g., the puromycin resistant cassette) or the drug resistant marker for positive selection inserted so as to remove the RS sequence (e.g., the Neo resistant cassette). This can prevent the deletion resulting from the recombination between the genome sequence between LoxP sequences at both ends of the puromycin resistant cassette contained in the targeting vector, which is immediately downstream of the Cκ polyA site, and the genome sequence between LoxP sequences at both ends of the Neo resistant cassette located in the RS region located about 25 kb downstream thereof Such a technique is useful when simultaneous removal of these two drug resistant cassettes alone is intended.

As a result of the studies that have been conducted to date, it was reported that the drug resistant cassette inserted into the intron enhancer region in the immunoglobulin κ chain locus (Xu et al., Immunity, 4:377-385, 1996) or the 3' enhancer region about 9-kb downstream of the Cκ exon (Gorman et al., Immunity, 5:241-252, 1996) would inhibit the VDJ recombination of the immunoglobulin κ chain gene and that the expression levels of the κ chain genes were lowered. These reports suggested that the presence of the drug resistant cassette would influence the expression of the κ chain genes based on the fact that the expression level would be restored to some extent upon removal of the drug resistant cassette with the utilization of the Cre/LoxP system. In the experiment wherein the murine Cκ region had been substituted with the human Cκ region (Zou et al., Science, 262:1271-1274, 1993), however, the expression levels of the murine/human chimeric Igκ chain genes were observed to be high, regardless of the presence of the drug resistant marker in the Cκ downstream region. This means that the influence of the presence of the drug resistant cassette inserted into a site in the vicinity of Igκ on the expression of the Igκ chain genes was not conclusive. Although the Igκ control region was utilized, the influence thereof on the expression of the gene contained in the expression unit independent from the endogenous Igκ gene was not fully elucidated.

Furthermore, the efficiency of inserting a target gene into a site downstream of the Igκ constant region with the use of a targeting vector can be increased by use of as murine ES cell having a drug resistant marker inserted into the RS element region about 25 Kb downstream of the Igκ light chain gene.

To easily identify a homologous recombinant, a drug resistant gene marker may be previously introduced into the position to be targeted by a foreign gene. For example, the murine ES cell TT2F, which is used in Examples of the present specification, is derived from F1 individuals between C57BL/6 line and CBA line. When the sequence of the genomic homologous region contained in a targeting vector is derived from C57BL/6 as previously described (Deng & Capecchi, Mol. Cell. Biol., 12:3365-71, 1992), homologous recombination may conceivably take place more efficiently in the allele derived from C57BL/6 line in the TT2F cell. In other words, it is possible to insert, for example, a G418 resistant marker, into the allele derived from the C57BL/6 line in advance by using a targeting vector containing DNA derived from the C57BL/6 line. Then, a targeting vector containing a puromycin resistant marker and genomic DNA derived from the C57BL/6 line is introduced into the G418 resistant line thus obtained. In this manner, the puromycin resistant and G418 sensitive line can be obtained. In this line, the G418 resistant gene is removed by homologous recombination between the targeting vector and the gene to be expressed in certain cells and/or tissue, and instead, a nucleic acid sequence encoding a desired protein (e.g., a structural gene) and the puromycin resistant marker are introduced. In this manner, an analysis step required for identifying a homologous recombinant, such as Southern analysis, can be eliminated.

After the puromycin resistant clone is picked up, the genomic DNA is prepared and subjected to Southern analysis to identify a homologous recombinant in the same manner as that described in PCT International Application WO 00/10383 (published March 2, 2000) filed by the applicant of the present invention. The puromycin resistant gene in the targeting vector is derived from Lox-P Puro plasmid described in WO 00/10383 and contains a Lox-P sequence at the ends thereof in a forward direction. Therefore, the puromycin resistant gene can be removed from the targeted pluripotent cells by the method described in WO 00/10383.

The targeting vector and technique/means for improving homologous recombination efficiency can be applied to all cells capable of introducing a gene and not limited to the case of forming a chimeric animal. For example, the targeting vector and the technique for improving homologous recombinant efficiency described in the present specification can be used for destroying or introducing a desired gene in gene therapy directed to a human or human cells (such as blood cells or immune cells).

### 2. Host embryos devoid of certain cells and/or tissue

Next, in a method of preparing a chimeric non-human animal according to the present invention, a host embryo of a non-human animal devoid of the certain cells and/or tissue (hereinafter, also referred to as a "deficient host embryo") is prepared. Examples of such a deficient host embryo include: a B-cell deficient embryo due to knock-out of an immunoglobulin heavy chain gene, when an immunoglobulin light chain gene is used as the control region (Tomizuka et al., Proc. Natl. Acad. Sci. U.S.A., 18:722-727, 2000); a T lymphocyte deficient embryo due to deletion of a T-cell receptor β-chain when T cell receptor gene is used as the control region (Mombaerts et al., Nature, 360: 225-227, 1992); a muscular tissue deficient embryo due to knock-out of the myogenin gene when the myoglobin gene is used as the control region (Nabeshima et al., Nature, 364:532-535, 1993); an embryo derived from a murine mutant, aphakia (ak) line, devoid of crystalline lens when the crystalline gene is used as the control region (Liegeois et al., Proc. Natl. Acad. Sci. U.S.A., 93: 1303-1307, 1996); an embryo devoid of the kidney tissue due to knock-out of the sall gene when the renin gene is used as the control region (Nishinakamura et al., Development, 128: 3105-3115, 2001); an embryo devoid of the liver tissue due to deletion of the c-Met gene when an albumin gene is used as the control region (Bladt et al., Nature, 376: 768-770, 1995); and an embryo deficient in the pancreatic tissue due to knock-out of the Pdx1 gene when a lipase gene is used as the control region (Jonsson et al., Nature, 371: 606-9, 1994). In the above, preferable deficient host embryos are exemplified; however, the deficient host embryo that may be used in the present invention is not limited to these.

As to selection of the development stage, genetic background or the like of a host embryo for efficiently producing a chimeric non-human animal, the conditions already specified with respect to the ES cell lines based on research are desirably employed. More specifically, in the case of a mouse, when a chimera is produced from the TT2 cell derived from CBA ×C57BL/6 F1 mouse or the TT2F cell (wild color, Yagi et al., Analytical Biochemistry, 214: 70-76, 1993), a host embryo desirably has a genetic background of Balb/c (white, available from CLEA Japan), ICR (white, available from CLEA Japan) or MCH (ICR) (white, available from CLEA Japan). Therefore, as a deficient host embryo, it is desirable to use a non-human animal embryo (e.g., 8-cell stage) obtained by back-crossing a non-human animal line devoid of certain cells and/or tissue with each of the aforementioned lines.

Since the cells and/or tissue that a host embryo is devoid of is compensated by pluripotent cells in accordance with blastocyst complementation (BC), the deficient host embryo may be an embryonic lethal as long as it can develop till the blastocyst stage required for producing a chimeric animal. Such an embryonic lethal appears with a rate of 1/4 in theory when animals heterozygous for gene defect are crossed with each other. Therefore, chimeric animals are created by using a plurality of embryos obtained by crossing in accordance with the following procedures and deficient embryos are selected as host embryos from the embryos obtained from the chimeric animals. The selection is performed by extracting DNA from the somatic tissue of a chimeric animal and subjecting the DNA to Southern analysis, PCR or the like.

### 3. Production of chimeric embryo and transplantation into surrogate mother

A chimeric non-human animal is produced from the ES cell line with transferred gene as prepared in Section 1 ("Preparation of pluripotent cells") in accordance with the method of Shinichi Aizawa (supra). More specifically, the gene-transferred pluripotent cell is injected into the blastocyst or 8-cell stage embryo of a deficient host embryo as described in Section 2 ("Host embryos devoid of certain cells and/or tissue") by use of a capillary or the like. Then, the blastocyst or 8-cell stage embryo is directly transplanted to the oviduct of a cognate surrogate mother, which is a non-human animal, or alternatively it is cultured for a day up to a blastocyst embryo, which is then transplanted to the uterus of a surrogate mother. Thereafter, the surrogate mother is allowed to give birth to obtain an offspring animal.

### 4. Expression of the transferred gene in chimeric non-human animals

The offspring animal is produced in accordance with the section 3 ("Production of chimeric embryo and transplantation into surrogate mother") from an embryo into which a pluripotent cell transferred gene was injected. The contribution rate of the pluripotent cell to the offspring animal can be roughly determined based on the coat color of the offspring animal. For example, when a gene-transferred cell line from TT2F cell (wild color: dark blown) is injected into a host mouse embryo having MCH(ICR) background (white), the rate of the wild color (dark brown) represents the contribution rate of the pluripotent cell. In this case, the contribution rate indicated by coat color correlates with that of a gene-transferred pluripotent cell in cells and/or tissue other than the deleted ones; however, depending upon the tissue, the contribution rate of the pluripotent cell is not sometimes consistent with that indicated by coat color. On the other hand, only the cells and/or tissue from gene-transferred pluripotent cell are present in the chimeric non-human animal, whereas the deleted cells and/or tissue from host embryo do not exist therein. The restoration of the cells and/or tissue deleted in the chimeric non-human animal by contribution of the gene-transferred pluripotent cell can be detected by the FACS (Fluorescence-Activated Cell Sorter) assay, ELISA (Enzyme-linked Immuno Sorbent Assay), or the like. Whether a nucleic acid sequence (or structural gene) inserted into the cells and/or tissue from the gene-transferred pluripotent cell is expressed is detected by the RT-PCR method (Kawasaki et al., P.N.A.S., 85:5698-5702, 1988) using RNA derived from the cells and/or tissue, Northern blot method (Ausubel et al., Current protocols in molecular biology, John Wiley & Sons, Inc., 1994), or the like. When an antibody specific to a desired protein encoded by the transferred nucleic acid sequence is already present, the expression of the protein can be detected by the Enzyme-linked Immuno Sorbent Assay using chimeric mouse serum (ELISA; Toyama and Ando, Monoclonal Antibody Experimental Manual, 1987, Kohdansha Scientific, Japan), Western blot (Ausubel et al., supra), or the like. Alternatively, if DNA encoding the nucleic acid sequence (or structural gene) to be transferred is appropriately modified previously such that a tag peptide detectable with an antibody is added to the protein encoded by the DNA, then the expression of the transferred gene can be detected with the antibody to the tag peptide or the like (e.g., POD labeled anti-His₆; Roche Diagnostics, Japan).

In the chimeric non-human animal prepared as described above, the transferred nucleic acid sequence (e.g., a structural gene) can be highly expressed at least in certain cells and/or tissue. If the desired protein expressed is a secretory protein-like blood or milk, the chimeric non-human animal can be used as a production system for a useful protein. Alternatively, if a protein whose functions are unknown is highly expressed, the function of the protein may be elucidated from findings accompanied with the high expression.

Furthermore, recently, the combination of the method for producing animal individuals from somatic cell nucleus-transplanted embryos with the gene targeting in somatic cell has made the gene modification possible as in mouse even in animal species (cow, sheep, pig, etc.) other than mouse (McCreath et al., Nature, 405: 1066-1069, 2000). For example, a cow devoid of B cells can be produced by knocking out an immunoglobulin heavy chain. Alternatively, a gene of interest can be inserted into an Ig gene or in the vicinity thereof obtained from an animal such as a mouse, cow, sheep or pig to cause an unfertilized egg, into which the nucleus of the fibroblast has been transplanted, to develop, and an ES cell can be prepared from the embryo, which has been developed into a blastocyst stage. From the ES cell thus obtained and the B cell deficient host embryo as mentioned above, a chimeric non-human animal can be produced (Cibelli et al., Nature Biotechnol., 16: 642-646, 1998). High expression of secretory proteins using a similar expression system is also possible not only in a mouse but also in other animal species. When a larger animal is used, production of a useful substance becomes possible in addition to analysis of the function of a gene. According to the method disclosed in the present invention, a foreign DNA expression unit that is constructed to be controlled by the control region of the murine or calf immunoglobulin gene can be inserted in the vicinity of the endogenous immunoglobulin locus of a fetal calf fibroblast cell line by the method of Kuroiwa et al. (Nat. Genet. 36: 775-80, 2004). It can be expected that the foreign DNA can be highly expressed in the blood serum of the calf clones prepared from the resulting genetically modified calf fibroblast cell line in accordance with the method of Kuroiwa et al. (Nat. Genet. 36: 775-80, 2004).

### 5. Production of progeny of chimeric non-human animal

The method of producing a chimeric non-human animal according to the present invention further comprises: crossing a chimeric non-human animal with a cognate non-human animal to produce transgenic animals; selecting, from among the transgenic animals, male and female transgenic (Tg) animals heterozygous for the transferred nucleic acid sequence; and crossing the male and female Tg animals to each other to obtain Tg animal progeny homozygous for the transferred nucleic acid sequence.

### 6. Tissue or cells derived from a chimeric non-human animal or its progeny

According to the present invention, it is possible to obtain tissue or cells derived from any one of the chimeric non-human animals or progenies thereof as mentioned above. The cells or tissue contains a genome in which a nucleic acid sequence encoding a desired protein is arranged such that the desired protein can be expressed under the control of a control region of a gene to be expressed in the cells or tissue and thus can express the desired protein.

Any tissue or cell may be used as long as it is derived from a chimeric non-human animal or its progeny and is capable of expressing a desired protein. Examples of such tissue or cell include B cells, spleen and lymph tissue.

The tissue or cells can be taken and cultured in accordance with a known method in the art. Whether the tissue or cells express a desired protein can also be confirmed by conventional methods. Such tissue or cells are useful for producing a hybridoma or protein as mentioned below.

### 7. Production of hybridoma

In the present invention, cells of a chimeric non-human animal capable of expressing a transferred nucleic acid sequence encoding a desired protein (in particular, B cell or spleen cell containing B cell, and cell from lymph tissue such as lymph node) are hybridized with a proliferative tumor cell (e.g., myeloma cell) to obtain hybridomas. A method of producing hybridomas may be based on procedures as described, for example, in the Andoh and Chiba (Introduction of Monoclonal Antibody Experimental Manipulation, 1991, Kohdansha Scientific, Japan).

Used as such a myeloma are, for example, cells with no ability to produce self-antibodies derived from a mammal such as a mouse, rat, guinea pig, hamster, rabbit, or human. Use of cell lines generally obtainable from mice, such as myeloma cell lines derived from 8-azaguanine resistant mice (BALB/c) P3X63Ag8U.1 (P3-U1) (Yelton, D. E. et al., Current Topics in Microbiology and Immunology, 81: 1-7, 1978); P3/NSI/1-Ag4-1 (NS-1) (Kohler, G. et al., European J. Immunology, 6: 511-519, 1976); Sp2/O-Ag14 (SP-2) (Shulman, M. et al., Nature, 276: 269-270, 1978); P3X63Ag8.653 (653) (Kearney, J. F. et al., J. Immunology, 123: 1548-1550, 1979); and P3X63Ag8 (X63) (Horibata, K. and Harris, A. W. Nature, 256: 495-497, 1975), is preferable. These cell lines are subcultured in an appropriate medium such as 8-azaguanine medium (RPMI-1640 medium containing glutamine, 2-mercaptoethanol, gentamicin, and fetal calf serum (hereinafter refers to as "FCS") supplemented with 8-azaguanine), Iscove's Modified Dulbecco's medium (hereinafter referred to as "IMDM"), or Dulbecco's Modified Eagle Medium (hereinafter referred to as "DMEM"). However, 3 to 4 days before cell fusion, they are subcultured in normal medium (e.g., DMEM medium containing 10% FCS). In this manner, at least 2 × 10⁷ cells are prepared until the day of cell fusion.

Used as cells capable of expressing a desired protein encoded by the transferred nucleic acid sequence are for example plasma cells and lymphocytes as the precursor cells, which may be obtained from any part of an animal individual and generally obtained from the spleen, lymph node, bone marrow, amygdale, peripheral blood or an appropriate combination thereof. Use of spleen cells is the most common.

At present, the most general means for fusing a spleen cell, which expresses a desired protein encoded by the transferred nucleic acid sequence, with a myeloma cell is a method using polyethylene glycol since cytotoxicity is relatively low and fusion is simple. More specifically, fusion can be performed as follows. First, spleen cells and myeloma cells are washed well with a serum free medium (e.g., DMEM) or phosphate buffered saline (generally referred to as "PBS"), mixed in a cell ratio of about 5:1 to about 10:1, and they are separated by centrifugation. The supernatant is removed and precipitated cells are loosened. To the loosened cells, the serum free medium containing 1 ml of 50% (w/v) polyethylene glycol (molecular weight 1,000 to 4,000) is added dropwise while stirring. Thereafter, 10 ml of the serum free medium is gently added to the mixture and separated by centrifugation. The supernatant is discarded and precipitated cells are resuspended in a normal medium (generally referred to as "HAT medium") containing hypoxanthine, aminopterin and thymidine and further human interleukin-6 in appropriate amounts, dispensed to wells of a culture plate, and cultured at 37°C for about 2 weeks in the presence of 5% carbon dioxide gas while supplying HAT medium appropriately during the culture.

When the myeloma cell is from a 8-azaguanine resistant cell line, namely a hypoxanthine guanine phosphoribosyl transferase (HGPRT) deficient cell line, myeloma cells not hybridized and myeloma-myeloma hybrid cells cannot survive in the HAT-containing medium. In contrast, spleen-spleen hybrid cells or spleen-myeloma hybrid cells can survive; however, spleen cell/spleen cell hybrids have a limited life. If culture is continued in the HAT-containing medium, therefore, only spleen-myeloma hybrid cells can survive.

The obtained hybridomas can be further screened by ELISA using a specific antibody against the desired protein encoded by the transferred nucleic acid sequence As a result, hybridoma producing the desired protein encoded by the transferred nucleic acid sequence can be selected.

### 8. Method of producing protein

The present invention further provides a method of producing a desired protein comprising producing the desired protein by using any one of the chimeric non-human animal or its progeny as described above, the tissue or cells as described above, and hybridomas as described above, followed by recovering the desired protein. More specifically, the chimeric non-human animal or its progeny is kept under the conditions in which the transferred nucleic acid sequence encoding a desired protein can be expressed, and the protein, as an expression product, is recovered from the blood, ascites fluid or the like of the animal. Alternatively, tissue or cells derived from a chimeric non-human animal or its progeny or the tissue or cells immortalized (for example, hybridomas immortalized by fusing them with myeloma cells) are cultured under such conditions that the transferred nucleic acid sequence encoding a desired protein can be expressed, and thereafter, the protein, as an expressed product, is recovered from the culture or the supernatant thereof. The expressed product can be recovered by using a known method such as centrifugation and further purified by using known methods, such as ammonium sulfate fractionation, partition chromatography, gel filtration chromatography, absorption chromatography (e.g., ion exchange chromatography, hydrophobic interaction chromatography, or affinity chromatography), preparative thin-layer chromatography, and HPLC, alone or in combination.

### 9. Methods of analyzing a biological function

The present invention further provides a method of analyzing a biological (or *in vivo)* function of a desired protein or a gene encoding a desired protein, comprising comparing the phenotype of the chimeric non-human animal or its progeny as prepared above with that of a control animal, i.e., a chimeric non-human animal which is produced from a corresponding wild-type ES cell and does not contain the nucleic acid sequence encoding a desired protein; and determining a difference in phenotype between them

In this method, any trait emerging *in vivo* due to the gene transfer can be detected by physicochemical methods, thereby identifying a biological function of the transferred nucleic acid sequence or the protein encoded thereby. For example, blood samples are taken from chimeric non-human animals or progeny thereof which are produced from ES cells containing a nucleic acid sequence encoding a desired protein and from control chimeric non-human animals which are produced from wild-type ES cells and contain no nucleic acid sequence encoding a desired protein; and the blood samples are analyzed by blood cell counter. By comparing blood levels of leukocytes, erythrocytes, platelets or the like between the two types of chimeric non-human animals, the effect of the desired protein encoded by the transferred nucleic acid sequence on proliferation and differentiation of blood cells can be clarified. In Examples as described later, DNA encoding erythropoietin (EPO) was used as the transferred nucleic acid sequence. In this case, the significant increase in red blood cells (i.e., trait) was observed in chimeric mice.

Now, further preferable embodiments of the present invention will be described taking the system using an immunoglobulin light chain gene as an example.

Immunoglobulin (Ig) is one of the secretory proteins produced in the largest amount in serum. For example, immunoglobulin occupies 10 to 20% of the serum protein in humans at a level of 10 to 100 mg/ml. Immunoglobulin (Ig) is produced in B cells, mainly in terminally differentiated B cells i.e. plasma cells, in a large amount. However, various factors including high transcriptional activity in the Ig locus, stability of mRNA, and function of plasma cells specialized for secretion and production of a protein, contribute to a high level of Ig expression. Furthermore, in an adult, B cells are produced in the bone marrow and migrate to the spleen, the small intestine Peyer's patch, and the systemic lymph tissue such as the lymph node with maturation. The product of the transferred gene produced in the control region of an Ig gene of the B cell is released into the blood or the lymph in the same manner as Ig and rapidly delivered throughout the body. The present invention is advantageous since a nucleic acid sequence encoding a desired protein (i.e., structural gene) is expressed by use of the Ig expression system enabling high expression.

To express a transferred gene efficiently, it is desirable to introduce the transferred gene into the gene encoding Ig light chain, preferably K-light chain. For example, 95% of mouse immunoglobulin contains κ-light chain and only one constant region gene is present there, whereas λ-light chain is present in 5% of the mouse immunoglobulin and has 4 different types of genes, any of which is employed. The heavy chain has 7 types of constant regions, i.e. µ, γ (4 types) α, and ε. Considering that a transferred gene is normally inserted at a single site of the Ig gene, use of κ-chain is desirable.

The transferred nucleic acid sequence is desirably expressed under such conditions that functional Ig light chain is produced. A chimeric non-human animal or its progeny according to the present invention preferably contains, on the genome, a gene expression unit having an immunoglobulin light chain gene and a promoter portion of the gene in the vicinity of the gene, and further a nucleic acid sequence (or a transferred gene) encoding a desired protein downstream of the promoter portion. To modify the genome of an animal such that the promoter portion of an immunoglobulin light chain gene is contained in the vicinity of the gene while a nucleic acid sequence (e.g., a transferred gene) encoding a desired protein is contained downstream of the promoter, a targeting vector is provided, in which the nucleic acid sequence encoding a desired protein is inserted. As the targeting vector, CκP2 targeting vector (see Example 12) is preferably used. The targeting vector contains a promoter portion of an immunoglobulin light chain gene in the vicinity of the gene. Downstream of the promoter portion, an appropriate restriction enzyme cleavage site is inserted for introducing the nucleic acid sequence encoding a desired protein (e.g., a transferred gene). At the restriction enzyme cleavage site, DNA (i.e., cDNA or genomic DNA) containing from the initiation codon to the termination codon of the transferred nucleic acid sequence is inserted. In addition, it may be preferable to arrange a translation promoting sequence like Kozak sequence upstream of the initiation codon. Furthermore, to easily identify a homologous recombinant, a drug resistant gene marker, preferably a puromycin resistant gene, may be inserted previously at an inserted position of a foreign gene.

Use may be made of murine ES cells, more specifically ES cells having a drug resistant marker inserted into the RS element region, which is located about 25 Kb downstream of the immunoglobulin κ light chain gene, whereby the efficiency of inserting a desired gene in the vicinity of the Igκ constant region using a targeting vector can be elevated.

Non-human animal ES cells can be transformed by a targeting vector in accordance with the method described by Shinich Aizawa (supra). Then, in the same manner as described in PCT International Application WO 00/10383 (published March 2, 2000) filed by the present applicant, puromycin resistant clones are picked up to prepare genomic DNA, which is subjected to Southern analysis to identify homologous recombinants. The puromycin resistant gene in the targeting vector is derived from the Lox-P Puro plasmid described in WO 00/10383 and includes a Lox-P sequence at both ends in a forward direction. Therefore, the puromycin resistant gene can be removed by the method as described in WO 00/10383, from the ES cell with transferred gene.

In the present invention, when an immunoglobulin light chain gene is used, a non-human animal line homozygous for destruction of its immunoglobulin heavy chain gene (as described in WO 00/10383) is preferably used as a deficient host embryo to inject an ES cell.

The prepared ES cell with transferred gene is injected into the blastocyst stage or 8 cell stage embryo from the deficient host embryo by using a capillary tube. The blastocyst stage or 8 cell stage embryo is directly transplanted into the oviduct of a surrogate mother of the cognate non-human animal, or is alternatively cultured for a day up to a blastocyst embryo, which is then transplanted to the uterus of the surrogate mother. Thereafter, the surrogate mother is allowed to give birth to obtain an offspring animal.

In the chimeric non-human animal, matured B lymphocytes from the host embryo are not present but only those from the ES cell with transferred gene are present. This is because the non-human animal, as a host embryo, whose immunoglobulin heavy chain has been knocked out, is devoid of matured B lymphocytes (B220 positive), whereby no immunoglobulin is detected in the blood (Tomizuka et al., Proc. Natl. Acad. Sci. U.S.A., 97: 722-727, 2000). The restoration of the production of matured B lymphocytes and antibodies in the chimeric non-human animal by contribution of the gene-transferred ES cell can be detected by the FACS analysis, ELISA, or the like. Whether the nucleic acid sequence inserted into the B cell from the targeting ES cell is expressed depends upon whether a site-directed recombination reaction takes place in the Ig light chain gene of the inserted allele. Thus, when recombination of the Ig light chain gene of the inserted allele is successfully performed and mRNA encodes a functional light chain, the transferred nucleic acid sequence (e.g., a structural gene) present concurrently on the mRNA is translated into a protein by the action of IRES. Furthermore, even when recombination of the κ-chain gene of inserted allele fails and the κ-chain or λ-chain gene of the other allele encodes a functional light chain, mRNA encoding the non-functional κ-chain and the transferred nucleic acid sequence is transcribed, with the result that protein derived from the transferred nucleic acid sequence can be expressed. The transferred nucleic acid sequence is not expressed when functional recombination of the κ-chain or λ-chain gene of the other allele successfully takes place in advance and the recombination of the Igκ-chain of the inserted allele is then shut off by the mechanism of allelic exclusion. In a non-human animal, B cells appear in the liver tissue of a fetus around day 12th of viviparity. Upon birth, the place where B-cells are developed changes to the bone marrow. In the fetus stage, the B cells remain in the initial stage of development; in other words, most of the B cells express a membrane-type immunoglobulin receptor. The number of B cells is low and the amount of mRNA encoding an immunoglobulin is low in the cells primarily expressing membrane-type Ig in the fetus compared to an adult. Based on these facts, the expression of the transferred nucleic acid sequence in the fetus may be extremely low compared to that of the adult. Production of antibodies increases from the weaning stage (3 weeks old). This phenomenon is presumably caused by an increase of the plasma cells, the terminal differentiation stage of B cells. Thereafter, B cells migrate into the lymph tissue such as the spleen, lymph node, and intestine Peyer's patch, and express antibodies and the inserted nucleic acid sequence. Likewise, a desired protein encoded by the inserted nucleic acid sequence is secreted into the blood and the lymph in the same manner as immunoglobulin and delivered throughout the body.

The expression of the transferred nucleic acid sequence in the B cells can be confirmed as follows. For example, expression of mRNA by a transferred gene can be detected by RT-PCR or Northern blot using RNA derived from the tissue or cell population containing B cells, such as spleen cells and peripheral blood nucleated cells. When a specific antibody is obtained against a desired protein encoded by a transferred nucleic acid sequence, the expression of a protein can be detected by ELISA or Western blot using the chimeric mouse serum. Alternatively, if DNA encoding a transferred nucleic acid sequence is appropriately modified such that a tag peptide detectable by an antibody is added to the DNA, the expression of the transferred nucleic acid sequence can be detected by an antibody against the tag peptide, etc.

The chimeric non-human animal thus obtained highly expresses the transferred nucleic acid sequence encoding a desired protein in a efficient and reliable manner. The reasons why the efficiency is high are principally based on the points described below.
(1) Since a host embryo used is devoid of B lymphocytes, B lymphocytes of a chimeric animal are all derived from the ES cells irrelevant to the chimeric rate.
(2) By virtue of use of the murine ES cells having a drug resistant marker inserted into the RS element region about 25 Kb downstream of the immunoglobulin κ light chain gene, homologous recombination takes place in the vicinity of the Igκ gene at an efficiency of 30% or more.
(3) Expression system for immunoglobulin is used.
(4) Expression of immunoglobulin is extremely low in the initial stage of development and explosively increases after the wearing stage. For this reason, the function of a transferred gene in the adult can be investigated, even if embryonic lethal is brought by high expression of the transferred gene.

Now, the present invention will be described in detail by way of Examples, which should not be construed as limiting the scope of the present invention.

### EXAMPLES

### Example 1

### Construction of pPSs hEPO in vitro vector

### 1-1. Introduction of PacI-FseI-NheI site in between SalI and HindIII of pBluescriptIISK(-)····· pBS+PFN

pBluescriptIISK(-) (Stratagene, U.S.A.) was digested with SalI and HindIII (Roche), the synthetic oligo DNAs shown below were annealed, and the resultant was introduced via ligation using the ligation kit ver. 2 (Takara Bio). The resultant was introduced into *Escherichia coli* DH5α, DNA was prepared from the resulting transformant, and the inserted fragment was subjected to sequencing.
S/PFN/Hd-S: TCGACTTAATTAAGGCCGGCCCTAGCTAGCA (SEQ ID NO: 1)
S/PFN/Hd-AS: AGCTTGCTAGCTAGGGCCGGCCTTAATTAAG (SEQ ID NO: 2)
1-2. Construction of small vector comprising promoter and signal sequence······ pPSs3.8

Based on the MUSIGKVR1 sequence (accession No: K02159) obtained from the GenBank, the genomic sequence upstream thereof was obtained from the UCSC mouse genome database. PCR primers that amplify a promoter region and an intron-containing signal sequence were designed.
PsecSP FW1:
CCTTAATTAAAGTTATGTGTCCTAGAGGGCTGCAAACTCAAGATC (SEQ ID NO: 3, including the PacI site)
PsecSP RV:
TTGGCCGGCCTTGGCGCCAGTGGAACCTGGAATGATAAACACAAAGATTATTG (SEQ ID NO: 4, including the SfoI·FseI site)

A reaction mixture was prepared by use of KOD-plus- (TOYOBO, Japan) in accordance with the instructions. To the reaction mixture (50 µl), the two primers as prepared above (10 pmol each), 1.6 mM of MgSO₄, and DNA derived from murine ES cells as a template were added. After the reaction mixture was kept at 94°C for 2 minutes, a PCR cycle consisting of 94°C for 15 seconds and 68°C for 1 minute was repeated 30 times. 0.8 kb amplified fragment was separated on 0.8% agarose gel. From the cut-out gel, the amplified fragment (the PS promoter fragment) was recovered by use of QIAquick Gel Extraction Kit (Qiagen, Germany) in accordance with the instructions. The PCR-amplified fragment thus recovered was digested with FseI and PacI (NEB) and separated on 0.8% agarose gel. From the cut-out gel, the enzyme-digested fragment was recovered by use of QIAquick Gel Extraction Kit (Qiagen, Germany) in accordance with the instructions.

After pBS+PFN of 1-1 above was digested with FseI and PacI (NEB), the reaction mixture was subjected to phenol/chloroform extraction and then to ethanol precipitation. The DNA fragment recovered above was inserted into the digested pBS+PFN. The resultant was introduced into *Escherichia coli* DH5α. From the resulting transformant, DNA was prepared, and the inserted fragment was sequenced. Clones having no mutation due to PCR were selected.

### 1-3. Obtaining a DNA fragment from the κ 5' intron enhancer region

Based on the genomic DNA sequence in the vicinity of the murine Ig_{K} gene obtained from the GenBank (NCBI, U.S.A.), the following DNA primers were synthesized.
5' enhancer FW Kp:
GGGGTACCAGCTTTTGTGTTTGACCCTTCCCTA (SEQ ID NO: 5, the KpnI site has been added)
5' enhancer RV Xh:
CCGCTCGAGAGCTAAACCTACTGTATGGACAGGG (SEQ ID NO: 6, the XhoI site has been added)

A reaction mixture was prepared by use of KOD-plus- (TOYOBO, Japan) in accordance with the instructions. To the reaction mixture (50 µl), the two primers as prepared above (10 pmol each) and DNA derived from BAC clone RP23-435I4 (GenBank Accession Number: AC090291) as a template were added. After the reaction mixture was kept at 94°C for 3 minutes, a PCR cycle consisting of 94°C for 15 seconds and 68°C for 40 seconds was repeated 30 times. About 0.48 kb amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with KpnI and XhoI and separated on 0.8% agarose gel. From the cut-out gel, the enzyme-digested fragment was recovered by use of QIAquick Gel Extraction Kit (Qiagen, Germany) in accordance with the instructions.

pBluescriptIISK(-) (Stratagene, U.S.A.) was digested with KpnI and XhoI and separated and purified by 0.8% agarose gel electrophoresis. The ends of pBIuescriptIISK(-) were dephosphorylated with alkaline phosphatase derived from the fetal calf intestine. The DNA fragments recovered above were inserted into the obtained pBluescriptIISK(-), which was then introduced into *Escherichia coli* DH5α. DNA was prepared from the resulting transformant and the inserted fragment was sequenced. Clones having no mutation due to PCR were selected, digested with KpnI and XhoI, and separated on 0.8% agarose gel. From the agarose gel thus recovered, the enzyme-digested fragment (the 5' enhancer fragment) was recovered by use of QIAquick Gel Extraction Kit (Qiagen) in accordance with the instructions.

### 1-4. Obtaining a DNA fragment of the κ 3' enhancer region

Based on the genomic DNA sequence in the vicinity of the murine Igκ gene obtained from the GenBank (NCBI, U.S.A.), the following DNA primers were synthesized.
3' enhancer FW Hd:
CCCAAGCTTAGCTCAAACCAGCTTAGGCTACACA (SEQ ID NO: 7, the HindIII site has been added)
3' enhancer RV Bm-2:
CGGGATCCCTAGAACGTGTCTGGGCCCCATGAA (SEQ ID NO: 8, the BamHI site has been added)

A reaction mixture was prepared by use of KOD-plus- (TOYOBO, Japan) in accordance with the instructions. To the reaction mixture (50 µl), the two primers as prepared above (10 pmol each) and DNA derived from BAC clone RP23-435I4 (GenBank Accession Number: AC090291) as a template were added. After the reaction mixture was kept at 94°C for 3 minutes, a PCR cycle consisting of 94°C for 15 seconds and 68°C for 40 seconds was repeated 30 times. About 0.8 kb amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with HindIII and BamHI and resolved on 0.8% agarose gel. From the cut-out gel, the enzyme-digested fragment was recovered by use of QIAquick Gel Extraction Kit (Qiagen, Germany) in accordance with the instructions.

pBluescriptIISK(-) (Stratagene, U.S.A.) was digested with HindIII and BamHI and separated and purified by 0.8% agarose gel electrophoresis. The' ends of pBluescriptIISK(-) were dephosphorylated with alkaline phosphatase derived from the fetal calf intestine. The DNA fragments recovered above were inserted into the obtained pBluescriptIISK(-), which was then introduced into *Escherichia coli* DH5α. DNA was prepared from the resulting transformant and the inserted fragment was sequenced. Clones having no mutation due to PCR were selected, digested with HindIII and BamHI, and separated on 0.8% agarose gel. From the agarose gel thus recovered, the enzyme-digested fragment (the 3' enhancer fragment) was recovered by use of QIAquick Gel Extraction Kit (Qiagen) in accordance with the instructions.

### 1-5. Obtaining κ polyA fragment

CkP2 KI vector was digested with EcoRI and HindIII, and about 440 bp fragment was separated on 0.8% gel. From the cut-out gel, the enzyme-digested fragment was recovered by use of QIAquick Gel Extraction Kit (Qiagen, Germany) in accordance with the instructions, and the fragment was blunt-ended using Blunting high (Toyobo) to obtain a κ polyA fragment.

### 1-6. Construction of κ 5' enhancer-containing vector· · · · ·pPSsE1

pPSs3.8 of 1-2 above was digested with XhoI and KpnI and separated and purified by 0.8% agarose gel electrophoresis. The ends thereof were dephosphorylated by alkaline phosphatase derived from the fetal calf intestine. Into the resultant was inserted the 5' intron enhancer fragment as recovered in 1-3 above, and the product was then introduced into *Escherichia coli* DH5α. DNA was prepared from the resulting transformant, and the ligated portion was sequenced.

### 1-7. Construction of κ 5' and 3' enhancer-containing vector ···· pPSsE2

pPSsE1 of 1-6 above was digested with BamHI and HindIII and separated and purified by 0.8% agarose gel electrophoresis. The ends thereof were dephosphorylated by alkaline phosphatase derived from the fetal calf intestine. Into the resultant was inserted the 3' enhancer fragment as recovered in 1-4 above, and the product was then introduced into *Escherichia coli* DH5α. DNA was prepared from the resulting transformant, and the ligated portion was sequenced.

### 1-8. Construction of PS in vitro vector· · · · ·pPSs5.5

pPSsE2 of 1-7 above was digested with HindIII and separated and purified by 0.8% agarose gel electrophoresis. The fragment was blunt-ended using Blunting high (Toyobo) and the ends thereof were dephosphorylated by alkaline phosphatase derived from *Escherichia coli* C75. Into the resultant was inserted the κ polyA fragment as recovered in 1-5 above, and the product was then introduced into *Escherichia coli* DH5α. DNA was prepared from the resulting transformant, the ligated portion was sequenced, and clones that had been inserted in the forward direction were selected.

### 1-9. Preparation of human erythropoietin DNA (without a signal sequence)

EPO(-SP) FW:
CAGTCCTGGGCGCCCCACCACGCCT (SEQ ID NO: 9, the SfoI site of EPO was used without modification)
EPO(-SP) RV:
TTGGCCGGCCTCATCTGTCCCCTGTCCTGCAGGCC (SEQ ID NO: 10, including the FseI site)

A reaction mixture was prepared by use of KOD-plus- (TOYOBO, Japan) in accordance with the instructions. To the reaction mixture (50 µl), the two primers as prepared above (10 pmol each) and human EPO cDNA as a template were added. After the reaction mixture was kept at 94°C for 2 minutes, a PCR cycle consisting of 94°C for 15 seconds and 68°C for 1 minute was repeated 30 times. 500 bp amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with SfoI and FseI (NEB) and separated on 0.8% agarose gel. From the cut-out gel, the enzyme-digested fragment was recovered by use of QIAquick Gel Extraction Kit (Qiagen, Germany) in accordance with the instructions.

### 1-10. Construction of pPSs hEPO in vitro vector ..... pPSs hEPO

pPSs5.5 of 1-8 above was digested with SalI and FseI, and the ends thereof were dephosphorylated by alkaline phosphatase derived from *Escherichia coli.* Into the resultant was inserted the DNA fragment prepared in 1-9 above, and the product was then introduced into *Escherichia coli* DH5α. DNA was prepared from the resulting transformant, the nucleotide sequence of the ligated portion was confirmed, and a pPSs hEPO *in vitro* vector was obtained (Fig. 1).

### Example 2

### Construction of pNPs hEPO in vitro vector

### 2-1. Construction of promoter and signal sequence-containing small vector ····· pNPs3.7

Based on the MMU231225 sequence (accession No. AJ231225) obtained from the GenBank, the genomic sequence upstream thereof was obtained from the UCSC mouse genome database. PCR primers that amplify a promoter region and an intron-containing signal sequence were designed.
P2SP FW 1:
CCTTAATTAAATATTTTCCTCCTTCTCCTACCAGTACCCACTCTT (SEQ ID NO: 11, including the Pacl site)
P2SP RV:
TTGGCCGGCCTTGGCGCCTCTGGACAGTATGACTAGAAAAAAGCAAAATAGAG (SEQ ID NO: 12, including the SfoI·FseI sites)

A reaction mixture was prepared by use of KOD-plus- (TOYOBO, Japan) in accordance with the instructions. To the reaction mixture (50 µl), the two primers as prepared above (10 pmol each), 1.6 mM of MgSO₄, and DNA derived from murine ES cells as a template were added. After the reaction mixture was kept at 94°C for 2 minutes, a PCR cycle consisting of 94°C for 15 seconds and 68°C for 1 minute was repeated 30 times. 0.5 kb amplified fragment was separated on 0.8% agarose gel. From the cut-out gel, the amplified fragment was recovered by use of QIAquick Gel Extraction Kit (Qiagen, Germany) in accordance with the instructions. The PCR amplified fragment thus recovered was digested with FseI and PacI (NEB) and separated on 0.8% agarose gel. From the cut-out gel, the enzyme-digested fragment was recovered by use of QIAquick Gel Extraction Kit (Qiagen, Germany) in accordance with the instructions.

After pBS+PFN of 1-1 above was digested with FseI and PacI (NEB), the resulting reaction mixture was subjected to phenol/chloroform extraction and then to ethanol precipitation. The DNA fragment recovered above was inserted thereinto. The resultant was inserted into *Escherichia coli* DH5α. From the resulting transformant, DNA was prepared, and the inserted fragment was sequenced. Clones having no mutation due to PCR were selected.

### 2-2. Construction of κ 5' enhancer-containing vector···· pNPsE1

The vector of 1-9 above was digested with XhoI and KpnI and separated and purified by 0.8% agarose gel electrophoresis. The ends thereof were dephosphorylated by alkaline phosphatase derived from the fetal calf intestine. Into the resultant was inserted the 5' intron enhancer fragment as recovered in 1-3 above, and the product was then introduced into *Escherichia coli* DH5α. DNA was prepared from the resulting transformant, and the ligated portion was sequenced.

### 2-3. Construction of κ 5' and 3' enhancer-containing vector····· pNPsE2

pNPsE1 of 2-2 above was digested with BamHI and HindIII and separated and purified by 0.8% agarose gel electrophoresis. The ends thereof were dephosphorylated by alkaline phosphatase derived from the fetal calf intestine. Into the resultant was inserted the 3' enhancer fragment as recovered in 1-4 above, and the product was then introduced into *Escherichia coli* DH5α. DNA was prepared from the resulting transformant, and the ligated portion was sequenced.

### 2-4. Construction of NP in vitro vector ······ pNPs5.4

pNPs3.7 of 2-1 above was digested with HindIII, the digested fragment was separated and purified by 0.8% agarose gel electrophoresis. The fragment was blunt-ended using Blunting high (Toyobo), and the ends thereof were dephosphorylated by alkaline phosphatase derived from *Escherichia coli* C75. Into the resultant was inserted the κ polyA fragment as recovered in 1-5 above, and the product was then introduced into *Escherichia coli* DH5α. DNA was prepared from the resulting transformant, the ligated portion was sequenced, and clones that had been inserted in the forward direction were selected.

### 2-5. Construction of pNPs hEPO in vitro vector

pNPs5.4 of 2-4 above was digested with Sall and FseI, and the ends thereof were dephosphorylated by alkaline phosphatase derived from *Escherichia coli.* Into the resultant was inserted the DNA fragment prepared in 1-9 above, and the product was then introduced into *Escherichia coli* DH5α. DNA was prepared from the resulting transformant, and the nucleotide sequence of the ligated portion was confirmed, and a pNPs hEPO *in vitro* vector was obtained (Fig. 2).

### Example 3

### Construction of pCks hEPO in vitro vector

### 3-1. Obtaining P2 promoter DNA fragment

CkP2 KI vector was digested with HindIII and SalI, and about 210 bp fragment was separated on 0.8% gel. From the cut-out gel, an enzyme-digested fragment was recovered by use of QIAquick Gel Extraction Kit (Qiagen, Germany) in accordance with the instructions, and the fragment was blunt-ended using Blunting high (Toyobo).

### 3-2. Construction of Ck in vitro vector···· pCks4.9

pNPs5.4 of 2-4 above was digested with PacI and SfoI, the digested fragment was separated and purified by 0.8% agarose gel electrophoresis. The fragment was blunt-ended using Blunting high (Toyobo), and the ends thereof were dephosphorylated by alkaline phosphatase derived from *Escherichia coli* C75. Into the resultant was inserted the P2 promoter fragment obtained in 3-1 above, and the product was then introduced into *Escherichia coli* DH5α. DNA was prepared from the resulting transformant, the ligated portion was sequenced, and clones that had been inserted in the forward direction were selected.

### 3-3. Preparation of human erythropoietin DNA fragment (containing a signal sequence)

hEPO-BI FW:
CGGGATCCCGGCCACCATGGGGGTGCACGAATGTCCTGCCT (SEQ ID NO: 13, containing the BamHI site)
hEPO-Xh RV:
CCGCTCGAGCGCTATCTGTCCCCTGTCCTGCAGGCC (SEQ ID NO: 14, containing the XhoI site)

A reaction mixture was prepared by use of KOD-plus- (TOYOBO, Japan) in accordance with the instructions. To the reaction mixture (50 µl), the two primers as prepared above (10 pmol each), 1.6 mM of MgSO₄, and DNA derived from murine ES cells as a template were added. After the reaction mixture was kept at 94°C for 3 minutes, a PCR cycle consisting of 94°C for 15 seconds and 68°C for 1 minute was repeated 30 times. 0.68 kb amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with BamHI and XhoI (Roche) and separated on 0.8% agarose gel. From the cut-out gel, the enzyme-digested fragment was recovered by use of QIAquick Gel Extraction Kit (Qiagen, Germany) in accordance with the instructions, and the fragment was blunt-ended using Blunting high (Toyobo).

### 3-4. Construction of pCks hEPO in vitro vector···· pCks hEPO

pCks4.9 of 3-2 above was digested with FseI, the digested fragment was separated and purified by 0.8% agarose gel electrophoresis. The fragment was blunt-ended using Blunting high (Toyobo), and the ends thereof were dephosphorylated by alkaline phosphatase derived from *Escherichia coli* C75. Into the resultant was inserted the human erythropoietin DNA fragment obtained in 3-3 above, and the product was then introduced into *Escherichia coli* DH5α. DNA was prepared from the resulting transformant, the ligated portion was sequenced, and clones that had been inserted in the forward direction were selected to obtain a pCks hEPO *in vitro* vector (Fig. 3).

### Example 4

### Introduction of vector into myeloma cell

P3-X63.Ag653 cells were subjected to 4-5 passages in RPMI 1640 medium containing 10% FBS at 37°C in the presence of 6.5% CO₂ and multiplied to approximately 1 x 10⁶ cells. The resulting cells were washed once with PBS, suspended in serum-free RPMI 1640 medium at a concentration of 6 x 10⁵ cells/ml, and fractions of 1 ml each were seeded on each well of a 6-well plate. The GeneJammer Transfection Reagent (6 µl, Stratagene, U.S.A.) was mixed with 100 µl of serum-free RPMI 1640 medium, the resultant was incubated at room temperature for 5 minutes, 5 µl of a solution of pPSs hEPO *in vitro* vector, pNPs hEPO *in vitro* vector, or pCks hEPO *in vitro* vector adjusted at 200 ng/µl with sterilized water was added, and the resultant was incubated at room temperature for 10 minutes to form a complex. A complex of a transfection reagent and a vector was added dropwise to the cells that had been seeded on a 6-well plate, the resultant was cultured at 37°C in the presence of 6.5% CO₂ for 4 hours, and 1.1 ml of RPMI 1640 medium containing 20% FBS was added thereto. After 24 hours, 1 ml of a solution of cultured cells was recovered in order to recover RNA. Forty eight hours after the transfection, the culture supernatant was recovered and then subjected to human EPO ELISA. The DIMRIE-C Reagent (Invitrogen, U.S.A.) was also used as a transfection reagent.

### Example 5

### Comparison of transcription levels of introduced human EPO gene

Twenty four hours after the transfection that had been carried out in Example 4, 1 ml of a solution of cultured cells was recovered, and total RNA was purified by Isogen (Nippon Gene) in accordance with the protocol. After the treatment with DNase, the product was dissolved in RNase-free sterilized water. Total RNA (50 ng) was subjected to reverse transcription using SuperScript III First-Strand Synthesis System (Invitrogen, U.S.A.) in accordance with the protocol. Thereafter, hEPO was subjected to RT-PCR using the following primers.
hEPO-RT FW5: GGCCAGGCCCTGTTGGTCAACTCTTC (SEQ ID NO: 15)
CkpolyA R2: CGCTTGTGGGGAAGCCTCCAAGACC (SEQ ID NO: 16)

As the internal standard, β-actin was subjected to RT-PCR using Mouse β-actin RT-PCR primer set (Toyobo, Japan).

A reaction mixture was prepared by use of LA-Taq (Takara Bio, Japan) in accordance with the instructions. To the reaction mixture (50 µl), the two primers as prepared above (10 pmol each) and cDNA were added. After the reaction mixture was kept at 94°C for 3 minutes, a PCR cycle consisting of 94°C for 15 seconds and 68°C for 1 minute was repeated 26, 29, and 32 times, and the resultants were electrophoresed on 2% agarose gel (Fig. 4). As a result, a vector of pNPs or pPSs type was found to be more effective on expression at the transcription level than the vector of pCks type.

### Example 6

### Comparison of human EPO level in culture supernatant

Forty eight hours after the transfection, the culture supernatant was recovered, and the human erythropoietin level in the culture supernatant was quantified using Quantikine IVD Human EPO Immunoassay (R&D SYSTEMS, U.S.A.) in accordance with the protocol (Fig. 5, Table I). The results indicated that the amount of human EPO secreted in the culture supernatant was increased in the order of the vectors of pCks, pNPs, and pPSs type. Since no significant differences were observed in the vectors of pNPs and pPSs types in the comparative experiment regarding the transcription level in Example 5, significant differences in human EPO level in the culture supernatants of vectors of pNPs and pPSs types were considered to result from differences generated after transcription.

### Example 7

### Murine RS element targeting vector

### (1) Construction of KO basic vector

The plasmid pLoxP-STneo described in WO 00/10383 (described above) was digested with XhoI to obtain a Neo resistant gene (LoxP-Neo) having LoxP sequences at both ends. The both ends of the LoxP-Neo gene were blunt-ended with T4 DNA polymerase to obtain LoxP-Neo-B.

The following DNAs were synthesized to add new restriction sites to pBluescript II SK(-) (TOYOBO, Japan).
LinkAl: TCGAGTCGCGACACCGGCGGGCGCGCCC (SEQ ID NO: 17)
LinkA2: TCGAGGGCGCGCCCGCCGGTGTCGCGAC (SEQ ID NO: 18)
LinkB 1: GGCCGCTTAATTAAGGCCGGCCGTCGACG (SEQ ID NO: 19)
LinkB2: AATTCGTCGACGGCCGGCCTTAATTAAGC (SEQ ID NO: 20)

pBluescript II SK(-) was treated with SalI and XhoI. The resulting reaction mixture was subjected to phenol/chloroform extraction and then to ethanol precipitation. In order to add new restriction sites NruI, SgrAI and AscI to the plasmid, linkers, LinkA 1 and LinkA2, were synthesized. The two linkers each formed of oligo nucleotide DNA were inserted into the plasmid treated with the restriction enzymes and the resulting construct was introduced into *Escherichia coli* DH5α. DNA was prepared from the resulting transformant. In this manner, plasmids pBlueLA were obtained.

Subsequently, pBlueLA was digested with NotI and EcoRI. The resulting reaction mixture was subjected to phenol/chloroform extraction and then to ethanol precipitation. In order to add new restriction sites PacI, FseI and SalI to the plasmid, linkers, LinkB1 and LinkB2, were synthesized. The two linkers each formed of oligo nucleotide DNA were inserted into the plasmid treated with the restriction enzymes and the resulting construct was introduced into *Escherichia coli* DH5α. DNA was prepared from the resulting transformant. In this manner, plasmids pBlueLAB were obtained pBlueLAB was digested with EcoRV, the reaction solution was subjected to phenol/chloroform extraction, followed by ethanol precipitation, LoxP-Neo-B was inserted into the resultant, and the product was then introduced into *Escherichia coli* DH5α. DNA was prepared from the resulting transformant to obtain a pBlueLAB-LoxP-Neo plasmid.

pMC1DT-A (Lifetech Oriental, Japan) was digested with XhoI and SalI and applied to 0.8% agarose gel. About 1 kb band was separated on the agarose gel and DT-A fragment was recovered by use of QIAquick Gel Extraction Kit (Qiagen, Germany) in accordance with the instructions.

After pBlueLAB-LoxP-Neo was digested with XhoI, the resulting reaction mixture was subjected to phenol/chloroform extraction and then to ethanol precipitation. After the DT-A fragment was inserted into the plasmid, the resulting construct was introduced into *Escherichia coli* DH5α. DNA was prepared from the resulting transformant. In this manner, the KO basic vector pBlueLAB-LoxP-Neo-DT-A was obtained.

### (2) Obtaining a 5' genomic region fragment upstream of the murine RS element

Based on the genomic DNA sequence in the vicinity of the murine RS element gene obtained from the GenBank (NCBI, U.S.A.), the following DNA primers were synthesized.
RS5' FW3:
ATAAGAATGCGGCCGCAAAGCTGGTGGGTTAAGACTATCTCGTGAAGTG (SEQ ID NO: 21)
RS5'RV3:
ACGCGTCGACTCACAGGTTGGTCCCTCTCTGTGTGTGGTTGCTGT (SEQ ID NO: 22)

A reaction mixture was prepared by use of KOD-plus- (TOYOBO, Japan) in accordance with the instructions. To the reaction mixture (50 µl), the two primers as prepared above (10 pmol each) and DNA derived from BAC clone RP23-435I4 (GenBank Accession Number: AC090291) as a template were added. After the reaction mixture was kept at 94°C for 2 minutes, a PCR cycle consisting of 94°C for 15 seconds and 68°C for 5 minutes was repeated 33 times. 5 kb amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with NotI and SalI and separated on 0.8% agarose gel. From the cut-out gel, the enzyme-digested fragment was recovered by use of QIAquick Gel Extraction Kit (Qiagen, Germany) in accordance with the instructions.

After pBlueLAB was digested with NotI and Sall, the resulting reaction mixture was subjected to phenol/chloroform extraction and then to ethanol precipitation. The DNA fragment recovered above was inserted into the digested pBlueLAB. The resulting plasmid was inserted into *Escherichia coli* DH5α. From the resulting transformant, DNA was prepared, and the inserted fragment was sequenced. Clones having no mutation due to PCR were selected and digested with NotI and Sall to obtain fragments. Of them, the 5 kb fragment was separated on 0.8% agarose gel. From the cut-out gel, the enzyme-digested fragment was recovered by use of QIAquick Gel Extraction Kit (Qiagen, Germany) in accordance with the instructions.

### (3) Obtaining a 3' genomic region fragment downstream of the murine RS element

The following DNA primers were synthesized based on the genomic DNA sequence in the vicinity of the murine RS element gene obtained from the GenBank (NCBI, U.S.A.).
RS3' FW2:
TTGGCGCGCCCTCCCTAGGACTGCAGTTGAGCTCAGATTTGA (SEQ ID NO: 23) RS3' RV3:
CCGCTCGAGTCTTACTGTCTCAGCAACAATAATATAAACAGGGG (SEQ ID NO: 24)

A reaction mixture was prepared by use of KOD-plus- (TOYOBO, Japan) in accordance with the instructions. To the reaction mixture (50 µl), the two primers as prepared above (10 pmol each) and DNA derived from BAC clone RP23-435I4 (GenBank Accession Number: AC090291) as a template were added. After the reaction mixture was kept at 94°C for 2 minutes, a PCR cycle consisting of 94°C for 15 seconds and 68°C for 2 minutes was repeated 33 times. 2 kb amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with AscI and XhoI and separated on 0.8% agarose gel. From the cut-out gel, the enzyme-digested fragment was recovered by use of QIAquick Gel Extraction Kit (Qiagen, Germany) in accordance with the instructions.

After pBlueLAB was digested with AscI and Xhol, the resulting reaction mixture was subjected to phenol/chloroform extraction and then to ethanol precipitation. The DNA fragment recovered above was inserted into the plasmid pBlueLAB, and the resulting plasmid was inserted into *Escherichia coli* DH5α. From the resulting transformant, DNA was prepared, and the inserted fragment was sequenced. Clones having no mutation due to PCR were selected and digested with AscI and XhoI. The obtained 2 kb fragment was separated on 0.8% agarose gel. From the cut-out gel, the enzyme-digested fragment were recovered by use of QIAquick Gel Extraction Kit (Qiagen, Germany) in accordance with the instructions.

### (4) Insertion of the 3' genomic region fragment downstream of the murine RS element into the basic vector

pBlueLAB-LoxP-Neo-DT-A was digested with AscI and XhoI, and the DNA fragment of about 7.6 kb was separated and purified by 0.8% agarose gel electrophoresis. After the genome fragment prepared in (3) above was inserted thereinto, the resulting plasmid was introduced into *Escherichia coli* XL10-Gold Ultracompetent Cells (Stratagene, U.S.A.). From the resulting transformant, DNA was prepared, and the nucleotide sequence of the ligated portion was confirmed.

### (5) Insertion of the 5' genomic region fragment upstream of the murine RS element into the KO basic vector comprising the 3' genomic region fragment downstream of the murine RS element

After the plasmid obtained in (4) above was digested with NotI and SaII, the resultant DNA fragment of 9.6 kb was separated and purified by 0.8% agarose gel electrophoresis. After the genome fragment prepared in (2) above was inserted thereinto, the resulting plasmid was introduced into *Escherichia coli* XL10-Gold Ultracompetent Cells (Stratagene, U.S.A.). From the resulting transformant, DNA was prepared, and the nucleotide sequence of the ligated portion was confirmed. In this manner, the murine RS element targeting vector pBlueRS-LoxP-Neo-DT-A-3'KO-5'KO was obtained (Fig. 6).

### Example 8

### Preparation of murine RS element targeting vector for electroporation

60 µg of pBlueRS-LoxP-Neo-DT-A-3'KO-5'KO was digested with NotI at 37°C for 5 hours by using a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector linearized with NotI was collected by centrifugation and sterilized by adding 70% ethanol. Then, 70% ethanol was removed in a clean ventilator and the resulting product was air-dried for 1 hour. To the dried product, HBS solution was added to prepare a 0.5 µg/µl DNA solution and stored at room temperature for 1 hour. In this way, the murine RS element targeting vector pBlueRS-LoxP-Neo-DT-A-3'KO-5'KO-NotI for electroporation was prepared.

### Example 9

### Preparation of a probe for Southern analysis of the genome

The following DNA primers were synthesized to obtain oligo DNA containing a 573-mer region upstream of the 5' KO based on the nucleotide sequence information of BAC clone RP23-435I4(GenBank Accession Number: AC090291).
RS5' Southern FW1: CATACAAACAGATACACACATATAC (SEQ ID NO: 25)
RS5' Southern RV2: GTCATTAATGGAAGGAAGCTCTCTA (SEQ ID NO: 26)

A reaction mixture was prepared using Takara Z Taq (Takara Shuzo, Japan) in accordance with the instructions. To the reaction mixture (50 µl), the two primers as prepared above (10 pmol each) and DNA derived from BAC clone RP23-434I as a template were added. After the reaction mixture was kept at 94°C for 2 minutes, a PCR cycle consisting of 94°C for 30 seconds, 60°C for 20 seconds, and 72°C for 1 minute was repeated 25 times. The 573-mer amplified fragment was separated on 0.8% agarose gel. From the cut-out gel, a probe, 5' KO-prob, for Southern analysis of the 5'-side genome was recovered by use of QIAquick Gel Extraction Kit (Qiagen, Germany) in accordance with the instructions.

Based on the nucleotide sequence information of BAC clone RP23-435I4 (GenBank Accession Number: AC090291), the following DNAs were synthesized to obtain oligo DNA containing 600-mer region downstream of 3' KO.
RS3' Southern FW1: TCTTACTAGAGTTCTCACTAGCTCT (SEQ ID NO: 27)
RS3' Southern RV2: GGAACCAAAGAATGAGGAAGCTGTT (SEQ ID NO: 28)

A reaction mixture was prepared by use of Takara Z Taq (Takara Shuzo, Japan) in accordance with the instructions. To the reaction mixture (50 µl), the two primers as prepared above (10 pmol each) and DNA derived from BAC clone RP23-434I as a template were added. After the reaction mixture was kept at 94°C for 2 minutes, a PCR cycle consisting of 94°C for 30 seconds, 60°C for 20 seconds, and 72°C for 1 minute was repeated 25 times. The 600-mer amplified fragment was separated on 0.8% agarose gel. From the cut-out gel, a probe, 3' KO prob, for Southern analysis of the 3' genome side was recovered by use of QIAquick Gel Extraction Kit (Qiagen, Germany) in accordance with the instructions.

### Example 10

### Obtaining RS element targeting murine ES cell

Murine ES cell can generally be established as mentioned below. Male and female mice were crossed. After fertilization, the embryo of 2.5 days old was taken and cultured *in vitro* in a medium for ES cell. The embryo was allowed to develop into the blastocyst stage and separated, and subsequently seeded on the feeder-cell culture medium and cultured. Then, the cell mass which grew in an ES-like form was dispersed in the ES medium containing trypsin, cultured on feeder-cell medium, and further subcultured in the ES medium. The grown cell was isolated.

To obtain RS element targeting murine ES cells by homologous recombination, the pBlueRS-LoxP-Neo-DT-A-3'KO-5'KO prepared in Example 7 was linearized with NotI (Takara Shuzo, Japan) and introduced into murine ES cell TT2F (Yagi et al., Analytical Biochem., 214: 70, 1993) in accordance with the established method (Shinichi Aizawa, Gene Targeting, in Bio-Manual Series 8, 1995, Yodosha, Japan).

TT2F cells were cultured in accordance with the method (Shinichi Aizawa, supra) using, as a trophocyte, the G418 resistant primary cultured cell (Invitrogen, Japan), which was treated with mitomycin C (Sigma, U.S.A.). The TT2F cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 240 V, room temperature). After electroporation, the cells were suspended in 10 ml of ES medium and seeded on a 100 mm plastic tissue-culture Petri dish (Falcon; Becton, Dickinson, U.S.A.) having feeder cells previously seeded therein. After 24 hours, the medium was replaced with fresh ES medium containing 200 µg/ml neomycin (Sigma, U.S.A.). The colonies generated after 7 days were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA of the neomycin-resistant TT2F cells was digested with EcoRI (Takara Shuzo, Japan) and separated by 0.8% agarose gel electrophoresis. Subsequently, Southern blot was performed by use of, as a probe, a DNA fragment (3' KO-prob, see Example 9), which was located downstream of the 3' homologous region of the targeting vector, to detect homologous recombinants. In the wild-type TT2F cell, a single band (about 5.7 kb) was detected by EcoRI digestion. In the homologous recombinant, detection of two bands (about 5.7 kb and about 7.4 kb) was expected. Actually, a new band of about 7.4 kb was detected in the neomycin resistant cell line (Fig. 7). The genomic DNA of clones which were confirmed as homologous recombinants by Southern analysis using 3' KO-prob was further digested with PstI (Takara Shuzo, Japan) and separated by 0.8% agarose gel electrophoresis. Subsequently, Southern blot analysis was performed by use of, as a probe, a DNA fragment (5' KO-prob, see Example 9), which is located upstream of the 5' homologous region of the targeting vector, to detect homologous recombinants. In the wild-type TT2F cell, a single band (about 6.1 kb) was detected by PstI digestion. In the homologous recombinant, detection of two bands (about 6.7 kb and about 6.1 kb) was expected. Actually, a new band of about 6.7 kb was detected in the neomycin resistant cell line (Fig. 7). These clones had the deletion of a vicinal chromosome region comprising the murine RS element, and instead, the insertion of a neomycin resistant gene (comprising restriction sites derived from the targeting vector at both ends). Southern analysis was performed by use of 3' KO-prob and 5' . KO-prob. As a result, when pBlueRS-LoxP-Neo-DT-A-3'KO-5'KO was linearized with NotI, 9 out of 72 cell lines were recombinants (12.5%).

The RS element targeting murine ES cell obtained was analyzed for karyotype in accordance with the method as described by Shinichi Aizawa (supra). As a result, it was confirmed that no abnormal karyotype was found in the ES cells.

### Example 11

### Preparation of chimeric mouse by using RS element targeting murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times. From the resulting mouse individuals, a host embryo was prepared.

The puromycin resistant murine ES cell line (obtained in Example 10 (#32)) was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of #32 (Example 10). When 480 injection embryos were transplanted, 80 chimeric mice were born. Chimeric mouse individuals were identified by evaluating whether the wild coat color (i.e., dark brown) derived from the ES cell was observed in white coat color derived from the host embryo. As a result, 48 out of 80 mice were found to clearly have the wild color partially in the coat color, i.e. having the contribution from the ES cell. In the comparative experiment of human EPO gene at the transcription level conducted in Example 32 below, the comparative experiment regarding the serum EPO level conducted in Example 33 below, and the experiment for peripheral blood cell analysis conducted in Example 34 below, the chimeric mouse obtained in Example 11 was used as the control.

### Example 12

### Preparation of pCκP2 KI vector

### (1) Preparation of a fragment in the vicinity of a cloning site

A genome fragment was prepared in which a mouse immunoglobulin κ-chain promoter (P2 promoter), restriction enzyme recognition sequences (SalI, FseI and NheI recognition sequences), a mouse immunoglobulin κ chain PolyA signal region, and a puromycin resistant gene expression unit, were introduced in order at a site downstream of the mouse immunoglobulin κ chain (Igκ) constant region gene. The method will be described more specifically below.

### (1.1) Preparation of a fragment upstream of a cloning site

The following DNAs were synthesized based on the gene sequence of mouse IgGκ obtained from the GenBank (NCBI, U.S.A.).
igkc1: atctcgaggaaccactttcctgaggacacagtgatagg (SEQ ID NO: 29)
igkc2: atgaattcctaacactcattcctgttgaagctcttgac (SEQ ID NO: 30)

An XhoI recognition sequence was added to the end of 5' primer igkc1, while an EcoRI recognition sequence was added to the end of 3' primer igkc2. A reaction mixture was prepared in accordance with the instructions attached to Takara LA-Taq (Takara Shuzo, Japan). To the reaction mixture (50 µl), the two primers as prepared above (10 pmol each) and, as a template, 25 ng of pBluescript SKII (+) (TOYOBO, Japan) into which a DNA fragment derived from λ clone containing Ig light chain Cκ-Jκ had been cloned (WO 00/10383), were added. After the reaction mixture was kept at 94°C for 1 minute, a PCR cycle consisting of 94°C for 30 seconds and 68°C for 3 minutes was repeated 25 times. The obtained reaction mixture was subjected to phenol/chloroform extraction, ethanol precipitation, digestion with EcoRI and XhoI, and 0.8% agarose gel electrophoresis to resolve the DNA fragment on the gel. Desired DNA fragment was recovered by use of Gene clean II (Bio 101, U.S.A.) to obtain amplified fragment A. After the vector pBluescript II KS- (Stratagene, U.S.A.) was digested with EcoRI and XhoI, the ends of the vector were dephosphorylated with E. *coli* alkaline phosphatase. Into the resultant vector was inserted the amplified fragment A, and the product was then introduced into *Escherichia coli* DH5α. DNA was prepared from the resulting transformant, and the nucleotide sequence was confirmed. In this manner, the plasmid plgCκA was obtained.

### (1.2) Preparation of a fragment downstream of the cloning site

The following DNAs were synthesized based on the mouse IgGκ gene sequence obtained from the GenBank (NCBI, U.S.A.).
igkc3 : atgaattcagacaaaggtcctgagacgccacc (SEQ ID NO: 31)
igkc4: atggatcctcgagtcgactggatttcagggcaactaaacatt (SEQ ID NO: 32)

An EcoRI recognition sequence was added to the end of 5' primer igkc3, while BamHI, XhoI and SalI recognition sequences were added to the end of 3' primer igkc4 in order from the 5' side. A reaction mixture was prepared in accordance with the instructions attached to Takara LA-Taq (Takara Shuzo, Japan). To the reaction mixture (50 µl), the two primers as prepared above (10 pmol each) and, as a template, 25 ng of pBluescript SKII (+) (TOYOBO, Japan) into which a DNA fragment derived from λ clone containing Ig light chain Cκ-Jκ had been cloned (WO 00/10383), were added. After the reaction mixture was kept at 94°C for 1 minute, a PCR cycle consisting of 94°C for 30 seconds, 55°C for 30 seconds, and 72°C for 1 minute was repeated 25 times. The obtained reaction mixture was subjected to phenol/chloroform extraction, ethanol precipitation, digestion with EcoRI and BamHI, and 0.8% agarose gel electrophoresis to resolve the DNA fragment on the gel. Desired DNA fragment was recovered by use of Gene Clean. II (Bio 101, U.S.A.) to obtain amplified fragment B. After the vector pIgCκA was digested with EcoRI and BamHI, the ends of the vector were dephosphorylated with *E*. *coli* alkaline phosphatase. Into the resultant pIgCκA vector was inserted the amplified fragment B, and the product was then introduced into *Escherichia coli* DH5α. DNA was prepared from the resulting transformant, and the nucleotide sequence was confirmed. In this manner, plasmid pIgCκAB was obtained.

### (2) Introduction of puromycin resistant gene

Lox-P Puro plasmid (WO 00/10383) was digested with EcoRI and XhoI and blunt-ended with T4 DNA polymerase. DNA fragments were separated by 0.8% agarose gel electrophoresis. The DNA fragment containing the loxP-puromycin resistant gene was recovered by use of Gene Clean II (Bio 101, U.S.A.). Plasmid pIgCκAB was digested with SalI and blunt-ended. Into the blunt-ended plasmid was inserted the obtained loxP-puromycin resistant gene fragment, and the plasmid was then introduced into *Escherichia coli* DH5α. DNA was prepared from the resulting transformant and the nucleotide sequence of the ligated portion was confirmed. In this manner, plasmid pIgCκABP was obtained.

### (3) Introduction of IRES gene

The following DNAs were synthesized based on the IRES gene sequence derived from encephalomyocarditis virus (available from the GenBank (NCBI, U.S.A.)).
eIRESFW: atgaattcgcccctctccctccccccccccta (SEQ ID NO: 33)
esIRESRV: atgaattcgtcgacttgtggcaagcttatcatcgtgtt (SEQ ID NO: 34)

An EcoRI recognition sequence was added to the end of 5' primer eIRESFW, while EcoRI and SalI recognition sequences were added to the end of 3' primer esIRESRV in order from the 5' side. A reaction mixture was prepared in accordance with the instructions attached to Takara LA-Taq (Takara Shuzo, Japan). To the reaction mixture (50 µl), the two primers as prepared above (10 pmol each) and, as a template, 150 ng of pIREShyg plasmid (Clontech, U.S.A.) were added. After the reaction mixture was kept at 94°C for 1 minute, a PCR cycle consisting of 94°C for 30 seconds, 55°C for 30 seconds, and 72°C for 1 minute was repeated 25 times. The obtained reaction mixture was subjected to 0.8% agarose gel electrophoresis to separate DNA fragments. Desired DNA fragment was recovered by use of Gene Clean II (Bio 101, U.S.A.). The obtained DNA fragment was inserted into pGEM-T vector (Promega, U.S.A.) and then introduced into *Escherichia coli* DH5α. DNA was prepared from the resulting transformant, and the nucleotide sequence was confirmed. In this manner, plasmid IRES-Sal/pGEM was obtained. The plasmid was digested with EcoRI and subjected to 0.8% agarose gel electrophoresis to separate DNA fragments. Desired DNA fragment was obtained by use of Gene Clean II (Bio 101, U.S.A.). The obtained IRES gene was inserted into the pIgCκ ABP plasmid digested with EcoRI and the resulting plasmid was introduced into *Escherichia coli* DH5α. DNA was prepared from the resulting transformant, and the nucleotide sequence of the ligated portion was confirmed. In this manner, plasmid pIgCκABPIRES was obtained.

### (4) Preparation of plasmid pΔCκSaI

Targeting vector plasmid for targeting the immunoglobulin gene κ-light chain described in WO 00/10383 was digested with SacII and was partially digested with EcoRI thereafter. The LoxP-PGKPuro portion was cut out after 0.8% agarose gel electrophoresis and the remaining 14.6 kb DNA was separated from the gel and recovered by use of Gene Clean II (Bio 101, U.S.A.). Into the obtained DNA were inserted the following synthesized DNAs. In this manner, a SalI recognition sequence was introduced.
SalIplus: agtcgaca (SEQ ID NO: 35)
Sal1minus: aatttgtcgactgc (SEQ ID NO: 36)

The obtained plasmid was introduced into *Escherichia coli* DH5α. DNA was prepared from the resulting transformant. In this manner, plasmid pΔCκSal was obtained.

### (5) Preparation of plasmid pKIκ

The pIgCκ ABPIRES plasmid obtained in (3) above was digested with XhoI. The DNA fragments were separated by 0.8% agarose gel electrophoresis. The DNA fragment containing Cκ-IRES-loxP-puromycin resistant gene was recovered by use of Gene Clean II (Bio 101, U.S.A.). After pΔCκSal plasmid prepared in (4) above was digested with SalI, the ends of the plasmid were dephosphorylated with *E. coli* alkaline phosphatase. Into the resulting plasmid was inserted the DNA fragment, and the product was then introduced into *Escherichia coli* DH5α. DNA was prepared from the resulting transformant and the nucleotide sequence of the ligated portion was confirmed. In this manner, plasmid pKIκ was obtained.

### (6) Preparation of pIgCκΔIRES fragment

The plasmid pIgCκ ABPIRES obtained in (3) above was partially digested with EcoRI and BgIII. The DNA fragments were separated by 0.8% agarose gel electrophoresis. The DNA fragment (i.e., pIgCκΔIRES fragment), from which the IRES portion had been removed, was recovered by use of Gene Clean II (Bio 101, U.S.A.).

### (7) Preparation of P2 promoter fragment

The following DNAs were synthesized based on the gene sequence of the mouse Igκ promoter region obtained from the GenBank (NCBI, U.S.A.).
P2F: CCCAAGCTTTGGTGATTATTCAGAGTAGTTTTAGATGAGTGCAT (SEQ ID NO: 37)
P2R: ACGCGTCGACTTTGTCTTTGAACTTTGGTCCCTAGCTAATTACTA (SEQ ID NO: 38)

A HindIII recognition sequence was added to the 5' primer P2F, and a Sail recognition sequence was added to the 3' primer P2R. The DNA fragment amplified with KOD plus (TOYOBO, Japan) using a mouse genome DNA as a template was extracted with phenol/chloroform and recovered by ethanol precipitation. The DNA fragment thus recovered was digested with HindIII and Sall and separated by 0.8% agarose gel electrophoresis. Desired DNA fragment was recovered by use of Gene Clean II (Bio 101, U.S.A.). After pBluescript IIKS-vector (Stratagene, U.S.A.) was digested with HindIII and SalI, the ends of the vector were dephosphorylated with *E. coli* alkaline phosphatase. Into the resultant vector was inserted the amplified fragment, and the product was then introduced into *Escherichia coli* DH5α. DNA was prepared from the resulting transformant, and the nucleotide sequence was confirmed. In this manner, a plasmid containing the gene sequence of the Igκ promoter region was obtained The obtained plasmid was digested with HindIII and SaIl, DNA fragments were separated by 0.8% agarose gel electrophoresis, and P2 promoter fragment was recovered by use of Gene Clean II (Bio 101, U.S.A.).

### (8) Preparation of partial-length CκpolyA fragment

The following DNAs were synthesized based on the gene sequence of the mouse IgCκ polyA region obtained from the GenBank (NCBI, U.S.A.).
PPF:
ACGCGTCGACGCGGCCGGCCGCGCTAGCAGACAAAGGTCCTGAGACGCCACC ACCAGCTCCCC (SEQ ID NO: 39)
PPR:
GAAGATCTCAAGTGCAAAGACTCACTTTATTGAATATTTTCTG (SEQ ID NO: 40)

SalI, FseI and NheI recognition sequences were added to the 5' primer PPF, while BglII recognition sequence was added to the 3' primer PPR. DNA fragment amplified by KOD plus (TOYOBO, Japan) using the murine genomic DNA as a template was recovered by phenol/chloroform extraction and ethanol precipitation. The DNA fragment thus recovered was digested with SalI and BglII and separated by 0.8% agarose gel electrophoresis. Desired DNA fragment was recovered by use of Gene clean II (Bio101, U.S.A.). After pSP72 vector (Promega, U.S.A.) was digested with SalI and BglII, the ends of the vector were dephosphorylated with *E. coli* alkaline phosphatase. Into the resultant vector was inserted the amplified fragment, and the product was then introduced into *Escherichia coli* DH5α. DNA was prepared from the resulting transformant, and the nucleotide sequence was confirmed. In this manner, a plasmid containing the gene sequence of the partial-length CκpolyA region was obtained. After the obtained plasmid was digested with SalI and BglII, DNA fragment was separated by 0.8% agarose gel electrophoresis, and the partial-length CκpolyA fragment was recovered by Gene Clean II (Bio101, U.S.A.).

### (9) Preparation of a full-length CκpolyA fragment

The following DNAs were synthesized based on the gene sequence of the mouse IgCκ polyA region obtained from the GenBank (NCBI, U.S.A.).
TPF:
GGAATTCAGACAAAGGTCCTGAGACGCCACCACCAGCTCCCC (SEQ ID NO: 41)
TPR:
CCCAAGCTTGCCTCCTCAAACCTACCATGGCCCAGAGAAATAAG (SEQ ID NO: 42)

An EcoRI recognition sequence was added to the 5' primer TPF, while a HindIII recognition sequence was added to the 3' primer TPR. DNA fragment amplified by KOD plus (TOYOBO, Japan) using the murine genomic DNA as a template was recovered by phenol/chloroform extraction and ethanol precipitation: The DNA fragment thus recovered was digested with EcoRI and HindIII and separated by 0.8% agarose gel electrophoresis. Desired DNA fragment was recovered by use of Gene clean II (Bio101, U.S.A.). After pBluescript IIKS- vector (Stratagene, U.S.A.) was digested with EcoRI and HindIII, the ends of the vector were dephosphorylated with E. *coli* alkaline phosphatase. Into the resultant vector was inserted the recovered and amplified fragment, and the product was then introduced into *Escherichia coli* DH5α. DNA was prepared from the resulting transformant, and the nucleotide sequence was confirmed. In this manner, a plasmid containing the gene sequence of the full-length Cκ polyA region was obtained. The obtained plasmid was digested with EcoRI and HindIII, DNA fragments were separated by 0.8% agarose gel electrophoresis, and the full-length CκpolyA fragment was recovered by use of Gene clean II (Bio101, U.S.A.).

### (10) Preparation of DNA fragment A consisting of full-length CκpolyA fragment, P2 promoter fragment, and partial-length CκpolyA fragment

After pBluescript IIKS- vector (Stratagene, U.S.A.) was digested with EcoRI and BglII, the ends of the vector were dephosphorylated with *E. coli* alkaline phosphatase. Into the resultant vector were inserted the full-length CκpolyA fragment, the P2 promoter fragment, and the partial-length CκpolyA fragment, and the product was then introduced into *Escherichia coli* DH5α. DNA was prepared from the resulting transformant, and it was confirmed at the nucleotide level that the full-length CκpolyA fragment, P2 promoter fragment, and partial-length CκpolyA fragment were inserted in order. In this manner, the gene sequence of a plasmid containing DNA fragment A was obtained. After the obtained plasmid was digested with EcoRI and BgIII, DNA fragments were separated by 0.8% agarose gel electrophoresis. DNA fragment A was recovered by use of Gene clean II (Bio101, U.S.A.).

### (11) Preparation of pIgCκΔIRES ProA plasmid

Into pIgCκΔIRES fragment whose ends had been dephosphorylated with *E*. *coli* alkaline phosphatase, DNA fragment A was inserted. The resulting plasmid was introduced into *Escherichia coli* DH5α. DNA was prepared from the resulting transformant. Whether DNA fragment A was introduced was confirmed at the nucleotide level. In this manner, the pIgCκΔIRES ProA plasmid containing the gene sequence of DNA fragment A was obtained.

### (12) Preparation of plasmid CκP2H

After pIgCκΔIRES ProA plasmid was digested with XhoI, DNA fragments were separated by 0.8% agarose gel electrophoresis. A DNA fragment constituted of the genomic region upstream of IgCκ, IgCκ, DNA fragment A, and Lox-P Puro fragment was recovered by use of Gene clean II (Bio101, U.S.A.). After plasmid pΔCκSalI was digested with SalI, the ends of the plasmid were dephosphorylated with *E. coli* alkaline phosphatase. Into the pΔCκSalI plasmid was inserted the recovered DNA fragment, and the product was then introduced into *Escherichia coli* XL10-GOLD (Stratagene, U.S.A.). DNA was prepared from the resulting transformant. Whether the DNA fragment constituted of the genomic region upstream of IgCκ, IgCκ, DNA fragment A, and Lox-P Puro fragment was introduced was determined at the nucleotide level. In this manner, the CκP2H plasmid was obtained.

### (13) Preparation of Cκ5' genomic plasmid

The following DNAs were synthesized based on the gene sequence of the mouse IgCκ obtained from the GenBank (NCBI, U.S.A.) and the gene sequence of the upstream genomic region.
5GF:
ATAAGAATGCGGCCGCCTCAGAGCAAATGGGTTCTACAGGCCTAACAACCT (SEQ ID NO: 43)
5GR:
CCGGAATTCCTAACACTCATTCCTGTTGAAGCTCTTGACAATGG (SEQ ID NO: 44)

A NotI recognition sequence was added to the 5' primer 5GF, while an EcoRI recognition sequence was added to the 3' primer 5GR. DNA fragments amplified by KOD plus (TOYOBO, Japan) using the murine genomic DNA as a template were recovered by phenol/chloroform extraction and ethanol precipitation. The DNA fragment thus recovered was digested with NotI and EcoRI and separated by 0.8% agarose gel electrophoresis. Desired DNA fragment was recovered by use of Gene clean II (Bio101, U.S.A.). After pBluescript IIKS- vector (Stratagene, U.S.A.) was digested with NotI and EcoRI, the ends of the vector were dephosphorylated with E. *coli* alkaline phosphatase. Into the resultant vector was inserted the recovered and amplified fragment, and the product was then introduced into *Escherichia coli* DH5α. DNA was prepared from the resulting transformant, and the nucleotide sequence was confirmed. In this manner, the Cκ5' genomic plasmid containing the gene sequence of the Cκ5' genomic region was obtained.

### (14) Preparation of plasmid CκP2KIΔDT

After CκP2H plasmid was digested with EcoRI and XhoI, 11 kb DNA fragment was separated by 0.8% agarose gel electrophoresis. DNA fragment having an EcoRI site at the 5' end and an XhoI site at the 3' end was recovered by use of Gene clean II (Bio101, U.S.A.). After Cκ5' genomic plasmid was digested with EcoRI and XhoI, the ends of the plasmid were dephosphorylated with *E*. *coli* alkaline phosphatase. Into the resulting plasmid was inserted the DNA fragment, and the product was then introduced into *Escherichia coli* XL10-GOLD (Stratagene, U.S.A.). DNA was prepared from the resulting transformant. Whether the recovered fragment was inserted into the Cκ5' genomic plasmid was determined at the nucleotide level. In this manner, the plasmid CκP2KIADT was obtained.

### (15) Preparation of DT-A fragment

After pKIκ plasmid was digested with XhoI and KpnI, DNA fragment of about 1 kb was separated by 0.8% agarose gel electrophoresis and DT-A fragment was then obtained by use of Gene clean II (Bio101, U.S.A.).

### (16) Preparation of pCκP2 KI vector

After plasmid CκP2KIΔDT was digested with Xhol and Kpnl, the ends of the plasmid were dephosphorylated with *E*. *coli* alkaline phosphatase. Into the resulting plasmid was inserted the DT-A fragment and the product was then introduced into *Escherichia coli* XL10-GOLD (Stratagene, U.S.A.). DNA was prepared from the resulting transformant. Whether the DT-A fragment was inserted into the plasmid CκP2KIΔDT was determined at the nucleotide level. In this manner, the pCκP2 KI vector was obtained (Fig. 8).

### Example 13

### Insertion of human EPO gene into pCκP2 KI vector

### (1) Preparation of human erythropoietin DNA fragment

hEPO Np: CCGCTCGAGCGGCCACCATGGGGGTGCACGAATGTCCTG (SEQ ID NO: 45)
hEPO Rp: CCGCTCGAGCGGTCATCTGTCCCCTGTCCTGCA (SEQ ID NO: 46)

A reaction mixture was prepared using KOD-plus- (TOYOBO, Japan) in accordance with the instructions. To the reaction mixture (50 µl), the two primers as prepared above (10 pmol each) and human EPO cDNA as a template were added. After the reaction mixture was kept at 94°C for 2 minutes, a PCR cycle consisting of 94°C for 15 seconds and 68°C for 1 minute was repeated 30 times. 580 bp amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with XhoI and separated on 0.8% agarose gel. From the cut-out gel, the enzyme-digested fragment was recovered by use of QIAquick Gel Extraction Kit (Qiagen, Germany) in accordance with the instructions.

pBluescript IISK(-) (Stratagene, U.S.A.) was digested with XhoI and then separated and purified by 0.8% agarose gel electrophoresis. The ends of the plasmid were dephosphorylated by alkaline phosphatase derived from the fetal calf intestine. Into the resulting plasmid was inserted the DNA fragment as recovered above, and the product was then introduced into *Escherichia coli* DH5α. DNA was prepared from the resulting transformant, and the inserted fragment was sequenced. A clone having no mutation due to PCR was selected, digested with XhoI, and separated on 0.8% agarose gel. From the cut-out gel, the human erythropoietin DNA fragment was recovered by use of QIAquick Gel Extraction Kit (Qiagen, Germany) in accordance with the instructions.

### (2) Construction of pCκP2 hEPO KI vector

After pCκP2 KI vector was digested with SalI, the ends of the vector were dephosphorylated with alkaline phosphatase derived from the fetal calf intestine. Into the resultant vector was inserted the human erythropoietin DNA fragment as prepared in (1) above and the product was then introduced into *Escherichia coli* XL10-Gold Ultracompetent Cells (Stratagene, U.S.A.). DNA was prepared from the resulting transformant and the nucleotide sequence of the ligated portion was confirmed. In this manner, the pCκP2 hEPO KI vector was obtained (Fig. 9).

### Example 14

### Preparation of pCkP2 hEPO KI vector for electroporation

60 µg of pCkP2 hEPO KI vector was digested with NotI at 37°C for 5 hours by using a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector which had been linearized with NotI was collected by centrifugation and sterilized by adding 70% ethanol. Then, 70% ethanol was removed in a clean ventilator and the linearized vector was air-dried for 1 hour. To the dried vector was added an HBS solution, thereby preparing a 0.5 µg/µl DNA solution, and the obtained DNA solution was stored at room temperature for 1 hour. In this manner, the pCkP2 hEPO KI vector for electroporation was prepared.

### Example 15

### Obtaining the CkP2 hEPO murine ES cell line using pCkP2 hEPO KI vector and RS element targeting murine ES cell line

To obtain the murine ES cell line having human EPO-cDNA inserted downstream of the immunoglobulin κ light chain gene by homologous recombination, the pCkP2 hEPO KI vector as prepared in Example 13 was linearized with NotI (Takara Shuzo., Japan) and introduced into the RS element targeting murine ES cell in accordance with the established method (Shinichi Aizawa, Bio-Manual Series 8, Gene Targeting, 1995, Yodosha, Japan).

The RS element targeting murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The RS element targeting murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi, Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U.S.A.). After 7 days, colonies were generated. Of them, 24 colonies were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, Fig. 5), which was at the 3' end of the Ig light chain Jκ-Cκ genomic DNA and had been used in the invention described in WO 00/10383 (see Example 48), to detect homologous recombinants. As a result, 15 homologous recombinants were obtained out of 24 clones (62.5%). In the wild-type TT2F cell, a single band was detected by EcoRI digestion. In the homologous recombinants, a new band was expected to appear below this band (Fig. 10). Actually, the new band was detected in the puromycin resistant cell line. In short, these clones had human EPO-cDNA inserted downstream of the immunoglobulin κ-chain gene of one of the alleles.

### Example 16

### Preparation of chimeric mouse by using CkP2 hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times. From the resulting mouse individuals, a host embryo was prepared.

The CkP2 hEPO murine ES cell lines (obtained in Example 15 (#57, #69)), which were confirmed that human EPO-cDNA had been inserted downstream of the immunoglobulin κ light chain gene, were thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of ES cells #57 and 69 (Example 15). When 100 injection embryos were transplanted, 23 and 36 chimeric mice were born, respectively. Chimeric mouse individuals were identified by evaluating whether the wild coat color (i.e., dark brown) derived from the ES cell was observed in white coat color derived from the host embryo. As a result, 15 and 29 out of 23 and 36 mice were found to clearly have the wild color partially in the coat color, i.e. having the contribution from the ES cell, respectively. From these results, it was demonstrated that the Ck EPO murine ES cell line #57 and 69, wherein both cell lines had human EPO-cDNA inserted downstream of the immunoglobulin κ chain gene, had a chimera formation potency, or a potency differentiating into normal murine tissue.

### Example 17

### Construction of pNP hEPO KI vector

### 1. Preparation of vector with the addition of linearized AscI site to pCkP2 KI vector of Example 12 ··· pCkP2+As KI

The following synthetic oligo DNAs were annealed to synthesize AscI linkers.
AscI top linker: GGCCAGGCGCGCCTTGC (SEQ ID NO: 47)
AscI bottom linker: GGCCGCAAGGCGCGCCT (SEQ ID NO: 48)

pCkP2 KI vector was digested with NotI (Roche), the digested fragment was separated and purified by 0.8% agarose gel electrophoresis, and AscI linkers were then introduced via ligation using the ligation kit ver 2 (Takara Bio). The product was introduced into *Escherichia coli* XL10-Gold Ultracompetent Cells (Stratagene, U.S.A.), DNA was prepared from the resulting transformant, and the nucleotide sequence of the inserted fragment was confirmed.

### 2. Introduction of NheI site between PacI and FseI of pBlueLAB ····· construction of pBlueLAB+Nh

pBlueLAB was digested with PacI (NEB), and buffer exchange was carried out using S-200 HR Microspin columns for nucleic acid purification (Amersham), followed by digestion with FseI (NEB). The digested fragment was separated and purified by 0.8% agarose gel electrophoresis, the synthetic oligo DNAs shown below were annealed, and the resultant was introduced via ligation using the ligation kit ver. 2 (Takara Bio, Japan). The resultant was introduced into *Escherichia coli* DH5α, DNA was prepared from the resulting transformant, and the inserted fragment was sequenced.
Pac-Nhe-Fse S: TAAGGGCTAGCTAGGGCCGG (SEQ ID NO: 49)
Pac-Nhe-Fse AS: CCCTAGCTAGCCCTTAAT (SEQ ID NO: 50)

### 3. Introduction of HpaI site between SalI and HindIII of pBlueLAB+Nh····· Construction of pBlueLAB+NhHp

pBlueLAB+Nh was digested with SalI and HindIII (Roche), the digested fragment was separated and purified by 0.8% agarose gel electrophoresis, the synthetic oligo DNAs shown below were annealed, and the resultant was introduced via ligation using the ligation kit ver. 2 (Takara Bio, Japan). The resultant was introduced into *Escherichia coli* DH5α, DNA was prepared from the resulting transformant, and the inserted fragment was sequenced.
S/HpaI/Hd-S: TCGAGTTAAC (SEQ ID NO: 51)
S/HpaI/Hd-AS: AGCTGTTAAC (SEQ ID NO: 52)

### 4. Vector prepared from pCkP2+As KI by removing Ck-polyA-containing P2 promoter region therefrom···· Construction of pCkpAP2

pCkP2+As KI was digested with HpaI-NheI (Roche), and a resulting 952-bp fragment was recovered by separation and purification by 0.8% agarose gel electrophoresis. pBlueLAB+NhHp was digested with HpaI and NheI (Roche) and separated and purified by 0.8% agarose gel electrophoresis. The ends thereof were dephosphorylated by alkaline phosphatase derived from the fetal calf intestine (Takara Bio, Japan). Into the resultant was introduced the 952-bp fragment obtained above via ligation using the ligation kit ver. 2 (Takara Bio, Japan). The resultant was introduced into *Escherichia coli* DH5α, DNA was prepared from the resulting transformant, and the ligated portion was sequenced.

### 5. Vector prepared by removing P2 promoter and introducing multicloning sites (SalI, FseI, and PacI) therein ··· pCkpAMCS

pCkpAP2 of 4 above was digested with HindIII and NheI (Roche), and about 4 kb fragment was recovered by 0.8% agarose gel electrophoresis. The synthetic oligo DNAs shown below were annealed, and the resultant was introduced via ligation using the ligation kit ver. 2 (Takara Bio, Japan).
SPFN linker-S: AGCTGTCGACTTAATTAAGGCCGGCCG (SEQ ID NO: 53)
SPFN linker-AS: CTAGCGGCCGGCCTTAATTAAGTCGAC (SEQ ID NO: 54)

### 6. Construction of large vector KI · · · · · pCkP2ΔP

pCkpAMCS of 5 above was digested with HpaI and NheI (Roche), and about 700-bp fragment containing Ck-polyA-MCS was recovered by 0.8% agarose gel electrophoresis. pCkP2+As KI was digested with HpaI-NheI (Roche), a 19.6 kb fragment was recovered by 0.8% agarose gel electrophoresis, and the ends thereof were dephosphorylated by alkaline phosphatase derived from *Escherichia coli* (Takara Bio, Japan). The fragment of about 700 bp above was introduced via ligation using the ligation kit ver. 2 (Takara Bio, Japan) The resultant was introduced into *Escherichia coli* XL10-Gold Ultracompetent Cells (Stratagene, U.S.A.), DNA was prepared from the resulting transformant, and the nucleotide sequence of the ligated portion was confirmed.

### 7. Construction of pNP hEPO KI vector

The pNPshEPO *in vitro* vector of Example 2 was digested with SalI and FseI, and about 1.2-kb fragment was separated and purified by 0.8% agarose gel electrophoresis. pCkP2Δ of 6 above was digested with Sall and FseI, and the ends thereof were dephosphorylated by alkaline phosphatase derived from *Escherichia coli* C75. Into the resultant was introduced the fragment of about 1.2-kb above. The resultant was introduced into *Escherichia coli* XL10-Gold Ultracompetent Cells (Stratagene, U.S.A.). DNA was prepared from the resulting transformant, and the nucleotide sequence of the ligated portion was confirmed to obtain a pNP hEPO KI vector (Fig. 11).

### Example 18

### Preparation of pNP hEPO KI vector for electroporation

60 µg of the pNP hEPO KI vector was digested with NotI at 37°C for 5 hours in a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector that was linearized with NotI was collected by centrifugation and sterilized by adding 70% ethanol. Then, 70% ethanol was removed in a clean ventilator, and the residue was air-dried for 1 hour. To this matter was added an HBS solution to prepare a 0.5 µg/µl DNA solution, which was stored at room temperature for 1 hour. In this way, pNP hEPO KI vector for electroporation was prepared.

### Example 19

### Obtaining NP hEPO murine ES cell line using pNP hEPO KI vector and RS element targeting murine ES cell line

To obtain a murine ES cell line with human EPO-cDNA inserted downstream of the immunoglobulin κ light chain gene by homologous recombination, the pNP hEPO KI vector as prepared in Example 18 was linearized with Notl (Takara Shuzo, Japan) and introduced into the RS element targeting murine ES cell line in accordance with the established method (Shinichi Aizawa, Bio-Manual Series 8, Gene Targeting, 1995, Yodosha, Japan).

The RS element targeting murine ES cell was cultured in accordance with the method of Shinichi Aizawa (supra) using, as a trophocyte, the G418 resistant primary cultured cell (Invitrogen, Japan) which had been treated with mitomycin C (Sigma, U.S.A.). The RS element targeting murine ES cells grown were treated with trypsin and suspended in HBS at 3 × 10⁷ cells/ml. Thereafter, 0.5 ml of the cell suspension was mixed with 10 µg of the vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U.S.A.). After 7 days, colonies were generated. Of them, 24 colonies were picked up and grown up to the confluent state in 24-well plates. Two thirds of the grown cells were suspended in 0.2 ml of a stock medium (FBS+10% DMSO, Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (Xhol to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect homologous recombinants. As a result, 11 homologous recombinants were obtained out of 24 clones (45.8%). In the wild-type TT2F cell, a single band was detected by EcoRI digestion. In the homologous recombinants, a new band was expected to appear below this band (Fig. 12). Actually, the new band was detected in the puromycin resistant cell line. In short, these clones had human EPO-cDNA inserted downstream of the immunoglobulin κ-light chain gene of one of the alleles.

### Example 20

### Preparation of chimeric mouse by using NP hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times From the resulting mouse individuals, a host embryo was prepared.

The NP hEPO murine ES cell line (obtained in Example 19 (#10)), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of ES cell #10 (Example 19). When 100 injection embryos were transplanted, 18 chimeric mice were born. Chimeric mouse individuals were identified by evaluating whether the wild coat color (i.e., dark brown) derived from the ES cell was observed in white coat color derived from the host embryo. As a result, 13 out of 23 or 36 mice were found to clearly have the wild color partially in the coat color, i.e. having the contribution from the ES cell. From these results, it was demonstrated that the NP hEPO murine ES cell line #10 comprising human EPO-cDNA inserted downstream of the immunoglobulin κ chain gene had a chimera formation potency, or a potency differentiating into normal murine tissue.

### Example 21

### Construction of pPS hEPO KI vector

pPSs hEPO *in vitro* vector of Example 1 was digested with SalI and XhoI, and a fragment of about 1.3 kb was separated and purified by 0.8% agarose gel electrophoresis. The pCkP2ΔP was digested with Sall and FseI, and the ends thereof were dephosphorylated with alkaline phosphatase derived from *Escherichia coli* C75. Into the resultant was inserted the fragment of about 1.3 kb above. The resultant was introduced into *Escherichia coli* XL10-Gold Ultracompetent Cells (Stratagene, U.S.A.). DNA was prepared from the resulting transformant and the nucleotide sequence of the ligated portion was confirmed. In this manner, the pPS hEPO KI vector was obtained (Fig. 13).

### Example 22

### Preparation of pPS hEPO KI vector for electroporation

60 µg of pPS hEPO KI vector was digested with NotI at 37°C for 5 hours by using a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector which had been linearized with NotI was collected by centrifugation and sterilized by adding 70% ethanol. Then, 70% ethanol was removed in a clean ventilator and the linearized vector was air-dried for 1 hour. To the dried vector was added an HBS solution, thereby preparing a 0.5 µg/µl DNA solution, and the obtained DNA solution was stored at room temperature for 1 hour. In this manner, the pPS hEPO KI vector for electroporation was prepared.

### Example 23

### Obtaining PS hEPO murine ES cell line using pPS hEPO KI vector and RS element targeting murine ES cell line

To obtain a murine ES cell line with human EPO-cDNA inserted downstream of the immunoglobulin κ light chain gene by homologous recombination, the pPS hEPO KI vector as prepared in Example 21 was linearized with NotI (Takara Shuzo, Japan) and introduced into the RS element targeting murine ES cell line in accordance with the established method (Shinichi Aizawa, Bio-Manual Series 8, Gene Targeting, 1995, Yodosha, Japan).

The RS element targeting murine ES cell was cultured in accordance with the method of Shinichi Aizawa (supra) using, as a trophocyte, the G418 resistant primary cultured cell (Invitrogen, Japan) which had been treated with mitomycin C (Sigma, U.S.A.). The RS element targeting murine ES cells grown were treated with trypsin and suspended in HBS at 3 × 10⁷ cells/ml. Thereafter, 0.5 ml of the cell suspension was mixed with 10 µg of the vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U. S.A.). After 7 days, colonies were generated. Of them, 24 colonies were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect homologous recombinants. As a result, 9 homologous recombinants were obtained out of 24 clones (37.5%). In the wild-type TT2F cell, a single band was detected by EcoRI digestion. In the homologous recombinants, a new band was expected to appear below this band (Fig. 14). Actually, the new band was detected in the puromycin resistant cell line. In short, these clones had human EPO-cDNA inserted downstream of the immunoglobulin κ-light chain gene of one of the alleles.

### Example 24

### Preparation of chimeric mouse by using PS hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times. From the resulting mouse individuals, a host embryo was prepared.

The PS hEPO murine ES cell line (obtained in Example 23 (#6)), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of PS EPO murine ES cell #6 (Example 23). When 160 injection embryos were transplanted, 53 chimeric mice were born. Chimeric mouse individuals were identified by evaluating whether the wild coat color (i.e., dark brown) derived from the ES cell was observed in white coat color derived from the host embryo. As a result, 26 out of 53 mice were found to clearly have the wild color partially in the coat color, i.e. having the contribution from the ES cell. From these results, it was demonstrated that the PS hEPO murine ES cell line #6 comprising human EPO-cDNA inserted downstream of the immunoglobulin κ chain gene had a chimera formation potency, or a potency differentiating into normal murine tissue.

### Example 25

### Obtaining CkP2 hEPO murine TT2F cell line using pCkP2 hEPO KI vector and murine TT2F cell line

To obtain a murine ES cell line with human EPO-cDNA inserted downstream of the immunoglobulin κ light chain gene by homologous recombination, the pCkP2 hEPO KI vector as prepared in Example 13 was linearized with NotI (Takara Shuzo, Japan) and introduced into the murine ES cell line TT2F (Yagi et al., Analytical Biochemistry, 214: 70, 1993) in accordance with the established method (Shinichi Aizawa, Bio-Manual Series 8, Gene Targeting, 1995, Yodosha, Japan).

The murine TT2F cells were cultured in accordance with the method of Shinichi Aizawa (supra) using, as a trophocyte, the G418 resistant primary cultured cell (Invitrogen, Japan) which had been treated with mitomycin C (Sigma, U.S.A.). The murine TT2F cells grown were treated with trypsin and suspended in HBS at 3 × 10⁷ cells/ml. Thereafter, 0.5 ml of the cell suspension was mixed with 10 µg of the vector DNA, placed in a gene pulsar cuvette (distance between electrodes: 0.4 cm; Biorad, U.S.A.), and subjected to electroporation (capacity: 960 µF, voltage: 240 V, room temperature). After electroporation, the cells were suspended in 10 ml of the ES medium (Shinichi Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U.S.A.). After 7 days, colonies were generated. Of them, 96 colonies in total were picked up and grown up to the confluent state in 24-well plates. Two thirds of the grown cells were suspended in 0.2 ml of a stock medium (ES medium+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect homologous recombinants. As a result, 11 homologous recombinants were obtained out of 96 clones (11.5%). In the wild-type TT2F cell, a single band was detected by EcoRI digestion. In the homologous recombinants, a new band was expected to appear below this band (Fig. 15). Actually, the new band was detected in the puromycin resistant cell line. In short, these clones had human EPO-cDNA inserted downstream of the immunoglobulin κ-light chain gene of one of the alleles.

### Example 26

### Obtaining CkP2 AP hEPO murine TT2F cell line by removing drug resistant gene from CkP2 hEPO murine TT2F cell line

To obtain a TT2F cell line into which the CkP2 ΔP hEPO gene has been introduced from the CkP2 hEPO murine TT2F cell line by removing a drug resistant gene (Puro^{r}), the pCAGGS-Cre vector (Sunaga et al., Mol Reprod Dev., 46: 109-113, 1997) was introduced into the CkP2 hEPO murine TT2F cell in accordance with the established method (Shinichi Aizawa, Bio-Manual Series 8, Gene Targeting, 1995, Yodosha, Japan).

The CkP2 hEPO murine TT2F cells were cultured in accordance with the method of Shinichi Aizawa (supra) using, as a trophocyte, the G418 resistant primary cultured cell (Invitrogen, Japan) which had been treated with mitomycin C (Sigma, USA.). The CkP2 hEPO murine TT2F cells grown were treated with trypsin and suspended in HBS at 3 × 10⁷ cells/ml. Thereafter, 0.5 ml of the cell suspension was mixed with 10 µg of the vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra), and 2.5 ml thereof was seeded on a 60 mm plastic tissue-culture Petri dish (Falcon; Becton Dickinson, U.S.A.) having feeder cells previously seeded therein. After 30 hours, 1,000 ES cells were seeded on a 100 mm plastic tissue-culture Petri dish (Falcon; Becton Dickinson, U.S.A.) having feeder cells previously seeded therein. After 6 days, colonies were generated. Of them, 48 colonies were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, Fig. 5), which was at the 3' end of the Ig light chain Jx-Cx genomic DNA and had been used in the invention described in WO 00/10383 (see Example 48), to detect ES cell lines from which the Puro^{r} gene flanked by LoxP sequences had been selectively removed. In the case of the Puro^{r} gene-carrying ES cells, two bands (15.6 K and 13.1 K) were detected upon digestion with EcoRI. In the case of the ES cells from which the Puro^{r} gene had been selectively removed, two bands (15.6 K and 10.2 K) were detected upon digestion with EcoRI (Fig. 15). As a result, 4 cells out of 48 cells were found to be TT2F cells (CkP2 ΔP hEPO murine TT2F cell line) (8.3%) prepared from the CkP2 hEPO murine TT2F cell line by removing the drug resistant gene (Puro^{r}).

### Example 27

### Preparation of chimeric mouse by using CkP2 ΔP hEPO murine TT2F cell line and B-lymphocyte deficient murine host embryo

A homozygote in which the immunoglobulin µl chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times. From the resulting mouse individuals, a host embryo was prepared.

The CkP2 ΔP hEPO murine TT2F cell line (obtained in Example 26 (#8)), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of #8 (Example 26). When 80 injection embryos were transplanted, 14 chimeric mice were born. Chimeric mouse individuals were identified by evaluating whether the wild coat color (i.e., dark brown) derived from the ES cell was observed in white coat color derived from the host embryo. As a result, 10 out of 14 mice were found to clearly have the wild color partially in the coat color, i.e. having the contribution from the ES cell. From these results, it was demonstrated that the CkP2 ΔP hEPO murine TT2F cell line #8 comprising human EPO-cDNA inserted downstream of the immunoglobulin κ chain gene had a chimera formation potency, or a potency differentiating into normal murine tissue.

### Example 28

### Obtaining CkP2 loxP hEPO murine ES cell line by removing drug resistant gene from CkP2 hEPO murine ES cell line

To obtain an ES cell line into which the CkP2 loxP hEPO gene has been introduced, from the CkP2 hEPO murine ES cell line by removing 2 types of drug resistant genes (Neo^{r} and Puro^{r}), the pCAGGS-Cre vector (Sunaga et al., Mol Reprod Dev., 46: 109-113, 1997) was introduced into the CkP2 hEPO murine ES cell (#30) in accordance with the established method (Shinichi Aizawa, Bio-Manual Series 8, Gene Targeting, 1995, Yodosha, Japan).

The CkP2 hEPO murine ES cells were cultured in accordance with the method of Shinichi Aizawa (supra) using, as a trophocyte, the G418 resistant primary cultured cell (Invitrogen, Japan) which had been treated with mitomycin C (Sigma, U.S.A.). The CkP2 hEPO murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of ES medium (Shinichi Aizawa, supra), and 2.5 ml thereof was seeded on a 60 mm plastic tissue-culture Petri dish (Falcon; Becton Dickinson, U.S.A.) having feeder cells previously seeded therein. After 30 hours, 1,000 ES cells were seeded on a 100 mm plastic tissue-culture Petri dish (Falcon; Becton Dickinson, .U.S.A.) having feeder cells previously seeded therein. After 6 days, colonies were generated. Of them, 46 colonies were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the murine ES cells was digested with EcoRI (Takara Shuzo, Japan) and separated by agarose gel electrophoresis. Subsequently, Southern blot was performed by use of, as a probe, a DNA fragment (Xhol to EcoRI, about 1.4 kb, WO 00/10383, Fig. 5), which was at the 3' end of the Ig light chain Jκ-Cκ genomic DNA and had been used in the invention described in WO 00/10383 (see Example 48), to detect ES cell lines from which the Puro^{r} gene flanked by LoxP sequences had been selectively removed. In the case of the Puro^{r} gene-carrying ES cells, two bands (15.6 K and 13.1 K) were detected upon digestion with EcoRI. In the case of the ES cells from which the Puro^{r} gene had been selectively removed, two bands (15.6 K and 10.2 K) were detected upon digestion with EcoRI (Fig. 10). With the use of a Southern blot membrane obtained in the same manner as described above, an ES cell line from which the Neo^{r} gene flanked by the LoxP sequences had been selectively removed was detected using the 3' KO-prob used in Example 10 as a probe. In the case of the Neo^{r} gene-carrying ES cells, two bands (7.4 K and 5.7 K) were detected upon digestion with EcoRI. In the case of the ES cells from which the Neo^{r} gene had been selectively removed, two bands (5.7 K and 4.4 K) were detected upon digestion with EcoRI (Fig. 10). As a result, 1 cell out of 46 cells was found to be an ES cell line (CkP2 loxP hEPO murine ES cell line) (2.2%) prepared from the CkP2 human EPO gene-introduced ES cell line from which two types of drug resistant genes (Neo^{r} and Puro^{r}) had been simultaneously removed.

### Example 29

### Preparation of chimeric mouse by using CkP2 loxP hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times From the resulting mouse individuals, a host embryo was prepared.

The CkP2 loxP hEPO murine ES cell line (obtained in Example 28 (#18)), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of #18 (Example 28). When 240 injection embryos were transplanted, 80 chimeric mice were born. Chimeric mouse individuals were identified by evaluating whether the wild coat color (i.e., dark brown) derived from the ES cell was observed in white coat color derived from the host embryo. As a result, 52 out of 80 mice were found to clearly have the wild color partially in the coat color, i.e. having the contribution from the ES cell. From these results, it was demonstrated that the CkP2 loxP hEPO murine ES cell line #18 comprising human EPO-cDNA inserted downstream of the immunoglobulin κ chain gene had a chimera formation potency, or a potency differentiating into normal murine tissue.

### Example 30

### Obtaining PS loxP hEPO murine ES cell line by removing drug resistant gene from PS hEPO murine ES cell line

To obtain an ES cell line into which the PS loxP hEPO gene has been introduced from the PS hEPO murine ES cell line by removing 2 types of drug resistant genes (Neo^{r} and Puro^{r}), the pCAGGS-Cre vector (Sunaga et al, Mol Reprod Dev., 46: 109-113, 1997) was introduced into the PS hEPO murine ES cell (#6) in accordance with the established method (Shinichi Aizawa, Bio-Manual Series 8, Gene Targeting, 1995, Yodosha, Japan).

The PS hEPO murine ES cells were cultured in accordance with the method of Shinichi Aizawa (supra) using, as a trophocyte, the G418 resistant primary cultured cell (Invitrogen, Japan) which had been treated with mitomycin C (Sigma, U.S.A.). The PS hEPO murine ES cells grown were treated with trypsin and suspended in HBS at 3 × 10⁷ cells/ml. Thereafter, 0.5 ml of the cell suspension was mixed with 10 µg of the vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra), and 2.5 ml thereof was seeded on a 60 mm plastic tissue-culture Petri dish (Falcon; Becton Dickinson, U.S.A.) having feeder cells previously seeded therein. After 30 hours, 1,000 ES cells were seeded on a 100 mm plastic tissue-culture Petri dish (Falcon; Becton Dickinson, U.S.A.) having feeder cells previously seeded therein. After 6 days, colonies were generated. Of them, 192 colonies were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, Fig. 25), which was at the 3' end of the Ig light chain Jκ-Cκ genomic DNA and had been used in the invention described in WO 00/10383 (see Example 48), to detect ES cell lines from which the Puro^{r} gene flanked by LoxP sequences had been selectively removed. In the case of the Puro^{r} gene-carrying ES cells, two bands (15.6 K and 12.7 K) were detected upon digestion with EcoRI. In the case of the ES cells from which the Puro^{r} gene had been selectively removed, two bands (15.6 K and 9.8 K) were detected upon digestion with EcoRI (Fig. 14). With the use of a Southern blot membrane obtained in the same manner as described above, an ES cell line from which the Neo^{r} gene flanked by the LoxP sequences had been selectively removed was detected using the 3' KO-prob used in Example 10 as a probe. In the case of the Neo^{r} gene-carrying ES cells, two bands (7.4 K and 5.7 K) were detected upon digestion with EcoRI. In the case of the ES cells from which the Neo^{r} gene had been selectively removed, two bands (5.7 K and 4.4 K) were detected upon digestion with EcoRI (Fig. 14). As a result, 2 cells out of 192 cells were found to be an ES cell line (PS loxP hEPO murine ES cell line) (1.0%) prepared from the PSEPO murine ES cell line from which two types of drug resistant genes (Neo^{r} and Puro^{r}) had been simultaneously removed.

### Example 31

### Preparation of chimeric mouse by using PS loxP hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci: U.S.A., 97: 722-7, 2000) was back-crossed with MCH (ICR) (CLEA Japan, Japan) three or more times. From the resulting mouse individuals, a host embryo was prepared.

The PS loxP hEPO murine ES cell line (obtained in Example 30 (#48)), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of #48 (Example 30). When 240 injection embryos were transplanted, 70 chimeric mice were born. Chimeric mouse individuals were identified by evaluating whether the wild coat color (i.e., dark brown) derived from the ES cell was observed in white coat color derived from the host embryo. As a result, 34 out of 70 mice were found to clearly have the wild color partially in the coat color, i.e. having the contribution from the ES cell. From these results, it was demonstrated that PS loxP hEPO murine ES cell line #48 comprising human EPO-cDNA inserted downstream of the immunoglobulin κ chain gene had a chimera formation potency, or a potency differentiating into normal murine tissue.

### Example 32

### Comparison of human EPO gene transcription levels among CkP2 hEPO murine ES cell-derived chimeric mouse, NP hEPO murine ES cell-derived chimeric mouse, and PS hEPO murine ES cell-derived chimeric mouse

### Preparation of mRNA sample from spleen of chimeric mouse:

As controls, spleen samples were extracted from 1-week-old, 2-week-old, and 4-week-old mice each from 3 chimeric mice derived from the RS element targeting murine ES cell line #32 prepared in Example 11, 3 chimeric mice derived from the CkP2 hEPO murine ES cell line #57 prepared in Example 16 (chimeric rate: 100% to 70%), 3 chimeric mice derived from the NP hEPO murine ES cell line #10 prepared in Example 20 (chimeric rate: 100%), and 3 chimeric mice derived from the PS hEPO murine ES cell line #6 prepared in Example 24 (chimeric rate: 100%). The spleens (about 50 mg) were subjected to freezing in liquid nitrogen immediately thereafter. Isogen (1 ml, Nippon Gene, Japan) was added to the frozen samples, the samples were broken using a homogenizer, and RNA was extracted in accordance with the instructions. The resulting RNA samples were subjected to DNase treatment at 37°C for 15 minutes (deoxyribonuclease; RT-grade, Wako Pure Chemical Industries Ltd., Japan). Further, purified RNA samples were obtained using RNasy Mini (Qiagen, Germany).

### Comparison of human EPO gene transcription levels:

cDNA was synthesized from 250 ng of the resulting purified RNA samples using SuperScript III (Invitrogen). Thereafter, the resultant was subjected to RNase treatment at 37°C for 20 minutes, and 2.0 µl of 1:10 diluent with RNase-free sterilized water was used in the subsequent PCR procedure.

As primers for confirming human EPO gene expression, the following oligo DNAs were synthesized.
hEPO-RT FW5: GGCCAGGCCCTGTTGGTCAACTCTTC (SEQ ID NO: 55)
CkpolyAR2: CGCTTGTGGGGAAGCCTCCAAGACC (SEQ ID NO: 56)

PCR was carried out under the following conditions in order to confirm expression of the human EPO gene. A two-step cycle consisting of incubation at 94°C for 10 seconds and incubation at 68°C for 1 minute was repeated 35 times using LA taq (Takara Shuzo, Japan). As a result, no amplified band was detected in 1-week-old, 2-week-old, and 4-week-old mice of the control group; however, amplified band was detected in spleen tissue of all 1-week-old or older chimeric mice that had been prepared with the use of murine ES cells into which human EPO genes had been introduced. The density of the amplified bands each representing the 2-week-old and 4-week-old NP hEPO murine ES cell-derived chimeric mice was substantially the same as that each representing the PS hEPO murine ES cell-derived chimeric mice, and the density of the amplified bands each representing the CkP2 hEPO murine ES cell-derived chimeric mice was somewhat lower than the above density (Fig. 16).

In order to confirm that the amounts of mRNA used are uniform among sample groups, the following oligo DNAs were synthesized as primers for confirming expression of the murine glyceraldehyde-3-phosphate dehydrogenase (GAPDH) gene.
mGAPDH5: CACCATGGAGAAGGCCGGGGCCCAC (SEQ ID NO: 57)
mGAPDH3: ATCATACTTGGCAGGTTTCTCCAGG (SEQ ID NO: 58)

PCR was carried out under the following conditions in order to confirm expression of the murine GAPDH gene. A three-step PCR cycle of incubation at 94°C for 30 seconds, 65°C for 30 seconds, and 72°C for 30 seconds was repeated 25 times using LA taq (Takara Shuzo, Japan). As a result, murine GAPDH expression levels were found to be substantially the same among all samples. Also, differences in band density among samples that had been detected using primers for confirming human EPO gene expression were found to indicate differences in human EPO gene expression levels in spleen tissue of chimeric mice (Fig. 16). These results indicate that a vector of pNP or pPS type is more effective on gene expression at the transcription level compared with a vector of pCkP2 type. These results were found to be correlated with the results obtained by the *in vitro* experiment of Example 5.

### Example 33

### Comparison of serum human EPO levels of CkP2 hEPO murine ES cell-derived chimeric mouse, NP hEPO murine ES cell-derived chimeric mouse, PS hEPO murine ES cell-derived chimeric mouse, Ck ΔP hEPO murine TT2F cell-derived chimeric mouse, CkP2 loxP hEPO murine ES cell-derived chimeric mouse, PS loxP hEPO murine ES cell-derived chimeric mouse

As controls, blood samples were obtained from the orbital sinus of 10 chimeric mice derived from the RS element targeting murine ES cell line #32 prepared in Example 11 (chimeric rate: 80% to 20%), 6 chimeric mice derived from the CkP2 hEPO murine ES cell line #69 prepared in Example 16 (chimeric rate: 80% to 20%), 4 chimeric mice derived from the NP hEPO murine ES cell line #10 prepared in Example 20 (chimeric rate: 100%), 10 chimeric mice derived from the PS hEPO murine ES cell line #6 prepared in Example 24 (chimeric rate: 80% to 20%), 9 chimeric mice derived from the CkP2 ΔP hEPO murine TT2F cell prepared in Example 27 (chimeric rate: 60% to 10%), 20 chimeric mice derived from the CkP2 loxP hEPO murine ES cell line #18 prepared in Example 29 (chimeric rate: 90% to 10%), and 13 chimeric mice derived from the PS loxP hEPO murine ES cell line #48 prepared in Example 31 (chimeric rate: 90% to 10%), when they were 8 week old. The human EPO levels in the obtained blood serums were assayed using the ELISA kit (Quantikine IVD, *In vitro* diagnostic human erythropoietin, R&D system). As a result, the serum human EPO levels obtained from 10 chimeric mice derived from the RS element targeting murine ES cell line #32 prepared in Example 11 as the control (chimeric rate: 80% to 20%) were found to be at the lower detection limit (12.5 pg/ml) or lower. The average serum human EPO level of 6 chimeric mice derived from the CkP2 hEPO murine ES cell line #69 prepared in Example 16 (chimeric rate: 80% to 20%) was 5.3 ng/ml, that of 4 chimeric mice derived from the NP hEPO murine ES cell line #10 prepared in Example 20 (chimeric rate: 100%) was 14.7 ng/ml, that of 10 chimeric mice derived from the PS hEPO murine ES cell line #6 prepared in Example 24 (chimeric rate: 80% to 20%) was 47.9 ng/ml, that of 9 chimeric mice derived from the CkP2 ΔP hEPO murine TT2F cell prepared in Example 27 (chimeric rate: 60% to 10%) was 63.4 ng/ml, that of 20 chimeric mice derived from the CκP2 loxP hEPO murine ES cell line #18 prepared in Example 29 (chimeric rate: 90% to 10%) was 211 ng/ml, and that of 13 chimeric mice derived from the PS loxP hEPO murine ES cell line #48 prepared in Example 31 (chimeric rate: 90% to 10%) was 1,103 ng/ml. Fig. 17 and Table II show human EPO levels assayed at 1-, 2-, 4-, 6-, and 8-week old using the blood serums of chimeric mice prepared in Example above. These results indicate that serum human EPO levels increased in CkP2 hEPO murine ES cell-derived chimeric mouse, NP hEPO murine ES cell-derived chimeric mouse, PS hEPO murine ES cell-derived chimeric mouse, CkP2 ΔP hEPO murine TT2F cell-derived chimeric mouse, CkP2 loxP hEPO murine ES cell-derived chimeric mouse, and PS loxP hEPO murine ES cell-derived chimeric mouse, in that order.

These results indicate that the blood human EPO levels of chimeric mice increased in the order of a vector of pCκP2, pNP, and pPS types, and these results were found to be correlated with the results obtained in the *in vitro* experiment of Example 6. The correlation of these results with the results obtained in Example 6 suggests that the expression level of the gene introduced into the blood serum of a chimeric mouse prepared using the present expression system could be deduced by the *in vitro* assay system using myeloma cells Also, the serum human EPO levels were found to be increased by removing drug resistant genes (CkP2 loxP hEPO type and CkP2 ΔP hEPO type) from CkP2 hEPO murine ES (TT2F) cells. When a chimeric mouse was prepared using ES cells which were prepared by removing drug resistant genes from PS hEPO murine ES cells, the serum human EPO levels synergistically increased compared with the increased amount resulted from single operation. This result was very interesting. Specifically, the procedures of changing the Ig_{K} promoter region to be used and changing the leader sequence coding region inherent to the transfer gene into the intron-containing Igk-derived leader sequence coding region or removing a drug resistant gene in the vicinity of the transfer gene can be independently effective for improving the expression level of the transfer gene of a chimeric mouse. Further, through the performance of all of the procedures of changing the Igκ promoter region to be used, changing the leader sequence coding region inherent to the transfer gene into the intron-containing Igk-derived leader sequence coding region, and removing a drug resistant gene in the vicinity of the transfer gene, the expression level of the transfer gene is synergistically improved in a chimeric mouse, compared with the expression level improved via single procedure. This indicates that the method disclosed by the present invention is very effective for functional analysis of a gene and a product thereof *in vivo.*

### Example 34

### Analysis of peripheral blood cells of CkP2 hEPO murine ES cell-derived chimeric mouse, NP hEPO murine ES cell-derived chimeric mouse, PS hEPO murine ES cell-derived chimeric mouse, CkP2 ΔP hEPO murine TT2F cell-derived chimeric mouse, CkP2 loxP hEPO murine ES cell-derived chimeric mouse, and PS loxP hEPO murine ES cell-derived chimeric mouse

As controls, blood samples were obtained from the orbital sinus of 10 chimeric mice derived from the RS element targeting murine ES cell line #32 prepared in Example 11 (chimeric rate: 80% to 20%), 6 chimeric mice derived from the CkP2 hEPO murine ES cell line #69 prepared in Example 16 (chimeric rate: 80% to 20%), 4 chimeric mice derived from the NP hEPO murine ES cell line #10 prepared in Example 20 (chimeric rate: 100%), 10 chimeric mice derived from the PS hEPO murine ES cell line #6 prepared in Example 24 (chimeric rate: 80% to 20%), 9 chimeric mice derived from the CkP2 ΔP hEPO murine TT2F cell prepared in Example 27 (chimeric rate: 60% to 10%), 20 chimeric mice derived from the CkP2 loxP hEPO murine ES cell line #18 prepared in Example 29 (chimeric rate: 90% to 10%), and 13 chimeric mice derived from the PS loxP hEPO murine ES cell line #48 prepared in Example 31 (chimeric rate: 90% to 10%), when they were 8 week old. The peripheral blood cells were analyzed using a blood cell analyzer (ADVIA 120 hematology system, Bayer Medical, Japan).

Compared with the control mouse groups, the blood erythrocyte counts of all the chimeric mouse groups into which human EPO genes had been introduced were increased by 1.64 times or greater on average. However, there was no significant difference in blood erythrocyte counts among groups. The hematocrit value of the control groups was 58% on average; however, the hematocrit value of all the chimeric mouse groups into which human EPO genes had been introduced was at least 90% on average. Thus, the hematocrit value was significantly increased by the introduction of human EPO genes. As with the case of blood erythrocyte counts, there was no significant difference among groups. Even though significant difference was observed in the serum human EPO levels among groups of Example 33, blood cell analysis would not exhibit any significant difference. This is considered to result from the fact that the human EPO gene expression level of chimeric mice prepared from the CkP2 hEPO murine ES cell that exhibits the least expression level has already reached the upper limit of increase. This indicates that the method disclosed by the present invention is effective for functional analysis of a gene and a product thereof *in vivo.*

### Example 35

### Construction of pCk loxPV KI vector

The pCk loxPV KI vector (Fig. 18), which is a pCkP2 KI vector comprising a drug resistant gene (Puro^{r}) flanked by loxPV sequences, comprising mutations introduced into part of the LoxP sequence (T at position 15, and G at position 20, from the 5' end of a 34-bp loxP sequence have been substituted with A and C, respectively; Lee et al., Gene, 216: 55-65, 1998), was constructed.

### 35-1. Preparation of PGK-Puro fragment

Lox-P Puro plasmid (WO 00/10383) was digested with BamHI and separated by 0.8% agarose gel electrophoresis. A PGK-Puro fragment was recovered from a gel containing a 1.7-kb fragment by use of QIAquick Gel Extraction Kit (Qiagen, Germany) in accordance with the instructions.

### 35-2. Preparation of Ck polyA partial fragment

Based on the pCkP2 KI vector of Example 12, the following DNA (primers) were designed.
Ck pA NheI F: CCTAGCTAGCAGACAAAGGTCCTGAGACGCCAC (SEQ ID NO: 57)
Ck pA PacI R: CCTTAATTAATGGATTTCAGGGCAACTAAAC (SEQ ID NO: 58)

A reaction mixture was prepared by use of KOD-plus- (TOYOBO, Japan) in accordance with the instructions. To the reaction mixture (50 *µ*l), the two primers as prepared above (7.5 pmol each) and the TT2F murine ES genome as a template were added. After the reaction mixture was kept at 94°C for 2 minutes, a PCR cycle consisting of 94°C for 15 seconds, 58°C for 30 seconds, and 68°C for 30 seconds was repeated 32 times. About 300 bp amplified fragment was separated on 2% agarose gel. From the cut-out gel, the amplified fragment was recovered by use of QIAquick Gel Extraction Kit in accordance with the instructions. About 300-bp amplified fragment was separated on 2% gel. From the cut-out gel, the amplified fragment was recovered by use of QIAquick Gel Extraction Kit in accordance with the instructions. The PCR-amplified fragment thus recovered was digested with NheI/PacI and size-fractionated on 2% agarose gel. From the cut-out gel, the enzyme-digested fragment was recovered by use of QIAquick Gel Extraction Kit in accordance with the instructions. The resulting fragment was subcloned into the NheI/PacI site of pBS+ PFN of Example 1, and the resultant was introduced into *Escherichia coli* Competent high DH5 (Toyobo, Japan). DNA was prepared from the resulting transformant and subjected to sequencing. Compared with full-length Ck polyA of the CkP2 KI vector, p (partial-length Ck polyA), which did not exhibit amplification error, was digested with PacI/NheI. 303 bp fragment was separated on 2% gel, and the partial-length Ck polyA fragment was recovered by use of QIAquick Gel Extraction Kit in accordance with the instructions.

### 35-3. Vector prepared from pCkP2 KI vector with the addition of single-stranded AscI site··· pCkP2+As KI

The synthetic oligo DNAs shown below were annealed to synthesize AscI linkers.
AscI top linker: GGCCAGGCGCGCCTTGC (SEQ ID NO: 47)
AscI bottom linker: GGCCGCAAGGCGCGCCT (SEQ ID NO: 48)

The pCkP2 KI vector was digested with Notl (Roche), the digested fragment was separated and purified by 0.8% agarose gel electrophoresis, and AscI linkers were introduced via ligation using the ligation kit ver. 2 (Takara Bio, Japan). The resultant was introduced into *Escherichia coli* XL10-Gold Ultracompetent Cells (Stratagene, U.S.A.), DNA was prepared from the resulting transformant, and the nucleotide sequence of the inserted fragment was confirmed.

### 35-4. Preparation of Ck 3' genome fragment

pCkP2+As KI of 35-3 above was digested with Hpy99I, and 8,820 bp fragment was separated on 0.8% agarose gel. A gel fragment containing the target fragment was cleaved, and DNA was recovered by use of QIAquick Gel Extraction Kit in accordance with the instructions. The recovered DNA was blunt-ended using Blunting high (Toyobo, Japan) in accordance with the instructions. DNA was purified via phenol extraction and digested with XhoI. 8,280 bp fragment was separated on 0.8% agarose gel, a gel fragment containing the target fragment was cleaved, and the Ck 3' genome fragment was recovered by use of QIAquick Gel Extraction Kit in accordance with the instructions.

### 35-5. Preparation of loxPV NheI/XhoI fragment

pBS+PFN of Example 1 was digested with Sall, the ends thereof were dephosphorylated by alkaline phosphatase derived from the fetal calf intestine, and the loxP-BgIII-PmeI-HpaI linkers synthesized from the following oligo DNAs were inserted thereinto.

### Mutant loxP F:

TCGATAACTTCGTATAAAGTATCCTATACGAAGTTATAGATCTATAACTTCGTATA AAGTATCCTATACGAAGTTATGTTTAAACGTTAACG (SEQ ID NO: 59)
Mutant loxP R:
TCGACGTTAACGTTTAAACATAACTTCGTATAGGATACTTTATACGAAGTTATAG ATCTATAACTTCGTATAGGATACTTTATACGAAGTTA (SEQ ID NO: 60)

The product was introduced into *Escherichia coli* Competent high DH5α, and DNA was prepared from the resulting transformant. The nucleotide sequence of the ligated portion was confirmed, and pBS2272 into which the linkers had been inserted in the intended direction was obtained.

pBS2272 was digested with BglII, the ends thereof were dephosphorylated by alkaline phosphatase derived from the fetal calf intestine, and the PGK-Puro fragment prepared in 35-1 was ligated thereto. The ligation product was introduced into *Escherichia coli* Competent high DH5α and DNA was prepared from the resulting transformant. The nucleotide sequence of the ligated portion was confirmed, and the ploxPV-puro plasmid into which the PGK-Puro fragment had been cloned in the intended direction was obtained

This plasmid was digested with NheI/PacI, and the ends thereof were dephosphorylated by alkaline phosphatase derived from the fetal calf intestine. The partial-length Ck polyA fragment prepared in 35-2 above was inserted thereinto. The resultant was transformed into XL10-Gold Ultracompetent Cells (Stratagene, U.S.A.), plasmid DNA was prepared from the resulting clone, and the nucleotide sequence was confirmed. The sequence of the ligated portion and the sequence of the inside were confirmed to obtain the error-free ploxPV-Puro-polyA plasmid.

Subsequently, the resultant was digested with HpaI/XhoI, the ends thereof were dephosphorylated by alkaline phosphatase derived from the fetal calf intestine, and the Ck 3' genome fragment prepared in 35-4 above was introduced thereinto. The resultant was transformed into XL10-Gold Ultracompetent Cells, plasmid DNA was prepared from the resulting clone, and the nucleotide sequence was confirmed. The nucleotide sequence of the ligated portion was confirmed, and the ploxPV NheI/XhoI plasmid that had been ligated as expected was obtained. This plasmid was digested with NheI/XhoI, and a loxPV NheI/XhoI fragment of about 10.5 kb was recovered.

### 35-6. Construction of pCk loxPV KI vector

pCkP2+As KI of 35-3 was digested with NheI/XhoI, and a 9,968 bp fragment was recovered by fractionation on 0.8% agarose gel. The ends thereof were dephosphorylated by alkaline phosphatase derived from the fetal calf intestine, and the resultant was ligated to the loxPV NheI/XhoI fragment prepared in 35-5. The nucleotide sequence of the ligated portion was confirmed, and the pCk loxPV KI vector was obtained (Fig. 18).

### Example 36

### Preparation of pCk loxPV KI vector for electroporation

60 µg of pCk loxPV KI vector was digested with NotI at 37°C for 5 hours by using a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector which had been linearized with NotI was collected by centrifugation and sterilized by adding 70% ethanol. Then, 70% ethanol was removed in a clean ventilator and the linearized vector was air-dried for 1 hour. To the dried vector was added an HBS solution, thereby preparing a 0.5 µg/µl DNA solution, and the obtained DNA solution was stored at room temperature for 1 hour. In this manner, the pCk loxPV KI vector for electroporation was prepared.

### Example 37

### Obtaining Ck loxPV murine ES cell line using pCk loxPV KI vector and RS element targeting murine ES cell line

To obtain a Ck loxPV murine ES cell line comprising the immunoglobulin κ light chain gene and, in a region downstream thereof, a drug resistant gene flanked by mutant loxP sequences (loxPV Puro^{r}) inserted via homologous recombination, the pCk loxPV KI vector prepared in Example 35 was linearized with Notl (Takara Shuzo, Japan), and the resulting vector was introduced into the RS element targeting murine ES cell in accordance with the established method (Shinichi Aizawa, Bio-Manual Series 8, Gene Targeting, 1995, Yodosha, Japan).

The RS element targeting murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The RS element targeting murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U.S.A.). After 7 days, colonies were generated. Of them, 24 colonies were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (Xhol to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect homologous recombinants. As a result, 6 homologous recombinants were obtained out of 24 clones (25%). In the wild-type RS element targeting murine ES cell, a single band was detected by EcoRI digestion. In the homologous recombinants, a new band was expected to appear below this band (Fig. 19). Actually, the new band was detected in the puromycin resistant cell line In short, these clones had the drug resistant gene (loxPV Puro^{r}) inserted downstream of the immunoglobulin κ-chain gene of one of the alleles.

### Example 38

### Obtaining ΔGP murine ES cell line by removing drug resistant gene from Ck loxPV murine ES cell line

To obtain a ΔGP murine ES cell line from the Ck loxPV murine ES cell line by removing 2 types of drug resistant genes (Neo^{r} and Puro^{r}), the pCAGGS-Cre vector (Sunaga et al., Mol Reprod Dev., 46: 109-113, 1997) was introduced into the Ck loxPV murine ES cell (#16) in accordance with the established method (Shinichi Aizawa, Bio-Manual Series 8, Gene Targeting, 1995, Yodosha, Japan).

The Ck loxPV murine ES cells were cultured in accordance with the method of Shinichi Aizawa (supra) using, as a trophocyte, the G418 resistant primary cultured cell (Invitrogen, Japan) which had been treated with mitomycin C (Sigma, U.S.A.). The Ck loxPV murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of ES medium (Shinichi Aizawa, supra), and 2.5 ml. thereof was seeded on a 60 mm plastic tissue-culture Petri dish (Falcon; Becton Dickinson, U.S.A.) having feeder cells previously seeded therein. After 30 hours, 1,000 ES cells were seeded on a 100 mm plastic tissue-culture Petri dish (Falcon; Becton Dickinson, U.S.A.) having feeder cells previously seeded therein. After 6 days, colonies were generated. Of them, 24 colonies were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect ES cell lines from which the Puro^{r} gene flanked by LoxP sequences had been selectively removed. In the case of the Puro^{r} gene-carrying ES cells, two bands (15.6 K and 12.5 K) were detected upon digestion with EcoRI. In the case of the ES cells from which the Puro^{r} gene had been selectively removed, two bands (15.6 K and 9.6 K) were detected upon digestion with EcoRI (Fig. 19). With the use of a Southern blot membrane obtained in the same manner as described above, an ES cell line from which the Neo^{r} gene flanked by the LoxP sequences had been selectively removed was detected using the 3' KO-prob used in Example 10 as a probe. In the case of the Neo^{r} gene-carrying ES cells, two bands (7.4 K and 5.7 K) were detected upon digestion with EcoRI. In the case of the ES cells from which the Neo^{r} gene had been selectively removed, two bands (5.7 K and 4.4 K) were detected upon digestion with EcoRI (Fig. 19). As a result, the ES cell line (ΔGP murine ES cell line) was obtained from the Ck loxPV murine ES cell line from which 2 types of drug resistant genes (Neo^{r} and Puro^{r}) had been simultaneously removed at a high efficiency of 23 cells out of 24 cells (96%) (Fig. 19). In Examples 28 and 30 above, simultaneous removal of two types of drug resistant genes was performed with the aid of Cre enzyme. However, the efficiency of obtaining cell lines from which two types of drug resistant genes had been simultaneously removed was as low as 2.2% or 1%. From these results, simultaneous and specific removal of two types of drug resistant genes flanked by the identical loxP sequences was found to be difficult; however, such removal was found to be realized with the use of loxP (loxPV) having mutant sequences.

### Example 39

### Construction of pCk loxPV hEPO KI vector

### 39-1. Construction of CkP2 ver. 3.1 vector

In order to remove the PacI recognition site of pCk loxPV KI that had been constructed in Example 35, the fragment was digested with PacI and then blunt-ended using Blunting high. Subsequently, self-circularization was carried out and the removal of the PacI recognition site was confirmed via sequencing. Thus, the CkP2 ver. 3.1 vector was obtained.

### 39-2. Preparation of human erythropoietin DNA fragment

hEPO Np: CCGCTCGAGCGGCCACCATGGGGGTGCACGAATGTCCTG (SEQ ID NO: 45)
hEPO Rp: CCGCTCGAGCGGTCATCTGTCCCCTGTCCTGCA (SEQ ID NO: 46)

A reaction mixture was prepared by use of KOD-plus- (TOYOBO, Japan) in accordance with the instructions. To the reaction mixture (50 µl), the two primers as prepared above (10 pmol each) and human EPO cDNA as a template were added. After the reaction mixture was kept at 94°C for 2 minutes, a PCR cycle consisting of 94°C for 15 seconds and 68°C for 1 minute was repeated 30 times. The resulting signal sequence-containing 580 bp amplified fragment was separated on 0.8% agarose gel. From the cut-out gel, the amplified fragment was recovered by use of QIAquick Gel Extraction Kit (Qiagen, Germany) in accordance with the instructions. The PCR-amplified fragment thus recovered was digested with XhoI and separated on 0.8% agarose gel. From the cut-out gel, the enzyme-digested fragment was recovered by use of QIAquick Gel Extraction Kit (Qiagen, Germany) in accordance with the instructions.

pBluescriptII SK(-) (Stratagene, U.S.A.) was digested with Xhol and separated and purified by 0.8% agarose gel electrophoresis. The ends thereof were dephosphorylated by alkaline phosphatase derived from the fetal calf intestine. The DNA fragment recovered above was inserted thereinto, and the resultant was introduced into *Escherichia coli* DH5α. DNA was prepared from the resulting transformant, and the inserted fragment was sequenced. Clones having no mutation due to PCR were selected, digested with XhoI, and then separated on 0.8% agarose gel. From the cut-out gel, the enzyme-digested fragment was recovered by use of QIAquick Gel Extraction Kit (Qiagen, Germany) in accordance with the instructions.

### 39-3. Construction of pCkloxPV hEPO KI vector

The CkP2 ver. 3.1 vector of 39-1 was digested with SalI, and the ends thereof were dephosphorylated by alkaline phosphatase derived from the fetal calf intestine. The DNA fragment prepared in 39-2 was introduced thereinto, and the resultant was then introduced into *Escherichia coli* XL10-Gold Ultracompetent Cells (Stratagene, U.S.A.). DNA was prepared from the resulting transformant, the nucleotide sequence of the ligated portion was confirmed, and the pCk loxPV hEPO KI vector was obtained (Fig. 20).

### Example 40

### Preparation of pCk loxPV hEPO KI vector for electroporation

60 µg of pCk loxPV hEPO KI vector was digested with NotI at 37°C for 5 hours by using a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector which had been linearized with NotI was collected by centrifugation and sterilized by adding 70% ethanol. Then, 70% ethanol was removed in a clean ventilator and the linearized vector was air-dried for 1 hour. To the dried vector was added an HBS solution, thereby preparing a 0.5 µg/µl DNA solution, and the obtained DNA solution was stored at room temperature for 1 hour. In this manner, the pCk loxPV hEPO KI vector for electroporation was prepared.

### Example 41

### Obtaining CL hEPO murine ES cell line using pCk loxPV hEPO KI vector and RS element targeting murine ES cell line

To obtain a CL hEPO murine ES cell line comprising the immunoglobulin κ light chain gene and, in a region downstream thereof, human EPO-cDNA inserted via homologous recombination, the pCk loxPV hEPO KI vector prepared in Example 39 was linearized with NotI (Takara Shuzo, Japan), and the resulting vector was introduced into the RS element targeting murine ES cell in accordance with the established method (Shinichi Aizawa, Bio-Manual Series 8, Gene Targeting, 1995, Yodosha, Japan).

The RS element targeting murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The RS element targeting murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U.S.A.). Colonies generated 7 days thereafter were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect homologous recombinants. In the wild-type RS element targeting murine ES cell, a single band was detected by EcoRI digestion. In the homologous recombinants, a new band was expected to appear below this band (Fig. 21). Actually, the new band was detected in the puromycin resistant cell line. In short, these clones had human EPO-cDNA inserted downstream of the immunoglobulin κ-chain gene of one of the alleles.

### Example 42

### Preparation of chimeric mouse by using CL hEPO murine ES cell line and B-lymphocyte deficient murine host embryo

A homozygote in which the immunoglobulin µ chain gene was knocked out is devoid of functional B lymphocytes and thus no antibodies are produced ( Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times. From the resulting mouse individuals, a host embryo was prepared.

The CL hEPO murine ES cell line (obtained in Example 41), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of the CL hEPO murine ES cell line (Example 41). When the prepared injection embryos were transplanted, chimeric mice were born. Chimeric mouse individuals were identified by evaluating whether the wild coat color (i.e., dark brown) derived from the ES cell was observed in white coat color derived from the host embryo. From these results, it was demonstrated that CL hEPO murine ES cell line comprising human EPO-cDNA inserted downstream of the immunoglobulin κ chain gene had a chimera formation potency, or a potency differentiating into normal murine tissue.

### Example 43

### Construction of pNP loxPV hEPO KI vector

### 43-1. Construction of pCk loxPVΔP (mutant loxP large vector)

The CkP2 ver. 3.1 vector prepared in Example 39-1 was digested with HpaI/NheI, and about 19.5 kb fragment was recovered by fractionation on 0.8% agarose gel. Ends thereof were dephosphorylated by alkaline phosphatase derived from the fetal calf intestine. pCkpAMCS of Example 17-5 was digested with HpaI and NheI (Roche) and about 700 bp fragment containing Ck-polyA-MCS was recovered from 0.8% agarose gel. The resultant was introduced into the above vector. The nucleotide sequence of the ligated portion and the nucleotide sequence of the inside were confirmed to obtain pCk loxPVΔP.

### 43-2. Construction of pNP loxPV hEPO KI vector

The pNPs hEPO *in vitro* vector prepared in Example 2 was digested with SalI and FseI, and about 1.2 kb fragment was separated and purified by 0.8% agarose gel electrophoresis. pCk loxPVΔP of 43-1 was digested with SalI and FseI, and the ends thereof were dephosphorylated by alkaline phosphatase derived from *Escherichia coli* C75. Into the resultant was introduced the fragment of about 1.2 kb above. The resultant was introduced into *Escherichia coli* XL10-Gold Ultracompetent Cells s (Stratagene, U.S.A.). DNA was prepared from the resulting transformant, the nucleotide sequence of the ligated portion was confirmed, and the pNP loxPV hEPO KI vector was obtained (Fig. 22).

### Example 44

### Preparation of pNP loxPV hEPO KI vector for electroporation

60 µg of pNP loxPV hEPO KI vector was digested with NotI at 37°C for 5 hours by using a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector which had been linearized with NotI was-collected by centrifugation and sterilized by adding 70% ethanol. Then, 70% ethanol was removed in a clean ventilator and the linearized vector was air-dried for 1 hour. To the dried vector was added an HBS solution, thereby preparing a 0.5 µg/µl DNA solution, and the obtained DNA solution was stored at room temperature for 1 hour. In this manner, the pNP loxPV hEPO KI vector for electroporation was prepared.

### Example 45

### Obtaining NL hEPO murine ES cell line using pNP loxPV hEPO KI vector and RS element targeting murine ES cell line

To obtain a NL hEPO murine ES cell line comprising the immunoglobulin κ light chain gene and, in a region downstream thereof, human EPO-cDNA inserted via homologous recombination, the pNP loxPV hEPO KI vector prepared in Example 43 was linearized with NotI (Takara Shuzo, Japan), and the resulting vector was introduced into the RS element targeting murine ES cell in accordance with the established method (Shinichi Aizawa, Bio-Manual Series 8, Gene Targeting, 1995, Yodosha, Japan).

The RS element targeting murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The RS element targeting murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U.S.A.). Colonies generated 7 days thereafter were picked up and grown up to the confluent state in 24-well plates. Two thirds of the grown cells were suspended in 0.2 ml of a stock medium (FBS+ 10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect homologous recombinants. In the wild-type RS element targeting murine ES cell, a single band was detected by EcoRI digestion. In the homologous recombinants, a new band was expected to appear below this band (Fig. 23). Actually, the new band was detected in the puromycin resistant cell line. In short, these clones had human EPO-cDNA inserted downstream of the immunoglobulin κ-chain gene of one of the alleles.

### Example 46

### Preparation of chimeric mouse by using NL hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times.

The NL hEPO murine ES cell line (obtained in Example 45), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse Embryos to be injected (or injection embryos) were prepared by use of the NL hEPO murine ES cell line (Example 45). When the injection embryos prepared were transplanted, chimeric mice were born. Chimeric mouse individuals were identified by evaluating whether the wild coat color (i. e., dark brown) derived from the ES cell was observed in white coat color derived from the host embryo. From these results, it was demonstrated that NL hEPO murine ES cell line comprising human EPO-cDNA inserted downstream of the immunoglobulin κ chain gene had a chimera formation potency, or a potency differentiating into normal murine tissue.

### Example 47

### Construction of pUS hEPO KI vector

The pPSs hEPO *in vitro* vector of Example 1 was digested with Sall and FseI. A DNA fragment of about 1.3 kb was separated and purified by 0.8% agarose gel electrophoresis. pCk loxPVΔP prepared in Example 43 was digested with SalI and FseI, and the ends thereof were dephosphorylated by alkaline phosphatase derived from *Escherichia coli* C75. Into the resultant was inserted the fragment of about 1.3 kb above. The resultant was introduced into *Escherichia coli* XL10-Gold Ultracompetent Cells (Stratagene, U.S.A.). DNA was prepared from the resulting transformant and the nucleotide sequence of the ligated portion was confirmed. In this manner, the pUS hEPO KI vector was obtained (Fig. 24).

### Example 48

### Preparation of pUS hEPO KI vector for electroporation

60 µg of pUS hEPO KI vector was digested with NotI at 37°C for 5 hours by using a buffer (H buffer for restriction enzyme; Roche Diagnostics, Japan) supplemented with spermidine (1 mM, pH 7.0; Sigma, U.S.A.). After extraction with phenol/chloroform, 2.5 volumes of 100% ethanol and 0.1 volumes of 3M sodium acetate were added to the resulting mixture and stored at -20°C for 16 hours. The vector which had been linearized with NotI was collected by centrifugation and sterilized by adding 70% ethanol. Then, 70% ethanol was removed in a clean ventilator and the linearized vector was air-dried for 1 hour. To the dried vector was added an HBS solution, thereby preparing a 0.5 µg/pl DNA solution, and the obtained DNA solution was stored at room temperature for 1 hour. In this manner, the pUS hEPO KI vector for electroporation was prepared.

### Example 49

### Obtaining PL hEPO murine ES cell line using pUS hEPO KI vector and RS element targeting murine ES cell line

To obtain a PL hEPO murine ES cell line comprising the immunoglobulin κ light chain gene and, in a region downstream thereof, human EPO-cDNA inserted via homologous recombination, the pUS hEPO KI vector prepared in Example 47 was linearized with NotI (Takara Shuzo, Japan), and the resulting vector was introduced into the RS element targeting murine ES cell in accordance with the established method (Shinichi Aizawa, Bio-Manual Series 8, Gene Targeting, 1995, Yodosha, Japan).

The RS element targeting murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The RS element targeting murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra) and seeded on a 100 mm plastic tissue-culture Petri dish (Falcon; Becton Dickinson, U.S.A.) having feeder cells previously seeded therein. After 36 hours, the medium was replaced with fresh ES medium containing 0.8 µg/ml puromycin (Sigma, U.S.A.). Colonies generated 7 days thereafter were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, Fig. 25), which was at the 3' end of the Ig light chain Jκ-Cκ genomic DNA and had been used in the invention described in WO 00/10383 (see Example 48), to detect homologous recombinants. In the wild-type RS element targeting murine ES cell, a single band was detected by EcoRI digestion. In the homologous recombinants, a new band was expected to appear below this band (Fig. 25). Actually, the new band was detected in the puromycin resistant cell line. In short, these clones had human EPO-cDNA inserted downstream of the immunoglobulin κ-chain gene of one of the alleles.

### Example 50

### Preparation of chimeric mouse by using PL hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times.

The PL hEPO murine ES cell line (obtained in Example 49), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of the PL hEPO murine ES cell line (Example 49). When the prepared injection embryos were transplanted, chimeric mice were born. Chimeric mouse individuals were identified by evaluating whether the wild coat color (i.e., dark brown) derived from the ES cell was observed in white coat color derived from the host embryo. From these results, it was demonstrated that PL hEPO murine ES cell line comprising human EPO-cDNA inserted downstream of the immunogloblin κ chain gene had a chimera formation potency, or a potency differentiating into normal murine tissue.

### Example 51

### Obtaining Ck loxPV hEPO murine ES cell line from CL hEPO murine ES cell line by removing drug resistant gene

To obtain an ES cell line into which the Ck lo×PV hEPO gene has been introduced from the CL hEPO murine ES cell line by removing 2 types of drug resistant genes (Neo^{r} and Puro^{r}), the pCAGGS-Cre vector (Sunaga et al., Mol Reprod Dev., 46: 109-113, 1997) was introduced into the CL hEPO murine ES cell in accordance with the established method (Shinichi Aizawa, Bio-Manual Series 8, Gene Targeting, 1995, Yodosha, Japan).

The CL hEPO murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The CL hEPO murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra), and 2.5 ml thereof was seeded on a 60 mm plastic tissue-culture Petri dish (Falcon; Becton Dickinson, U.S.A.) having feeder cells previously seeded therein. After 30 hours, 1,000 ES cells were seeded on a 100 mm plastic tissue-culture Petri dish (Falcon; Becton Dickinson, U.S.A.) having feeder cells previously seeded therein. Colonies generated 6 days thereafter were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the puromycin-resistant RS element targeting murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect an ES cell line from which the Puro^{r} gene flanked by LoxP sequences had been selectively removed In the case of the Puro^{r} gene-carrying ES cells, two bands (15.6 K and 13.1 K) were detected upon digestion with EcoRI. In the case of the ES cells from which the Puro^{r} gene had been selectively removed, two bands (15.6 K and 10.2 K) were detected upon digestion with EcoRI (Fig. 21). With the use of a Southern blot membrane obtained in the same manner as described above, an ES cell line from which the Neo^{r} gene flanked by the LoxP sequences had been selectively removed was detected using the 3' KO-prob used in Example 10 as a probe. In the case of the Neo^{r} gene-carrying ES cells, two bands (7.4 K and 5.7 K) were detected upon digestion with EcoRI. In the case of the ES cells from which the Neo^{r} gene had been selectively removed, two bands (5.7 K and 4.4 K) were detected upon digestion with EcoRI (Fig. 21). As a result, the ES cell line (Ck loxPV hEPO murine ES cell line) was found to be obtained from the CL hEPO murine ES cell line from which 2 types of drug resistant genes (Neo^{r} and Puro^{r}) had been simultaneously removed.

### Example 52

### Preparation of chimeric mouse by using Ck loxPV hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times.

The Ck loxPV hEPO murine ES cell line (obtained in Example 51), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of the Ck loxPV hEPO murine ES cell line (Example 51). When the prepared injection embryos were transplanted, chimeric mice were born. Chimeric mouse individuals were identified by evaluating whether the wild coat color (i.e., dark brown) derived from the ES cell was observed in white coat color derived from the host embryo. Among the obtained ofsprings, there were mouse individuals which clearly had the wild color partially in the coat color, i.e. having the contribution from the ES cell. From these results, it was demonstrated that Ck loxPV hEPO murine ES cell line comprising human EPO-cDNA inserted downstream of the immunogloblin κ chain gene had a chimera formation potency, or a potency differentiating into normal murine tissue.

### Example 53

### Obtaining NP loxPV hEPO murine ES cell line from NL hEPO murine ES cell line by removing drug resistant gene

To obtain an ES cell line into which the NP loxPV hEPO gene has been introduced from the NL hEPO murine ES cell line by removing 2 types of drug resistant genes (Neo^{r} and Puro^{r}), the pCAGGS-Cre vector (Sunaga et al., Mol Reprod Dev., 46: 109-113, 1997) was introduced into the CL hEPO murine ES cell in accordance with the established method (Shinichi Aizawa, Bio-Manual Series 8, Gene Targeting, 1995, Yodosha, Japan).

The NL hEPO murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The NL hEPO murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra), and 2.5 ml thereof was seeded on a 60 mm plastic tissue-culture Petri dish (Falcon; Becton Dickinson, U.S.A.) having feeder cells previously seeded therein. After 30 hours, 1,000 ES cells were seeded on a 100 mm plastic tissue-culture Petri dish (Falcon; Becton Dickinson, U.S.A.) having feeder cells previously seeded therein. Colonies generated 6 days thereafter were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect an ES cell line from which the Puro^{r} gene flanked by LoxP sequences had been selectively removed. In the case of the Puro^{r} gene-carrying ES cells, two bands (15.6 K and 12.9 K) were detected upon digestion with EcoRI. In the case of the ES cells from which the Puro^{r} gene had been selectively removed, two bands (15.6 K and 10.0 K) were detected upon digestion with EcoRI (Fig. 23). With the use of a Southern blot membrane obtained in the same manner as described above, an ES cell line from which the Neo^{r} gene flanked by the LoxP sequences had been selectively removed was detected using the 3' KO-prob used in Example 10 as a probe. In the case of the Neo^{r} gene-carrying ES cells, two bands (7.4 K and 5.7 K) were detected upon digestion with EcoRI. In the case of the ES cells from which the Neo^{r} gene had been selectively removed, two bands (5.7 K and 4.4 K) were detected upon digestion with EcoRI (Fig. 23). As a result, the ES cell line (NP loxPV hEPO murine ES cell line) was found to be obtained from the CL hEPO murine ES cell line from which 2 types of drug resistant genes (Neo^{r} and Puro^{r}) had been simultaneously removed.

### Example 54

### Preparation of chimeric mouse by using NP loxPV hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times.

The NP loxPV hEPO murine ES cell line (obtained in Example 53), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of the NP loxPV hEPO murine ES cell line (Example 53). When the prepared injection embryos were transplanted, chimeric mice were born. Chimeric mouse individuals were identified by evaluating whether the wild coat color (i.e., dark brown) derived from the ES cell was observed in white coat color derived from the host embryo. Among the obtained ofsprings, there were mouse individuals which clearly had the wild color partially in the coat color, i.e. having the contribution from the ES cell. From these results, it was demonstrated that NP loxPV hEPO murine ES cell line comprising human EPO-cDNA inserted downstream of the immunogloblin κ chain gene had a chimera formation potency, or a potency differentiating into normal murine tissue.

### Example 55

### Obtaining US hEPO murine ES cell line from PL hEPO murine ES cell line by removing drug resistant gene

To obtain an ES cell line into which the US hEPO gene has been introduced from the PL hEPO murine ES cell line by removing 2 types of drug resistant genes (Neo^{r} and Puro^{r}), the pCAGGS-Cre vector (Sunaga et al., Mol Reprod Dev., 46: 109-113, 1997) was introduced into the CL hEPO murine ES cell in accordance with the established method (Shinichi Aizawa, Bio-Manual Series 8, Gene Targeting, 1995, Yodosha, Japan).

The PL hEPO murine ES cells were cultured in accordance with the method (Shinichi Aizawa, supra) using, as a trophocyte, the G418 resistant primary cultured cell (Invitrogen, Japan) treated with mitomycin C (Sigma, U.S.A.). The PL hEPO murine ES cells grown were treated with trypsin and suspended in HBS at 3 × 10⁷ cells/ml. Thereafter, 0.5 ml of the cell suspension was mixed with 10 µg of vector DNA, placed in a gene pulsar cuvette (distance between electrodes: 0.4 cm; Biorad, U.S.A.), and subjected to electroporation (capacity: 960 µF, voltage: 250 V, room temperature). After electroporation, the cells were suspended in 10 ml of the ES medium (Shinichi Aizawa, supra), and 2.5 ml thereof was seeded on a 60 mm plastic tissue-culture Petri dish (Falcon; Becton Dickinson, U. S.A.) having feeder cells previously seeded therein. After 30 hours, 1,000 ES cells were seeded on a 100 mm plastic tissue-culture Petri dish (Falcon; Becton Dickinson, U.S.A.) having feeder cells previously seeded therein. Colonies generated 6 days thereafter were picked up and grown up to the confluent state in 24-well plates. Two thirds of the grown cells were suspended in 0.2 ml of a stock medium (FBS+10% DMSO; Sigma, U.S.A.) and stored at -80°C. The remaining one thirds was seeded on a 12-well gelatin coated plate and cultured for 2 days. From 10⁶ to 10⁷ cells, genomic DNA was prepared by use of Puregene DNA Isolation Kits (Gentra System, U.S.A.). The genomic DNA from the murine ES cells was digested with EcoRI (Takara Shuzo, Japan) and separated by agarose gel electrophoresis. Subsequently, Southern blot was performed by use of, as a probe, a DNA fragment (XhoI to EcoRI, about 1.4 kb, WO 00/10383, particularly Fig. 25), which was at the 3' end of the Ig light chain Jκ-Cκ genomic DNA and had been used in the invention described in WO 00/10383 (see Example 48), to detect an ES cell line from which the Puro^{r} gene flanked by LoxP sequences had been selectively removed. In the case of the Puro^{r} gene-carrying ES cells, two bands (15.6 K and 12.7 K) were detected upon digestion with EcoRI. In the case of the ES cells from which the Puro^{r} gene had been selectively removed, two bands (15.6 K and 9.8 K) were detected upon digestion with EcoRI (Fig. 25). With the use of a Southern blot membrane obtained in the same manner as described above, an ES cell line from which the Neo^{r} gene flanked by the LoxP sequences had been selectively removed was detected using the 3' KO-prob used in Example 10 as a probe. In the case of the Neo^{r} gene-carrying ES cells, two bands (7.4 K and 5.7 K) were detected upon digestion with EcoRI. In the case of the ES cells from which the Neo^{r} gene had been selectively removed, two bands (5.7 K and 4.4 K) were detected upon digestion with EcoRI (Fig. 25). As a result, the ES cell line (US hEPO murine ES cell line) was found to be obtained from the PL hEPO murine ES cell line from which 2 types of drug resistant genes (Neo^{r} and Puro^{r}) had been simultaneously removed.

### Example 56

### Preparation of chimeric mouse by using US hEPO murine ES cell line and B-lymphocyte deficient murine host embryo

A homozygote in which the immunoglobulin µ chain gene was knocked out is devoid of functional B lymphocytes and thus no antibodies are produced (Kitamura et al., Nature, 350: 423-426, 1991). A male and female of such a homozygote were raised in clean environment and crossed to obtain an embryo. This embryo was used as a host in this Example for producing a chimeric mouse. In this case, most of the functional B lymphocytes of the chimeric mouse were derived from the ES cell externally injected. In this Example, a mouse from which the immunoglobulin µ chain gene was knocked out and described in the report of Tomizuka et al. (Proc. Natl. Acad. Sci. U.S.A., 97: 722-7, 2000) was back-crossed with MCH (ICR) (CLEA Japan, Japan) three or more times.

The US hEPO murine ES cell line (obtained in Example 55), which was confirmed that human EPO-cDNA had been inserted downstream of the immunoglobulin κ chain gene, was thawed from frozen stocks. The ES cells were injected at a rate of 8-10 cells/embryo into the 8-cell embryo which was obtained by crossing the male and female homozygous mice in which the immunoglobulin µ chain gene was knocked out. The embryo was cultured in the ES medium (Shinichi Aizawa, Bio-Manual Series 8, Gene Targeting, 1995, Yodosha, Japan) overnight to develop into the blastocyst. About 10 embryos were transplanted in each one of the two uteri of a surrogate MCH (ICR) mouse (CLEA Japan, Japan) 2.5 days after pseudopregnancy treatment was applied to the mouse. Embryos to be injected (or injection embryos) were prepared by use of the US hEPO murine ES cell line (Example 55). When the prepared injection embryos were transplanted, chimeric mice were born. Chimeric mouse individuals were identified by evaluating whether the wild coat color (i.e., dark brown) derived from the ES cell was observed in white coat color derived from the host embryo. Among the obtained ofsprings, there were mouse individuals which clearly had the wild color partially in the coat color, i.e. having the contribution from the ES cell. From these results, it was demonstrated that US hEPO murine ES cell line comprising human EPO-cDNA inserted downstream of the immunogloblin κ chain gene had a chimera formation potency, or a potency differentiating into normal murine tissue.

### Example 57

### Comparison of human EPO gene transcription level of CL hEPO murine ES cell-derived chimeric mouse, NL hEPO murine ES cell-derived chimeric mouse, and PL hEPO murine ES cell-derived chimeric mouse

### (1) Preparation of mRNA sample from spleen of chimeric mouse

As controls, spleen samples were extracted from 1-week-old, 2-week-old, and 4-week-old mice each from chimeric mice derived from the RS element targeting murine ES cell line prepared in Example 11, chimeric mice derived from the CL hEPO murine ES cell line prepared in Example 42, chimeric mice derived from the NL hEPO murine ES cell line prepared in Example 46, and chimeric mice derived from the PL hEPO murine ES cell line prepared in Example 50. The spleens (about 50 mg) were subjected to freezing in liquid nitrogen immediately thereafter. Isogen (1 ml, Nippon Gene, Japan) was added to the frozen samples, the samples were broken using a homogenizer, and RNA was extracted in accordance with the instructions. The resulting RNA samples were subjected to DNase treatment at 37°C for 15 minutes (deoxyribonuclease; RT-grade, Wako Pure Chemical Industries Ltd., Japan). Further, purified RNA samples were obtained using RNasy Mini (Qiagen, Germany).

### Comparison of human EPO gene transcription levels:

cDNA was synthesized from 250 ng of resulting purified RNA samples using SuperScript III (Invitrogen). Thereafter, the resultant was subjected to RNase treatment at 37°C for 20 minutes, and 2.0 µl of 1:10 diluent with sterilized water was used in the subsequent PCR procedure.

As primers for confirming human EPO gene expression, the following oligo DNAs were synthesized.
hEPO-RT FW5: GGCCAGGCCCTGTTGGTCAACTCTTC (SEQ ID NO: 61)
CkpolyAR2: CGCTTGTGGGGAAGCCTCCAAGACC (SEQ ID NO: 62)

PCR was carried out under the following conditions in order to confirm expression of the human EPO gene. A two-step cycle consisting of incubation at 94°C for 10 seconds and incubation at 68°C for 1 minute was repeated 35 times using LA taq (Takara Shuzo, Japan). As a result, no amplified band was detected in 1-week-old, 2-week-old, and 4-week-old mice of the control group; however, amplified band was detected in spleen tissue of all 1-week-old or older chimeric mice that had been prepared with the use of murine ES cells into which human EPO genes had been introduced. The density of the amplified bands each representing the 2-week-old and 4-week-old NL hEPO murine ES cell-derived chimeric mice was substantially the same as that each representing the PL hEPO murine ES cell-derived chimeric mice, and the density of the amplified band representing the CL hEPO murine ES cell-derived chimeric mouse was somewhat lower than the above density.

In order to confirm that the amounts of mRNA used are even among sample groups, the following oligo DNAs were synthesized as primers for confirming the murine glyceraldehyde-3-phosphate dehydrogenase (GAPDH) gene expression.
mGAPDH5: CACCATGGAGAAGGCCGGGGCCCAC (SEQ ID NO: 63)
mGAPDH3: ATCATACTTGGCAGGTTTCTCCAGG (SEQ ID NO: 64)

PCR was carried out under the following conditions in order to confirm expression of the murine GAPDH gene. A three-step PCR cycle of incubation at 94°C for 30 seconds, 65°C for 30 seconds, and 72°C for 30 seconds was repeated 25 times using LA taq (Takara Shuzo, Japan). As a result, murine GAPDH expression levels were found to be substantially the same among all samples. Also, differences in band density among samples that had been detected using primers for confirming human EPO gene expression were found to indicate differences in human EPO gene expression levels in spleen tissue of chimeric mice. These results indicate that a vector of pNP loxPV or pPS loxPV type is more effective at the transcription level compared with a vector of pCk loxPV type. These results were found to be correlated with the results obtained by the *in vitro* and *in vivo* experiments of Example 5 and of Example 32. These results also indicate that partial modification of the loxP sequences added to the both ends in order to selectively remove a drug resistant gene (Puro^{r}) from the ES cell genome would not influence the expression of the transfer gene.

### Example 58

### Comparison of serum human EPO level of CL hEPO murine ES cell-derived chimeric mouse, NL hEPO murine ES cell-derived chimeric mouse, PL hEPO murine ES cell-derived chimeric mouse, Ck loxPV hEPO murine ES cell-derived chimeric mouse, NP loxPV hEPO murine ES cell-derived chimeric mouse, and US hEPO murine ES cell-derived chimeric mouse

As controls, blood samples were obtained from the orbital sinus of chimeric mice derived from the RS element targeting murine ES cell line prepared in Example 11, chimeric mice derived from the CL hEPO murine ES cell line prepared in Example 42, chimeric mice derived from the NL hEPO murine ES cell line prepared in Example 46, chimeric mice derived from the PL hEPO murine ES cell line prepared in Example 50, chimeric mice derived from the Ck loxPV hEPO murine ES cell line prepared in Example 52, chimeric mice derived from the NP loxPV hEPO murine ES cell line prepared in Example 54, and chimeric mice derived from the US hEPO murine ES cell line prepared in Example 56, when they were 8 week old. The serum human EPO levels were assayed using the ELISA kit (Quantikine IVD, *In vitro* diagnostic human erythropoietin, R&D system). As a result, the serum human EPO levels obtained from chimeric mice derived from the RS element targeting murine ES cell line prepared in Example 11 as the control were found to be at the lower detection limit (12.5 pg/ml) or lower; however, the expression levels were significant in all chimeric mice into which human EPO genes had been introduced. Also, increase in serum human EPO levels is observed in CL hEPO murine ES cell-derived chimeric mouse, NL hEPO murine ES cell-derived chimeric mouse, PL hEPO murine ES cell-derived chimeric mouse, Ck loxPV hEPO murine ES cell-derived chimeric mouse, NP loxPV hEPO murine ES cell-derived chimeric mouse, and US hEPO murine ES cell-derived chimeric mouse, in that order.

These results indicate that the blood human EPO levels of chimeric mice increase in the order of a vector of pCk loxPV, pNP loxPV, and pUS types, and these results were found to be correlated with the results obtained in the *in vitro* experiment of Example 6 or the results obtained in the *in vivo* experiment of Example 33, regardless of partial modification of the loxP sequences that had been added to both ends so as to selectively remove drug resistant genes (Puro^{r}). The correlation of these results with the results obtained in Example 6 suggests that the expression level of the gene introduced into the blood serum of a chimeric mouse prepared using the present expression system could be deduced by the *in vitro* assay system using myeloma cells. Also, the serum human EPO levels were found to be increased by removing drug resistant genes (Ck loxPV hEPO murine ES cells) from CL hEPO murine ES cells. When a chimeric mouse was prepared using ES cells from NL hEPO or PL hEPO murine ES cells by removing drug resistant genes therefrom, the serum human EPO levels synergistically increased compared with the amount of increase resulting from single operation. This result was very interesting. Specifically, the procedures of changing the Igκ promoter region to be used and changing the leader sequence coding region inherent to the transfer gene into the intron-containing Igk-derived leader sequence coding region or removing a drug resistant gene in the vicinity of the transfer gene can be independently effective for improving the expression level of the transfer gene of a chimeric mouse. Further, through the performance of all of the procedures of changing the Igκ promoter region to be used, changing the leader sequence coding region inherent to the transfer gene into the intron-containing Igk-derived leader sequence coding region, and removing a drug resistant gene in the vicinity of the transfer gene, the expression level of the transfer gene is synergistically improved in a chimeric mouse, when compared with the expression level improved via single procedure. This indicates that the method disclosed by the present invention is very effective for functional analysis of a gene and a product thereof *in vivo.*

### Example 59

### Analysis of peripheral blood cells of CL hEPO murine ES cell-derived chimeric mouse, NL hEPO murine ES cell-derived chimeric mouse, PL hEPO murine ES cell-derived chimeric mouse, Ck loxPV hEPO murine ES cell-derived chimeric mouse, NP loxPV hEPO murine ES cell-derived chimeric mouse, and US hEPO murine ES cell-derived chimeric mouse

As controls, blood samples were obtained from the orbital sinus of chimeric mice derived from the RS element targeting murine ES cell line prepared in Example 11; chimeric mice derived from the CL hEPO murine ES cell line prepared in Example 42, chimeric mice derived from the NL hEPO murine ES cell line prepared in Example 46, chimeric mice derived from the PL hEPO murine ES cell line prepared in Example 50, chimeric mice derived from the Ck loxPV hEPO murine ES cell line prepared in Example 52, chimeric mice derived from the NP loxPV hEPO murine ES cell line prepared in Example 54, and chimeric mice derived from the US hEPO murine ES cell line prepared in Example 56, when they were 8 week old. The peripheral blood cells were analyzed using a blood cell analyzer (ADVIA 120 hematology system, Bayer Medical, Japan). Compared with the control mouse groups, blood erythrocyte counts of all chimeric mouse groups into which human EPO genes had been introduced were significantly increased. However, there was no significant difference in blood erythrocyte counts among groups. The hematocrit value of the control groups was 58% on average; however, the hematocrit value of all the chimeric mouse groups into which human EPO genes had been introduced was significantly enhanced. Thus, the hematocrit value was significantly increased by the introduction of human EPO genes. As with the case of blood erythrocyte counts, no significant differences were observed among group. Even though significant difference was observed in the serum human EPO levels among groups of Example 58, blood cell analysis would not exhibit any significant difference. This is considered to result from the fact that the human EPO gene expression level of chimeric mice prepared from the CL hEPO murine ES cell that exhibits the least expression level has already reached the upper limit of increase. This indicates that the method disclosed by the present invention is effective for functional analysis of a gene and a product thereof *in vivo.*

### Industrial applicability

The chimeric non-human animal or its progeny according to the present invention, cells or tissue derived therefrom, or hybridomas thereof enables the expression of foreign DNA at a level significantly higher, for example, several hundred times higher, than a level that is attained via conventional techniques. Accordingly, the present invention can be utilized as a method for mass production of desired proteins. Also, with the use of a chimeric non-human animal into which a foreign gene whose functions are unknown has been introduced or a progeny thereof, the present invention can be utilized for analysis of the *in vivo* function of the gene of interest based on differences in phenotypes.

All publications, patents, and patent applications cited herein are incorporated herein by reference in their entirety.

### Sequence Listing Free Text

SEQ ID NO: 1 to SEQ ID NO:46: primer
SEQ ID NO:47 to SEQ ID NO:54:linker
SEQ ID NO:55 to SEQ ID NO:58: primer
SEQ ID NO:59 to SEQ ID NO:60:linker
SEQ ID NO:61 to SEQ ID NO:64: primer

## Claims

1. A pluripotent cell derived from a non-human animal comprising foreign DNA that encodes a desired protein in such a manner that the expression of the desired protein is regulated by the control region of a gene expressed in certain cells and/or tissue, wherein the foreign DNA is bound to a nucleic acid fragment comprising a leader sequence coding region derived from the gene expressed in certain cells and/or tissue.

2. The pluripotent cell according to claim 1, wherein the gene expressed in certain cells and/or tissue is an immunoglobulin gene.

3. The pluripotent cell according to claim 2, wherein the immunoglobulin gene is an immunoglobulin light chain gene.

4. The pluripotent cell according to claim 1, wherein the nucleic acid fragment further comprises a promoter of the gene expressed in certain cells and/or tissue.

5. The pluripotent cell according to claim 4, wherein the nucleic acid fragment further comprises the whole or part of the 5' non-translational region between the promoter and the leader sequence coding region of the gene expressed in certain cells and/or tissue.

6. The pluripotent cell according to claim 1, wherein the nucleic acid fragment comprises the promoter/the whole or part of the 5' non-translational region/the leader sequence coding region of the gene expressed in certain cells and/or tissue.

7. The pluripotent cell according to claim 6, wherein the nucleic acid fragment comprises the promoter/the whole or part of the 5' non-translational region/the leader sequence coding region of the immunoglobulin gene derived from a non-human animal.

8. The pluripotent cell according to claim 1, wherein the nucleic acid fragment is greater than 300 bp.

9. The pluripotent cell according to any one of claims 1 to 8, which comprises a sequence encoding a polyA signal region ligated to a site downstream of foreign DNA encoding a desired protein.

10. The pluripotent cell according to claim 1, wherein one or more drug resistant marker genes used for introducing the foreign DNA into the genome have been removed

11. The pluripotent cell according to claim 1, wherein the alleles of the gene expressed in certain cells and/or tissue are inactivated.

12. The pluripotent cell according to claim 1, which is an embryonic stem (ES) cell.

13. The pluripotent cell according to claim 1, wherein the non-human animal is a mouse.

14. A method for preparing a chimeric non-human animal that overexpresses foreign DNA encoding a desired protein, comprising the steps of: preparing a pluripotent cell derived from a non-human animal according to any one of claims 1 to 13 and introducing the resulting cell into a host embryo to obtain a chimeric embryo; transplanting the chimeric embryo to a surrogate mother of a cognate non-human animal; and selecting a chimeric non-human animal that expresses foreign DNA encoding a desired protein from among the resulting offspring animals.

15. The method according to claim 14, wherein the chimeric non-human animal is a mouse.

16. The method according to claim 14, wherein the pluripotent cell is an embryonic stem (ES) cell.

17. A chimeric non-human animal, which is prepared by the method according to any one of claims 14 to 16 and which overexpresses foreign DNA encoding a desired protein.

18. A progeny of a non-human animal, which is prepared by mutual crossing of the chimeric non-human animals according to claim 17 or crossing of the chimeric non-human animal and a cognate non-human animal and which overexpresses foreign DNA encoding a desired protein.

19. A method for preparing a protein, comprising expressing desired foreign DNA using the chimeric non-human animal according to claim 17 or the progeny of a chimeric non-human animal according to claim 18, a cell or tissue obtained therefrom, or a hybridoma obtained therefrom, and recovering a protein produced and encoded by the DNA.

20. A method for analyzing *in vivo* function of a desired protein or DNA encoding the desired protein, comprising comparing a phenotype of the chimeric non-human animal according to claim 17 or the progeny of a chimeric non-human animal according to claim 18 with a phenotype of a corresponding wild-type non-human animal that does not contain foreign DNA encoding a desired protein and thereby determining a difference between the phenotypes.
